# EUROPEAN PATENT APPLICATION

(11) **EP 2 921 858 A1**
(43) Date of publication of application: **23.09.2015**
(21) Application number: 13800145.8
(22) Date of filing: 05.06.2013
(51) Int. Cl.: G01N 33/68

(54) **METHOD FOR OBTAINING DATA THAT ARE USEFUL FOR THE DIAGNOSIS, PROGNOSIS AND CLASSIFICATION OF PATIENTS WITH CHRONIC OBSTRUCTIVE PULMONARY DISEASE (COPD) AND/OR LUNG CANCER**

(30) Priority: 05.06.2012 ES 201230874
(71) Applicant: Servicio Andaluz de Salud, 41071 Sevilla (ES); Consejo Superior De Investigaciones Científicas (CSIC), 41013 Sevilla (ES); Universidad De Sevilla, 41013 Sevilla (ES)
(72) Inventor: PAZ-ARES GONZAGA, Luis Gonzalo, E-41071 Sevilla (ES); PASTOR HERRERA, María Dolores, E-41013 Sevilla (ES); MOLINA PINELO, Sonia, E-41013 Sevilla (ES); CARNERO MOYA, Amancio, E-41013 Sevilla (ES); SALINAS, Ana, E-41013 Sevilla (ES); BARBOSA DE SOUZA NOGAL, Ana, E-41013 Sevilla (ES)
(74) Representative: Hoffmann Eitle
(86) International application number: PCT/ES2013/070364
(87) International publication number: WO 2013/182725

(57) **Abstract**

The invention describes a method for obtaining useful data for the diagnosis, prognosis and classification of individuals with chronic obstructive pulmonary disease (COPD) and/or lung cancer, a diagnostic kit, a device and uses thereof for the diagnosis, prognosis and classification of patients into a) individuals without COPD or lung cancer, b) individuals with COPD, c) individuals with adenocarcinoma, d) individuals with COPD and adenocarcinoma or e) individuals with COPD and squamous carcinoma.

## Description

The present invention falls within the field of molecular biology and medicine and relates to a method for obtaining useful data for the diagnosis, prognosis and classification of individuals into:
- individuals presenting neither COPD nor lung cancer
- individuals with COPD
- individuals with adenocarcinoma
- individuals with squamous carcinoma
- individuals with COPD and adenocarcinoma
- individuals with COPD and squamous carcinoma.

### BACKGROUND ART

Lung cancer (LC) is the cause most commonly associated with cancer-related deaths worldwide; it is the cause of over 1.3 million deaths each year, representing 12.7 % of new cases of cancer. This neoplasm is divided into two main groups according to its clinicopathological characteristics: small-cell lung carcinoma (SCLC) and non-small-cell lung carcinoma (NSCLC). About 75-85 % of LC cases belong to the non-small-cell group. Non-small-cell lung carcinoma is histologically subdivided in two main categories. Squamous cell lung carcinoma (SCC), also known as epidermoid carcinoma, usually arises in the bronchial epithelium. Adenocarcinoma, generally arising in the peripheral airways and in the alveoli. The therapeutic options available to date for the treatment of lung cancer (LC) include surgery, chemotherapy and radiation therapy, which may be used separately or in different combinations. However, despite all the advances that have been made, the survival rate at 5 years for this type of tumor does not exceed 15 %.

The main risk factor for the development of LC is smoking. About 85-90 % of LC cases are caused by this habit. Moreover, tobacco consumption is also responsible for the development of other diseases, such as chronic obstructive pulmonary disease (COPD). The World Health Organization (WHO) estimates that 3 million people die each year from COPD. As with LC, not all smokers develop COPD. However, about 50 % of smokers eventually develop COPD. COPD also increases the relative risk of lung cancer by two to six times. Several studies suggest that inflammation may be one of the main processes involved in the pathogenesis of both diseases. The wide family of cytokines may be involved in mediating this process.

Cytokines are a diverse group of proteins including pro-inflammatory cytokines, cytokines derived from T cells, chemotactic cytokines (chemokines) from eosinophils, neutrophils, monocytes/macrophages and T cells, anti-inflammatory cytokines and various growth factors. Several studies carried out on patients diagnosed with COPD reveal that there is an increase of pro-inflammatory cytokine and chemokine levels (IL-1β, IL-2, IL-6, IL-8, IL-13, IP-10, INF-γ, MCP-1 and TNF-α) in samples obtained by sputum induction compared to healthy controls. Along these same lines, the tumor necrosis factor α-(TNF-α) and soluble TNF receptors are higher in the sputum of patients with COPD compared with healthy smokers.

Also, studies on lung cancer highlight the role of cytokines in this pathology correlating with studies on COPD. For example, the studies on polymorphisms reveal that there are specific polimorphisms in the IL-1A and 1B genes increasing the risk of LC, especially among older subjects with a history of heavy smoking.

Despite the work done in this field, the results obtained are insufficient to explain the role that inflammation plays in both conditions, as well as what possible mechanisms they share and which ones are independent of each other. In this sense, several inflammatory markers, both in LC and COPD, have been independently analyzed. However, it is still necessary to find an alternative method for the prediction, diagnosis and/or prognosis to allow the subclassing of individuals afflicted with lung cancer and/or chronic obstructive pulmonary disease.

### DESCRIPTION OF THE INVENTION

The authors of the present invention have analyzed the members of the cytokine and growth factor families in bronchoalveolar lavage (BAL) from patients with COPD, with adenocarcinoma, with squamous carinoma, simultaneously with COPD and adenocarcinoma, and patients with COPD and squamous cell cancer. Also, they have validated the results for IL-11 and CCL-1, discovering that both may be predictive biomarkers for adenocarcinoma and may improve the early diagnosis of lung adenocarcinoma in high-risk smokers, independently of the presence or absence of COPD.

The present invention provides a method for obtaining useful data for the diagnosis, prognosis and classification of individuals with said diseases.

Consequently, a first aspect of the invention relates to the use of cytokines and of growth factors selected from IL-6sR, IL-1a, IL-11, CCL-1, EOTAXIN-2, PDGFAA, TNFRI, TNFRII, EGF, MIP-1B, MIG, MCP-1, IGFBP2, IGFBP1, GDF-15, VEGF or any combinations thereof for the prediction, diagnosis, prognosis and classification of individuals into:
a) individuals without COPD or lung cancer,
b) individuals with COPD,
c) individuals with adenocarcinoma,
d) individuals with squamous carcinoma,
e) individuals with COPD and adenocarcinoma, or
f) individuals with COPD and squamous carcinoma.

Another aspect of the invention relates to the simultaneous use of the cytokines and of the growth factors selected from the list consisting of IL-6sR, IL-1a, IL-11, CCL-1, EOTAXIN-2, PDGFAA, TNFRI, TNFRII, EGF, MIP-1B, MIG, MCP-1, IGFBP2, IGFBP1, GDF-15, and VEGF for the prediction, diagnosis, prognosis and classification of the individuals into:
a) individuals without COPD or lung cancer,
b) individuals with COPD,
c) individuals with adenocarcinoma,
d) individuals with squamous carcinoma,
e) individuals with COPD and adenocarcinoma, or
f) individuals with COPD and squamous carcinoma.

A preferred embodiment relates to the use of IGFBP1, MIP1β, CCL-1, MIG, PDGFAA, GDF-15, VEGF and EGF for the prediction, diagnosis, prognosis and classification of the individuals into:
a) individuals without COPD or lung cancer,
b) individuals with COPD,
c) individuals with adenocarcinoma,
d) individuals with squamous carcinoma,
e) individuals with COPD and adenocarcinoma, or
f) individuals with COPD and squamous carcinoma.

From the examples of the present invention, a correlation is shown between high expression levels of IL-11 on the one hand, and cellular proliferation, invasiveness, metastases and poor prognosis on the other. Also, IL-11 and CCL-1 showed statistically significant expression differences in patients with adenocarcinoma compared to the remaining groups of patients.

Therefore, another preferred embodiment relates to the use of IL-11 and/or CCL-1 for predicting or prognosticating, or for early diagnosis of lung adenocarcinoma in an individual. In a more preferred embodiment, the individual is a smoker.

Another aspect of the invention relates to a method for obtaining useful data, hereinafter the first method of the invention, for the diagnosis, prognosis and classification of patients into a) individuals without COPD or lung cancer, b) individuals with COPD, c) individuals with COPD and lung cancer, c) individuals with adenocarcinoma, d) individuals with squamous carcinoma, e) individuals with COPD and adenocarcinoma, or f) individuals with COPD and squamous carcinoma, comprising:
i) obtaining an isolated biological sample from an individual, and
ii) quantifying the expression products of cytokines and growth factors selected from the list consisting of: IL-6sR, IL-1a, IL-11, CCL-1, EOTAXIN-2, PDGFAA, TNFRI, TNFRII, EGF, MIP-1B, MIG, MCP-1, IGFBP2, IGFBP1, GDF-15, and VEGF or any combination thereof.

In a preferred embodiment of this aspect of the invention, the expression of the cytokines and of the growth factors IL-6sR, IL-1a, IL-11, CCL-1, EOTAXIN-2, PDGFAA, TNFRI, TNFRII, EGF, MIP-1B, MIG, MCP-1, IGFBP2, IGFBP1, GDF-15, and VEGF is simultaneously quantified.

In another preferred embodiment, the method of the invention further comprises:
iii) comparing the amounts obtained in step (ii) to a reference amount.

The reference amount is obtained from the constitutive expression values from the gene(s) for the cytokines or growth factors in a group of healthy patients or, preferably, not presenting COPD or lung cancer.

More preferably, the first method of the invention comprises simultaneously quantifying the expression products of IGFBP1, MIP1β, CCL-1, MIG, PDGFAA, GDF-15, VEGF and EGF.

Steps (ii) and/or (iii) of the methods described above may be totally or partially automated, for example, by means of robotic sensor equipment for detecting the amount in step (ii) or the computerized comparison in step (iii).

An "isolated biological sample" includes, but is not limited to, cells, tissues and/or biological fluids from an organism, obtained by any method known to one skilled in the art. Preferably, the biological sample isolated from an individual in step (i) is the lavage or fluid or bronchoalveolar lavage (BAL).

The term "individual", as used in the description, refers to animals, preferably mammals, and more preferably, humans. The term "individual" is not intended to be limiting in any respect; it may be of any age, sex of physical condition.

The detection of the amount of IL-6sR, IL-1a, IL-11, CCL-1, EOTAXIN-2, PDGFAA, TNFRI, TNFRII, EGF, MIP-1B, MIG, MCP-1, IGFBP2, IGFBP1, GDF-15, VEGF may be carried out by any means known in the art. The authors of the present invention have demonstrated that the semi-quantitative or quantitative detection of the amount or concentration of antibodies against these cytokines and growth factors makes it possible to differentiate between the different histological types of lung cancer. Thus, a differential diagnosis in individuals afflicted by the mentioned diseases may be established, thus enabling it to be subclassified.

The measurement of the amount or concentration of these cytokines and growth factors, preferably semi-quantitatively or quantitatively, may be carried out directly or indirectly. The direct measurement relates to the measurement of the amount or concentration of the gene expression products, based on a signal obtained directly from the transcripts of such genes, or from the proteins, and which is directly correlated to the number of RNA molecules produced by the genes. Said signal - also referred to an intensity signal - may be obtained, for example, by measuring an intensity value of a chemical or physical property of said products. The indirect measurement includes the measure obtained from a secondary component or a biological measurement system (for example, the measure of cellular responses, ligands, "labels" or enzymatic reaction products).

The term "amount", as used in the description, relates to, but is not limited to, the absolute or relative amount of the gene expression products or of the antibodies, as well as any other value or parameter related to the same, or which may be derived therefrom. Such values or parameters comprise signal intensity values obtained from any physical or chemical properties of said expression products obtained by indirect measurement. Additionally, said values or parameters include all those obtained by indirect measurement, for example, any of the measurement systems described elsewhere herein.

The term "comparison", as used in the description, relates to, but is not limited to, the comparison of the amount of gene expression products or the amount of antibodies against IL-6sR, IL-1a, IL-11, CCL-1, EOTAXIN-2, PDGFAA, TNFRI, TNFRII, EGF, MIP-1B, MIG, MCP-1, IGFBP2, IGFBP1, GDF-15, VEGF from the biological sample to be analyzed, also called problem biological sample, to an amount of the gene expression products or to an amount of antibodies against IL-6sR, IL-1a, IL-11, CCL-1, EOTAXIN-2, PDGFAA, TNFRI, TNFRII, EGF, MIP-1B, MIG, MCP-1, IGFBP2, IGFBP1, GDF-15, VEGF from one or several desirable reference samples. The reference sample may be analyzed, for example, simultaneously or consecutively, along with the problem biological sample. The comparison described in paragraph (iii) of the method of the present invention may be carried out either manually or with computer assistance.

The term "expression product", also called "gene product", relates to the biochemical material, either RNA or protein, resulting from the expression of a gene. Sometimes, a measure of the amount of gene product is used in order to infer how active a gene is.

The term "reference amount", as used in the description, relates to an absolute amount or to a relative amount (with respect to the reference gene) of expression products of the genes or of antibodies against IL-6sR, IL-1a, IL-11, CCL-1, EOTAXIN-2, PDGFAA, TNFRI, TNFRII, EGF, MIP-1B, MIG, MCP-1, IGFBP2, IGFBP1, GDF-15, VEGF which makes it possible to discriminate between a) individuals without COPD or lung cancer, b) individuals with COPD, c) individuals with adenocarcinoma, d) individuals with squamous carcinoma, e) individuals with COPD and adenocarcinoma or f) individuals with COPD and squamous carcinoma.

The suitable reference amounts may be determined by means of the method of the present invention from a reference sample that may be analyzed, for example, simultaneously or consecutively, along with the biological problem sample. Thus, for example, but without limitation, the reference sample may be the negative controls, that is, the amounts detected by the method of the invention in samples from individuals not suffering from any of these diseases.

The soluble form of the interleukin 6 receptor (IL-6sR), with a molecular weight of approximately 50 kDa, has been found in urine from adult humans (Novick, D. et al., (1989) J. Exp. Med. 170:1409), in culture media conditioned by growing a human mieloma cell line (Nakajima, T. et al., (1992) Jpn. J. Cancer Res. 83:373), in supernatants from the culture of PHA-stimulated human PBMCs and from HTLV-1-positive T cell lines (Honda, M. et al., (1992) J. Immunol. 148:2175), and in serum from HIV-positive blood donors (Honda, M. et al., (1992) J. Immunol. 148:2175). This soluble form of the receptor apparently arises from the proteolytic cleavage of IL-6 R membrane linkage. Its amino acid sequence may be found under GenBank (NCBI) Accession Number NP_000556.1 and/or the SEQ ID NO: 2.

Within the context of the present invention, IL-6sR is also defined by a nucleotide or polynucleotide sequence, which constitutes the protein-coding sequence set forth in SEQ ID NO: 2, and which may comprise several variants from:
a) nucleic acid molecules coding for a polypeptide comprising the amino acid sequence of SEQ ID NO: 2,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 2, and wherein the polypeptide coded by said nucleic acids has the activity and structural characteristics of the IL-6sR protein. Among said nucleic acid molecules is the one collected in the GenBank (NCBI) NM_000565.3 sequence or SEQ ID NO: 1.

The IL-1a or interleukin 1 alpha gene (L-1A, IL1, IL1-ALPHA, IL1F1, IL-1 alpha, hematopoietin-1, interleukin-1 alpha, preinterleukin 1 alpha, pro-interleukin-1-alpha), is located within chromosome 2 (2q14) and codes for a protein which is a member of the family of interleukin 1 cytokines. It is a pleiotropic cytokine involved in several immunological responses, inflammatory processes and hematopoiesis. This cytokine is produced by monocytes and macrophages as a proprotein, which is proteolytically processed and released in response to cell damage, and induces apoptosis. This gene and another 8 genes of the interleukin 1 family form a gene cluster in chromosome 2. It has been suggested that the polymorphism of these genes is associated with rheumatoid arthritis and with Alzheimer's disease.

Within the context of the present invention, IL-1a is also defined by a nucleotide or polynucleotide sequence, which constitutes the protein-coding sequence set forth in SEQ ID NO: 4, and which may comprise several variants from:
a) nucleic acid molecules coding for a polypeptide comprising the amino acid sequence of SEQ ID NO: 4,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 4, and wherein the polypeptide coded by said nucleic acids has the activity and structural characteristics of the IL-6sR protein. Among said nucleic acid molecules is the one collected in the GenBank (NCBI) NM_000575.3 sequence or SEQ ID NO: 3.

The IL-11 or interleukin 11 gene (AGIF, IL-11), is located within chromosome 19 (19q13.3-q13.4), and codes for a protein which is a member of the family of cytokines gp130. These cytokines direct the assembly of receptor complexes with multiple subunits.

Within the context of the present invention, IL-11 is also defined by a nucleotide or polynucleotide sequence, which constitutes the protein-coding sequence set forth in SEQ ID NO: 6, and which may comprise several variants from:
a) nucleic acid molecules coding for a polypeptide comprising the amino acid sequence of SEQ ID NO: 6,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 6, and wherein the polypeptide coded by said nucleic acids has the activity and structural characteristics of the IL-11 protein. Among said nucleic acid molecules is the one collected in the GenBank (NCBI) NM_000641.2 sequence or SEQ ID NO: 5.

The CCL-1 or *chemokine (C-C motif*) *ligand 1* gene (-309, P500, SCYA1, SISe, TCA3, C-C motif chemokine 1; T lymphocyte-secreted protein I-309; inflammatory cytokine I-309; small inducible cytokine A1 (I-309, homologous to mouse Tca-3); small-inducible cytokine A1), is located within chromosome 17 (17q12) and codes for a protein which is a member of the family of cytokines related to the subfamily of CXC cytokines, characterized by two cysteins separated by a single amino acid. This cytokine is secreted by activated T cells and displays chemotactic activity for monocytes but not for neutrophils. It binds to the chemokine receptor CCR8.

Within the context of the present invention, CCL-1 is also defined by a nucleotide or polynucleotide sequence, which constitutes the protein-coding sequence set forth in SEQ ID NO: 8, and which may comprise several variants from:
a) nucleic acid molecules coding for a polypeptide comprising the amino acid sequence of SEQ ID NO: 8,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 8, and wherein the polypeptide coded by said nucleic acids has the activity and structural characteristics of the CCL-1 protein. Among said nucleic acid molecules is the one collected in the GenBank (NCBI) NM_002981.1 sequence or SEQ ID NO: 7.

The EOTAXIN-2 or *chemokine (C-C motif) ligand 24* gene (CCL24, Ckb-6, MPIF-2, MPIF2, SCYA24, C-C motif chemokine 24; CK-beta-6; eosinophil chemotactic protein 2; myeloid progenitor inhibitory factor 2; small inducible cytokine subfamily A (Cys-Cys), member 24; small-inducible cytokine A24), is located within chromosome 17 (17q11.23) and codes for a protein which is a member of the family of small cytokines CC. CC Cytokines are characterized by two adjacent cysteins. The cytokine coded by this gene displays chemotactic activity against T lymphocytes, minimal activity in neutrophils, and displays no activity in activated T lymphocytes. The protein is also a strong suppressor of colony formation by multipotent hematopoietic progenitor cell lines.

Within the context of the present invention, EOTAXIN-2 is also defined by a nucleotide or polynucleotide sequence, which constitutes the protein-coding sequence set forth in SEQ ID NO: 10, and which may comprise several variants from:
a) nucleic acid molecules coding for a polypeptide comprising the amino acid sequence of SEQ ID NO: 10,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 10, and wherein the polypeptide coded by said nucleic acids has the activity and structural characteristics of the CCL-1 protein. Among said nucleic acid molecules is the one collected in the GenBank (NCBI) NM_002991.2 sequence or SEQ ID NO: 9.

The PDGFAA or *platelet-derived growth factor alpha polypeptide* gene (PDGF-A, PDGF1, DGF A-chain; PDGF subunit A; PDGF-1; platelet-derived growth factor A chain; platelet-derived growth factor alpha chain; platelet-derived growth factor alpha isoform 2 preproprotein; platelet-derived growth factor subunit A), is located within chromosome 7 (7p22) and codes for a protein which is a member of the family of platelet-derived growth factors. The four members of this family are mitogenic factors for cells of mesenchimal origin and are characterized by an eight-cystein motif. The gene product may exist either as a homodimer or as a heterodimer with the beta polypeptide of platelet-derived growth factor, where the dimers are connected by disulfide bridges. Two splicing variants have been identified for this gene.

Within the context of the present invention, PDGFA is also defined by a nucleotide or polynucleotide sequence, which constitutes the protein-coding sequence set forth in SEQ ID NO: 12, and which may comprise several variants from:
a) nucleic acid molecules coding for a polypeptide comprising the amino acid sequence of SEQ ID NO: 12,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 12, and wherein the polypeptide coded by said nucleic acids has the activity and structural characteristics of the PDGFA protein. Among said nucleic acid molecules is the one collected in the GenBank (NCBI) NM_002607.5 sequence or SEQ ID NO: 11.

The TNFRI gene, or *tumor necrosis factor receptor superfamily, member 1A* (TNFRSF1A, CD120a, FPF, MGC19588, TBP1, TNF-R, TNF-R-I, TNF-R55, TNFAR, TNFR1, TNFR55, TNFR60, p55, p55-R, p60, TNF-R1; TNF-RI; TNFR-I; tumor necrosis factor binding protein 1; tumor necrosis factor receptor 1A isoform beta; tumor necrosis factor receptor superfamily member 1A; tumor necrosis factor receptor type 1; tumor necrosis factor-alpha receptor), is located within chromosome 12 (12p13.2) and codes for a protein which is a member of the superfamily of platelet-derived growth factors. The four members of the TNF receptor. It is one of the main receptors for tumor necrosis factor alpha. The receptor may activate NF-kappaB, mediate apoptosis and function as a regulator of inflammation. The anti-apoptotic BAG4/SODD protein and the TRADD and TRAF2 proteins have been observed to interact with this receptor and therefore play a role in receptor-mediated signal transduction.

Within the context of the present invention, TNFRI is also defined by a nucleotide or polynucleotide sequence, which constitutes the protein-coding sequence set forth in SEQ ID NO: 14, and which may comprise several variants from:
a) nucleic acid molecules coding for a polypeptide comprising the amino acid sequence of SEQ ID NO: 14,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 14, and wherein the polypeptide coded by said nucleic acids has the activity and structural characteristics of the TNFRI protein. Among said nucleic acid molecules is the one collected in the GenBank (NCBI) NM_001065.3 sequence or SEQ ID NO: 13.

The TNFRII or *tumor necrosis factor receptor superfamily, member 1B* gene (TNFRSF1 B, CD120b, TBPII, TNF-R-II, TNF-R75, TNFBR, TNFR1B, TNFR2, TNFR80, p75, p75TNFR, TNF-R2; TNF-RII; p75 TNF receptor; p80 TNF-alpha receptor; soluble TNFR1B variant 1; tumor necrosis factor beta receptor; tumor necrosis factor binding protein 2; tumor necrosis factor receptor 2; tumor necrosis factor receptor superfamily member 1B; tumor necrosis factor receptor type II), is located within chromosome 1 (1p36.22) and codes for a protein which is a member of the superfamily of TNF receptors. This protein and TNF-receptor 1 form a heterocomplex which mediates the recruitment of two apoptotic proteins, C-IAP1 and C-IAP2, which have E3 ubiquitin ligase activity.

Within the context of the present invention, TNFRII is also defined by a nucleotide or polynucleotide sequence, which constitutes the protein-coding sequence set forth in SEQ ID NO: 16, and which may comprise several variants from:
a) nucleic acid molecules coding for a polypeptide comprising the amino acid sequence of SEQ ID NO: 16,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 16, and wherein the polypeptide coded by said nucleic acids has the activity and structural characteristics of the TNFRII protein. Among said nucleic acid molecules is the one collected in the GenBank (NCBI) NM_001066.2 sequence or SEQ ID NO: 15.

The EGF or *epidermal growth factor* gene (HOMG4, URG, beta-urogastrone; pro-epidermal growth factor), is located within chromosome 4 (4q25) and codes for a protein which is a member of the epidermal growth factors superfamily.

Within the context of the present invention, EGF is also defined by a nucleotide or polynucleotide sequence, which constitutes the protein-coding sequence set forth in SEQ ID NO: 18, and which may comprise several variants from:
a) nucleic acid molecules coding for a polypeptide comprising the amino acid sequence of SEQ ID NO: 18,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 18, and wherein the polypeptide coded by said nucleic acids has the activity and structural characteristics of the EGF protein. Among said nucleic acid molecules is the one set forth in the sequence SEQ ID NO: 17.

The MIP-1B or *chemokine (C-C motif) ligand 4* gene (CCL4, CT2, AT744.1, G-26, HC21, LAG-1, LAG1, MGC104418, MGC126025, MGC126026, MIP-1-beta, MIP1B, MIP1B1, SCYA2, SCYA4, C-C motif chemokine 4; CC chemokine ligand 4; G-26 T-lymphocyte-secreted protein; MIP-1-beta(1-69); PAT 744; SIS-gamma; T-cell activation protein 2; lymphocyte activation gene 1 protein; lymphocyte-activation gene 1; macrophage inflammatory protein 1-beta; secreted protein G-26; small inducible cytokine A4 (homologous to mouse Mip-1b); small-inducible cytokine A4), is located within chromosome 17 (17q12).

Within the context of the present invention, MIP-1B is also defined by a nucleotide or polynucleotide sequence, which constitutes the protein-coding sequence set forth in SEQ ID NO: 20, and which may comprise several variants from:
a) nucleic acid molecules coding for a polypeptide comprising the amino acid sequence of SEQ ID NO: 20,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 20, and wherein the polypeptide coded by said nucleic acids has the activity and structural characteristics of the MIP-1B protein. Among said nucleic acid molecules is the one collected in the GenBank (NCBI) NM_002984.2 sequence or SEQ ID NO: 19.

The MIG or *chemokine (C-X-C motif) ligand 9* gene (CXCL9, CMK, Humig, MIG, SCYB9, crg-10, C-X-C motif chemokine 9; gamma-interferon-induced monokine; monokine induced by gamma interferon; monokine induced by interferon-gamma; small-inducible cytokine B9), is located within chromosome 4 (4q21).

Within the context of the present invention, MIG is also defined by a nucleotide or polynucleotide sequence, which constitutes the protein-coding sequence set forth in SEQ ID NO: 22, and which may comprise several variants from:
a) nucleic acid molecules coding for a polypeptide comprising the amino acid sequence of SEQ ID NO: 22,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 22, and wherein the polypeptide coded by said nucleic acids has the activity and structural characteristics of the MIG protein. Among said nucleic acid molecules is the one collected in the GenBank (NCBI) NM_002416.1 sequence or SEQ ID NO: 21.

The MCP-1 ó *chemokine (C-C motif*) *ligand 2* gene (CCL2, GDCF-2, HC11, HSMCR30, MCAF, MCP-1, MCP1, MGC9434, SCYA2, SMC-CF, C-C motif chemokine 2; monocyte chemoattractant protein 1; monocyte chemoattractant protein-1; monocyte chemotactic y activating factor; monocyte chemotactic protein 1; monocyte secretory protein JE; small inducible cytokine A2 (monocyte chemotactic protein 1, homologous to mouse Sig-je); small inducible cytokine subfamily A (Cys-Cys), member 2; small-inducible cytokine A2), is located within chromosome 17 (17q11.2-q12).

Within the context of the present invention, MCP-1 is also defined by a nucleotide or polynucleotide sequence, which constitutes the protein-coding sequence set forth in SEQ ID NO: 24, and which may comprise several variants from:
a) nucleic acid molecules coding for a polypeptide comprising the amino acid sequence of SEQ ID NO: 24,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 24, and wherein the polypeptide coded by said nucleic acids has the activity and structural characteristics of the MCP-1 protein. Among said nucleic acid molecules is the one collected in the GenBank (NCBI) NM_002982.3 sequence or SEQ ID NO: 23.

The IGFBP2 or *insulin-like growth factor binding protein 2* gene (IBP2, IGF-BP53, IBP-2; IGF-binding protein 2; IGFBP-2; insulin-like growth factor-binding protein 2), is located within chromosome 2 (2q33-q34).

Within the context of the present invention, IGFBP2 is also defined by a nucleotide or polynucleotide sequence, which constitutes the protein-coding sequence set forth in SEQ ID NO: 26, and which may comprise several variants from:
a) nucleic acid molecules coding for a polypeptide comprising the amino acid sequence of SEQ ID NO: 26,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 26, and wherein the polypeptide coded by said nucleic acids has the activity and structural characteristics of the IGFBP2 protein. Among said nucleic acid molecules is the one collected in the GenBank (NCBI) NM_000597.2 sequence or SEQ ID NO: 25.

The IGFBP1 or *insulin-like growth factor binding protein 1* gene (IGFBP1, AFBP, IBP1, IGF-BP25, PP12, hIGFBP-1, BP-1; IGF-binding protein 1; IGFBP-1; alpha-pregnancy-associated endometrial globulin; amniotic fluid binding protein; binding protein-25; binding protein-26; binding protein-28; growth hormone independent-binding protein; insulin-like growth factor-binding protein 1; placental protein 12), is located within chromosome 7 (7p13-p12).

Within the context of the present invention, IGFBP1 is also defined by a nucleotide or polynucleotide sequence, which constitutes the protein-coding sequence set forth in SEQ ID NO: 28, and which may comprise several variants from:
a) nucleic acid molecules coding for a polypeptide comprising the amino acid sequence of SEQ ID NO: 28,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 28, and wherein the polypeptide coded by said nucleic acids has the activity and structural characteristics of the IGFBP1 protein. Among said nucleic acid molecules is the one collected in the GenBank (NCBI) NM_000596.2 sequence or SEQ ID NO: 27.

The GDF-15 or *growth differentiation factor 15* gene (GDF-15, MIC-1, MIC1, NAG-1, PDF, PLAB, PTGFB, NRG-1; NSAID (nonsteroidal anti-inflammatory drug)-activated protein 1; NSAID-activated gene 1 protein; NSAID-regulated gene 1 protein; PTGF-beta; growth/differentiation factor 15; macrophage inhibitory cytokine 1; placental TGF-beta; placental bone morphogenetic protein; prostate differentiation factor), is located within chromosome 19 (19p13.11).

Within the context of the present invention, GDF-15 is also defined by a nucleotide or polynucleotide sequence, which constitutes the protein-coding sequence set forth in SEQ ID NO: 30, and which may comprise several variants from:
a) nucleic acid molecules coding for a polypeptide comprising the amino acid sequence of SEQ ID NO: 30,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 30, and wherein the polypeptide coded by said nucleic acids has the activity and structural characteristics of the GDF-15 protein. Among said nucleic acid molecules is the one collected in the GenBank (NCBI) NM_004864.2 sequence or SEQ ID NO: 29.

The VEGFA or *vascular endothelial growth factor A* gene (RP1-261G23.1, MGC70609, MVCD1, VEGF, VPF, vascular permeability factor), is located within chromosome 6 (6p12).

Within the context of the present invention, VEGF is also defined by a nucleotide or polynucleotide sequence, which constitutes the protein-coding sequence set forth in SEQ ID NO: 32, and which may comprise several variants from:
a) nucleic acid molecules coding for a polypeptide comprising the amino acid sequence of SEQ ID NO: 32,
b) nucleic acid molecules whose complementary strand hybridizes to the polynucleotide sequence of a),
c) nucleic acid molecules whose sequence differs from a) and/or b) due to the degeneracy of the gene code,
d) nucleic acid molecules coding for a polypeptide comprising the amino acid sequence with at least 80 %, 90 %, 95 %, 98 % or 99 % identity with SEQ ID NO: 32, and wherein the polypeptide coded by said nucleic acids has the activity and structural characteristics of the VEGF protein. Among said nucleic acid molecules is the one collected in the GenBank (NCBI) NM_001025366.2 sequence or SEQ ID NO: 31.

In another preferred embodiment, the detection of the amount of any of IL-6sR, IL-1a, IL-11, CCL-1, EOTAXIN-2, PDGFAA, TNFRI, TNFRII, EGF, MIP-1B, MIG, MCP-1, IGFBP2, IGFBP1, GDF-15, VEGF is carried out by means of an immunoassay. The term "immunoassay", as used in the present description relates to any analytical technique based on the reaction of conjugation of an antibody to an antigen. Exemplary immunoassays known in the art are, for example, without limitation: immunoblot, enzyme-linked immunosorbent assay (ELISA), linear immunoassay (LIA), radioimmunoassay (RIA), immunofluorescence, x-map or protein chips.

In another preferred embodiment, the immunoassay is an enzyme-linked immunosorbent assay or ELISA. ELISA is based on the premise that an immunoreactive (antigen or antibody) may be immobilized on a solid support, then placing this system in contact with a fluid phase containing a complementary reagent which may bind to a marker compound. There are different types of ELISA: direct ELISA, indirect ELISA or sandwich ELISA.

The term "marker compound", as used in the present description, relates to a compound capable of yielding a chromogenic, fluorogenic, radioactive and/or chemoluminiscent signal which enables the detection and quantification of the amount of antibodies against IL-6sR, IL-1a, IL-11, CCL-1, EOTAXIN-2, PDGFAA, TNFRI, TNFRII, EGF, MIP-1B, MIG, MCP-1, IGFBP2, IGFBP1, GDF-15, VEGF. The marker compound is selected from the list comprising radioisotopes, enzymes, fluorophores or any molecule which may be conjugated to another molecule or be directly detected and/or quantified. This marker compound may bind directly to the antibody, or via another compound. Some examples of marker compounds which bind directly are, without limitation, enzymes, such as alkaline phosphatase or peroxidase, radioactive isotopes, such as ³²P or ³⁵S, fluorochromes, such as fluorescein or metal particles, for direct detection by means of colorimetry, self-radiography, fluorimetry or metallography, respectively.

Another aspect of the invention relates to a method for the diagnosis, prognosis and classification of individuals, hereinafter the second method of the invention, comprising the steps (i)-(iii) according to the first method of the invention, and further comprises assigning the individual of step (i) to the group of individuals without COPD or lung cancer, when the expression of the *IGFBP1, MIP1β, CCL-1, MIG and PDGFAA* genes is not present.

In a preferred embodiment, the second method of the invention comprises the steps (i)-(iii) according to the first method of the invention, and further comprises assigning the individual of step (i) to the group of individuals with adenocarcinoma, when there is an increase of the expression product of the *IL-11* and/or *CCL-1* genes relative to a reference amount.

In another preferred embodiment, the second method of the invention comprises the steps (i)-(iii) according to the first method of the invention, and further comprises assigning the individual of step (i) to the group of individuals with COPD when the expression of *MIG* is detected, and the expression of *CCL-1* and *IGFBP1* is not detected.

In another preferred embodiment, the second method of the invention comprises the steps (i)-(iii) according to the first method of the invention, and further comprises assigning the individual of step (i) to the group of individuals with adenocarcinoma when the expression of *CCL-1* is detected and the amount of expression of *MIP1β* is lower than 25 pg/ml, preferably lower than 22 pg/ml, and even more preferably, lower than 20 pg/ml.

In another preferred embodiment, the second method of the invention comprises the steps (i)-(iii) according to the first method of the invention, and further comprises assigning the individual of step (i) to the group of individuals with squamous carcinoma when any expression level of *PDGFAA* or *MIP1β* is detected, and the expression of *CCL-1* is not detected.

In another preferred embodiment, the second method of the invention comprises the steps (i)-(iii) according to the first method of the invention, and further comprises assigning the individual of step (i) to the group of individuals with COPD and adenocarcinoma when any expression level of *CCL-1* is detected and *VEGF* expression is lower than 240 pg/ml, more preferably lower than 220 pg/ml, and even more preferably, lower than 200 pg/ml.

In another preferred embodiment, the second method of the invention comprises the steps (i)-(iii) according to the first method of the invention, and further comprises assigning the individual of step (i) to the group of individuals with COPD and squamous carcinoma when the expression of *GDF-15* is detected above 40 pg/ml, and more preferably above 50 pg/ml and the expression of *VEGF* is above 180 pg/ml, and more preferably above 200 pg/ml, respectively, and no expression of *CCL-1* or *EGF* is detected.

There is currently no cure for COPD; however, it is a preventable and treatable disease. For the treatment of COPD, the current most employed approaches are quitting smoking, rehabilitation and drug therapy (often the use of inhalers). Some patients will require long-term treatment with oxygen or lung transplant.

To this end, bronchodilators are drugs that relax the smooth muscle of the airways, which increases the airway caliber and improves airflow, thus reducing the symptoms of shortness of breath, etc., and resulting in a better quality of life of people with COPD. However, they do not slow the progression of the underlying disease. Bronchodilators are generally administered with an inhaler or through a nebuliser.

There are two main types of bronchodilators, β2 agonists and anticholinergics. Anticholinergics seem to be superior to β2 agonists in COPD. Anticholinergics reduce respiratory deaths, whereas β2 agonists have no effect on mortality from respiratory diseases. Each type may be long acting (with an effect that lasts 12 hours or more) or short acting (with a rapid onset of the effect, which lasts only a short time).

### β2 agonists

β2 agonists stimulate receptors in bronchial smooth muscle, causing it to relax. There are several β2 agonists available. Salbutamol (common brand name: Ventolin) and terbutalin are widely used as short-acting β2 agonists, providing a rapid relief of symptoms from COPD. Long-acting β2 agonists (LABAs), such as salmeterol and formoterol, are used as maintenance therapy, and their use leads to a better airflow, an improvement in exercise capacity, and improved quality of life.

### Anticholinergics

Anticholinergic drugs relax the airways by blocking the stimulation of cholinergic nerves. Ipratropium provides short action and rapid relief from COPD symptoms. Tiotropium is a long-acting anticholinergic, whose regular use is associated with improvements in the airflow, exercise capacity and quality of life. Ipratropium is associated with cardiovascular morbidity.

### Corticosteroids

Corticosteroids are used in the form of a tablet or inhaled for treating and preventing acute manifestations of COPD. Inhaled corticosteroids (ICS) have not been demonstrated to be beneficial for people with mild COPD; however, they have been demonstrated to diminish acute exacerbations in individuals with moderate or severe COPD. However, they are associated with higher rates of pneumonia.

### Other drugs

Theophylline is a bronchodilator and an inhibitor of phosphodiesterase, which in high doses may reduce the symptoms in some people with COPD. Side effects, such as nausea and heart stimulation, limit its use. The phosphodiesterase-4 antagonists roflumilast and cilomilast have gone through Phase 2 of clinical trials. Tumor necrosis factor antagonists, such as infliximab, suppress the immune system and reduce inflammation. Infliximab has been tested on patients with COPD, but there was no evidence of benefit.

### Lung cancer

The common treatments involve surgery, chemotherapy and radiation therapy. NSCLC *(non-small-cell lung carcinoma)* is treated with surgery, whereas SCLC *(small-cell lung carcinoma)* generally responds better to chemotherapy and radiation therapy. This is partly because SCLC often spreads very early, and these treatments are better when reaching cancer cells which have spread to other parts of the body.

### Lung cancer treatment

The treatment for lung cancer depends on the type of cancer, its dissemination and the condition of the patient. Common treatments involve palliative care, surgery, chemotherapy and radiation therapy.

### Surgery

If studies confirm non-small-cell lung carcinoma, the stage should be re-evaluated in order to determine whether the disease is localized or if it has spread to the point that it cannot be cured with surgery. To this end, computerized tomography and positron emission tomography (PET) are used. Blood analyses and pulmonary function tests are also necessary to assess if the patient is well enough to undergo surgery. If pulmonary function tests reveal poor respiratory reserve, surgery may be contraindicated.

In most cases of the first stages of non-small-cell lung cancer, the removal of the lung lobe (lobectomy) is the surgical treatment of choice. In patients who are not candidates for total lobectomy, a small sublobular excision (wedge resection) may be performed. However, the wedge resection poses a higher risk of recurrence of the disease than lobectomy. Brachytherapy with radioactive iodine in the wedge resection margins may reduce the risk of recurrence. Rarely, the removal of an entire lung (pneumonectomy) is performed.

Video-assisted thoracoscopic surgery and video-assisted lobectomy use a minimally invasive approach to lung-cancer surgery. The VATS lobectomy is equally effective compared to conventional open lobectomy, and with less post-surgery disease.

In small-cell lung carcinoma (SCLC), chemotherapy and/or radiation therapy are commonly used. However, the role of surgery in SCLC is being reconsidered. Surgery may improve the results when added to chemotherapy and radiation in the early stage.

### Radiation therapy

Radiation therapy is often administered along with chemotherapy, and may be used with curative intent in patients with non-small-cell lung carcinoma who are not eligible for surgery. This form of high-intensity radiation therapy is called radical radiation therapy. A refinement of this technique is accelerated continuous hyperfractionated radiation therapy, wherein a high dose of radiation is administered in a short period of time. For small-cell, the cases of lung carcinoma which are potentially curable, radiation to the chest in addition to chemotherapy is often recommended. Postoperative thoracic radiation therapy in general, should not be used after curative surgery for non-small-cell lung carcinoma.

If the cancer growth blocks a short section of the bronchi, brachytherapy (localized radiation therapy) may be directly administered inside the airway to open the passage. Compared to external radiation therapy, brachytherapy allows for a reduction in treatment time and reduces health workers' exposure to radiation.

Prophylactic cranial irradiation (PCI) is a kind of radiation therapy in the brain, used to reduce the risk of metastasis. PCI is more useful in small-cell lung carcinoma.

Recent improvements in orientation and images have given rise to the development of stereotactic radiation in the treatment of early-stage lung cancer. In this form of radiation therapy, high doses are delivered in a small number of sessions by means of stereotaxis. Its use is primarily in patients who are not eligible for surgery due to medical comorbidities.

Therefore, in non-small-cell and small-cell lung carcinomas patients, lower doses of radiation to the chest may be used to control the symptoms (palliative radiation therapy).

### Chemotherapy

The chemotherapy regimen depends on the type of tumor.

### Small-cell lung carcinoma

Although in a relatively early stage, small-cell lung carcinoma is primarily treated with chemotherapy and radiation. In small-cell lung carcinoma, the most widely-used chemotherapeutics are cisplatin and etoposide. Combinations thereof with carboplatin, gemcitabine, paclitaxel, vinorelbine, topotecan and irinotecan are also used.

### Non-small-cell lung carcinoma

In advanced non-small-cell lung carcinoma, chemotherapy improves survival and is used as a first-line treatment, as long as the patient is well enough to receive treatment. Two drugs are generally used, one of which is often based on platinum (cisplatin or carboplatin). Other drugs used are gemcitabine, paclitaxel and docetaxel.

Advanced non-small-cell lung carcinoma is often treated with cisplatin or carboplatin, combined with gemcitabine, paclitaxel, docetaxel, etoposide or vinorelbin. Recently, pemetrexed is also being used.

### Adjuvant chemotherapy

Adjuvant chemotherapy relates to the use of chemotherapy after curative surgery to improve its results. In non-small-cell lung cancer, samples of nearby lymph nodes are taken during surgery. If stage II or III of the disease is confirmed, the adjuvant chemotherapy improves survival at 5 years by 5 %. The combination of vinorelbin and cisplatin is more effective than old therapeutic regimens.

Adjuvant chemotherapy for patients with stage IB cancer is controversial, since the clinical trials have not clearly demonstrated a survival benefit. Trials on preoperative chemotherapy (neoadjuvant chemotherapy) in operable non-small-cell lung carcinoma have not been conclusive.

### Chemotherapy

In patients with stage-3 lung cancer who are not eligible for surgery, combined treatment with radiation therapy and chemotherapy significantly improves survival.

### Targeted therapy

In recent years, several specific molecular therapies have been developed for treating advanced lung cancer. Gefitinib (Iressa) is one such drug, focusing on the tirosin kinase domain of the epidermal growth factor receptor (EGFR), expressed in many cases of non-small-cell lung carcinoma. It has not been demonstrated to increase survival, although women, Asians, non-smokers and those with bronchioalveolar carcinoma seem to derive the most benefit from gefitinib.

Erlotinib (Tarceva), another inhibitor of EGFR tyrosine kinase, improves survival in non-small-cell lung carcinoma, and was approved by the FDA in 2004 for second-line treatment thereof. As with gefitinib, it also seems to work better in women, Asians, non-smokers and those with bronchioalveolar carcinoma, particularly those with specific mutations in EGFR.

The angiogenesis inhibitor, Bevacizumab (Avastin), (combined with paclitaxel and carboplatin), improves survival in patients with non-small-cell lung carcinoma. However, it increases the risk of lung bleeding, especially in patients with squamous-cell carcinoma.

The advances in cytotoxic drugs, pharmacogenetics and drug design look promising. A number of targeted agents are in the first stages of clinical research, such as ciclooxygenase-2 inhibitors, the apoptosis promoter exisulind, proteasome inhibitors, bexarotene, the epidermal growth factor receptor inhibitor cetuximab, and vaccines. Crizotinib has proven to be quite promising in the first clinical trials in a subgroup of non-small-cell lung carcinoma characterized in that the fusion oncogene EML4-ALK is found in some relatively young patients, occasional or non-smokers, with adenocarcinoma. Future fields of research involve inhibitors of the proto-oncogene ras, the inhibition of fosfoinositido-3-kinase, the inhibition of the deacetylase histone, and the replacement of the tumor suppressor gene.

Therefore, another aspect of the invention relates to the use of a pharmaceutical composition comprising an active principle selected among a β2 agonist, an anticholinergic, a compound of the group of corticosteroids, a phosphodiesterase inhibitor and an immune system suppressor, to prepare a medicine for treating an individual with COPD identifiable by the method of the invention.

Another aspect of the invention relates to the use of a pharmaceutical composition comprising an active principle selected among platinum coordination complexes (cisplatin or carboplatin), gemcitabine, paclitaxel, docetaxel, etoposide, vinorelbin, pemetrexed, gefitinib, erlotinib, bevacizumab or any combinations thereof, to prepare a medicine for treating an individual with adenocarcinoma and/or squamous carcinoma associated or not with COPD, identifiable by the method of the invention.

As used herein, the term "active principle", "active substance", "pharmaceutically active substance", "active ingredient" or "pharmaceutically active ingredient" means any component which potentially provides a pharmacological activity or another different effect in the diagnosis, cure, alleviation, treatment or prevention of a disease, or which affects the structure or function of the human body or the bodies of other animals. The term includes those components which promote a chemical change in the preparation of the drug and are present therein in a foreseen modified form providing the specific activity or effect.

Another aspect of the present invention relates to a kit or device, hereinafter the kit of the invention, comprising the necessary elements to quantify the expression of cytokines and growth factors selected from *IL-6sR, IL-1a, IL-11, CCL-1, EOTAXIN-2, PDGFAA, TNFRI, TNFRII, EGF, MIP-1B, MIG, MCP-1, IGFBP2, IGFBP1, GDF-15, VEGF* or any combinations thereof.

In a preferred embodiment, the kit comprises the necessary elements to quantify the expression of *IGFBP1, MIP-1β, CCL-1, MIG, PDGFAA, GDF-15, VEGF* and *EGF.*

In another preferred embodiment, the kit comprises the necessary tools to quantify the expression products of the cytokines and of the growth factors CCL-1 and/or IL-11. More preferably, the kit or device comprises the anti-CCL-1 and anti-IL-11 antibodies.

Even more preferably, the kit of the present invention comprises the antibodies selected from the list consisting in: anti-IL-6sR, anti-IL-1a, anti-IL-11, anti-CCL-1, anti-EOTAXIN-2, anti-PDGFAA, anti-TNFRI, anti-TNFRII, anti-EGF, anti-MIP-1B, anti-MIG, anti-MCP-1, anti-IGFBP2, anti-IGFBP1, anti-GDF-15, anti-VEGF antibodies or any combinations thereof. More preferably, the kit comprises the anti-IGFBP2, anti-MIP-1B, anti-CCL-1, anti-MIG, anti-PDGFAA, anti-GDF-15, anti-VEGF or anti-EGF antibodies.

In another preferred embodiment, the kit of the invention comprises secondary antibodies, or positive and/or negative controls. The kit may further include, without limitation, buffers, protein extraction solutions, agents for preventing contamination, protein degradation inhibitors, etc.

Also, the kit may include all the supports and containers required for start up and optimization. Preferably, the kit also comprises instructions to perform the methods of the invention.

Another aspect relates to the use of the kit of the invention for the diagnosis, prognosis and classification of a) individuals without COPD or lung cancer, b) individuals with COPD, c) individuals with adenocarcinoma, d) individuals with squamous carcinoma, e) individuals with COPD and adenocarcinoma or f) individuals with COPD and squamous carcinoma.

Another aspect of the invention relates to computer-readable storage means comprising program instructions capable of making a computer perform the steps of any of the methods of the invention (of the first or the second method of the invention).

In a preferred embodiment, the computer-readable storage means comprise at least one of the anti-IL-6sR, anti-IL-1a, anti-IL-11, anti-CCL-1, anti-EOTAXIN-2, anti-PDGFAA, anti-TNFRI, anti-TNFRII, anti-EGF, anti-MIP-1B, anti-MIG, anti-MCP-1, anti-IGFBP2, anti-IGFBP1, anti-GDF-15 and anti-VEGF antibodies, or any combinations thereof. In another more preferred embodiment, the computer-readable storage means comprises the anti-IGFBP1, anti-MIP1β, anti-CCL-1, anti-MIG, anti-PDGFAA, anti-GDF-15, anti-VEGF and anti-EGF antibodies.

The methods of the invention may include additional steps, such as, for example, protein separation by mono and bi-dimensional (2D-PAGE) electrophoresis, or prior digestion with trypsin of a protein mixture (of the sample), with subsequent purification and analysis of the peptides by means of mass spectrometry (MS), such as MALDI-TOF, or by means of multi-dimensional chromatography, by means of ICAT (isotope-coded affinity tags), DIGE (Differential gel electrophoresis) or protein arrays.

In another preferred embodiment, the computer-readable storage means comprise oligonucleotides or single-channel microarrays designed from a known sequence or mRNA of at least one of the *ILSsR, IL-1a, IL-11, CCL-1, EOTAXIN-2, PDGFAA, TNFRI, TNFRII, EGF, MIP-1B, MIG, MCP-1, IGFBP2, IGFBP1, GDF-15,* and *VEGF genes,* or any combinations thereof. Even more preferably, the computer-readable storage means comprise oligonucleotides or single-channel microarrays designed from a known sequence or mRNA of the *IGFBP1, MIP-1β, CCL-1, MIG, PDGFAA, VEGF* and *EGF* genes.

Thus, for example, the oligonucleotide sequences are constructed on the surface of the chip by means of sequential elongation of a growing chain with a single nucleotide using photolithography. Therefore, the oligonucleotides are anchored by the 3' end by means of a selective activation method of nucleotides, protected by a photolabile reagent, by the selective incidence of light through a photomask. The photomask may be physical or virtual.

Thus, the oligonucleotide probes may be from 10 to 100 nucleotides long, more preferably from 20 to 70 nucleotides long, and even more preferably from 24 to 30 nucleotides long. In order to quantify gene expression, preferably about 40 oligonucleotides are used per gene.

*In situ* synthesis on a solid support (for example, glass) may be performed using ink-jet technology, thus requiring longer probes. The supports may be, without limitation, NC or nylon filters or membranes (charged), silicon, or glass slides for microscopes coated with aminosilanes, polylisine, aldehydes or epoxy. The probe is each one of the samples of the chip. The target is the sample to be analyzed: messenger RNA, total RNA, a PCR fragment, etc.

Another aspect of the invention relates to a transmissible signal comprising program instructions capable of making a computer perform the steps of any of the methods of the invention.

The terms "polynucleotide" and "nucleic acid" are used interchangeably herein, referring to polymeric forms of nucleotides of any length, both ribonucleotides (RNA) and deoxyribonucleotides (DNA).

The terms "amino acid sequence", "peptide", "oligopeptide", "polypeptide" and "protein" are used interchangeably herein and relate to a polymeric form of amino acids of any length, which may be coding or non-coding, chemically or biochemically modified.

Throughout the description and the claims, the word "comprises" and variants thereof are not intended to exclude other technical features, additives, components or steps. For those skilled in the art, other objects, advantages and characteristics of the invention may be deduced from both the description and the practical use of the invention. The following examples and drawings are provided by way of illustration, and are not intended to limit the present invention.

### BRIEF DESCRIPTION OF THE DRAWINGS

Fig. 1. Component association hierarchical cluster.
Fig. 2. Analysis of significant expression differences of the 16 proteins of interest by calculating the p value, p<0.05*, p<0.01**, p<0.001***.
Fig. 3. Analysis of the 16 proteins of interest by Western blotting with specific antibodies.
Fig. 4. Validation of the differential expression of proteins of interest by ELISA.
Fig. 5. Analysis of the sensitivity and specificity of the proteins of interest, for each group of the studied pathologies, from the expression data obtained by the ELISA methodology.
Fig. 6. Study profile.
Fig. 7. A. Cluster heat map of 80 supervised proteins differentially expressed among the control group and the disease groups.
Cluster-analysis dendrograms of the samples and the proteins, shown in the top and left, respectively. Up- and down-regulation of the protein is indicated in red and blue, respectively. B. Expression levels of 16 proteins of interest after map analysis. The expression levels of each protein measurement, with respect to its mean level of expression by the condition. The error bars represent values of the median, p <0.05*, p <0.01**, p <0.005***.

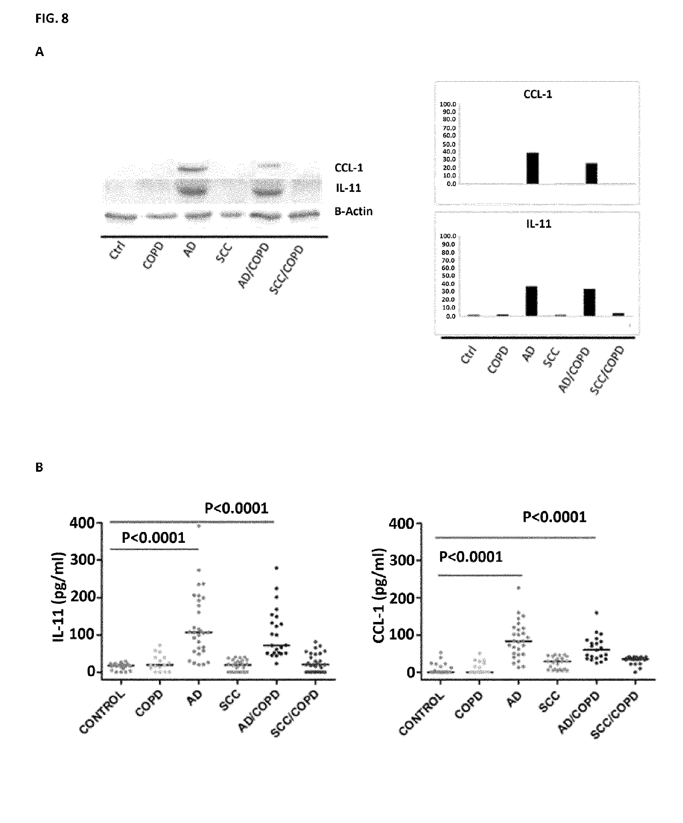

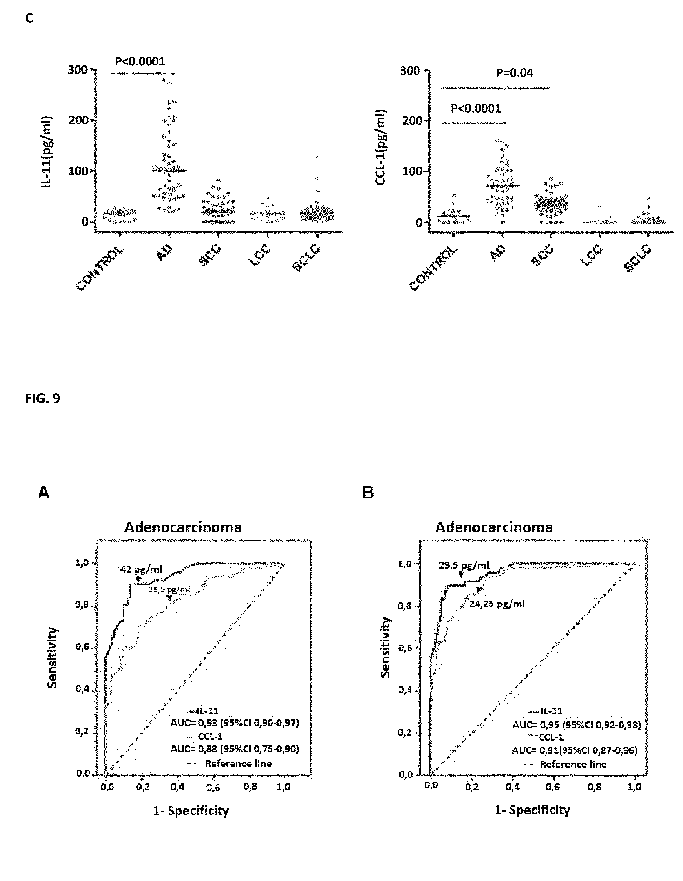

Fig. 8. The candidate proteins chosen, CCL-1 and IL-11 were validated by Western blot and ELISA. A. Western blot analysis of IL-11 and CCL-1 proteins in different samples. B. Analysis of IL-11 and CCL-1 protein levels by ELISA in the first validation cohort. The black horizontal lines are the medians. C. Analysis of IL-11 and CCL-1 protein levels by ELISA in the second validation cohort. The black horizontal lines are the median.
Fig. 9. ROC curves for IL-11 and CCL-1. A. ROC curves for IL-11 and CCL-1 in patients with adenocarcinoma vs. all groups of the first validation cohort. B. ROC curves for IL-11 and CCL-1 in patients with adenocarcinoma vs. all groups of the second validation cohort.

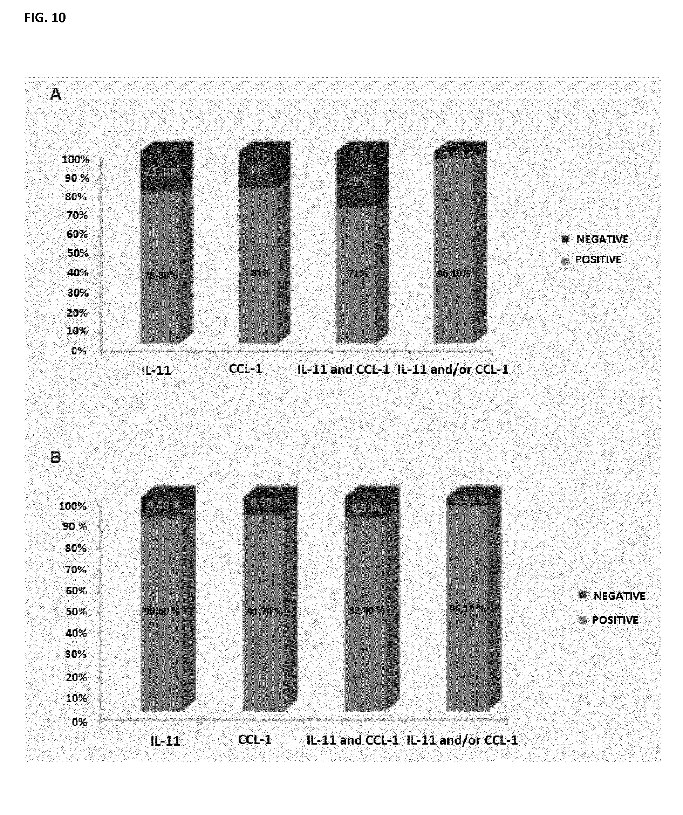

Fig. 10. Positive results rate for IL-11 and CCL-1 in patients with adenocarcinoma. A. Positive results rate for IL-11, CCL-1, IL-11 and CCL-1, IL-11 and/or CCL-1 in all patients with adenocarcinoma in the first cohort. B. Positive result rate for IL-11, CCL-1, IL-11 and CCL-1, IL-11 and/or CCL-1 in all patients with adenocarcinoma in the additional cohort.

### EJEMPLOS

### EXAMPLARY EMBODIMENTS OF THE INVENTION

The invention is illustrated by means of several assays performed by the inventors.

### EXAMPLE 1. DETERMINATION OF CYTOKINES WITH DIFFERENTIAL EXPRESSION

### Patients and samples

A total of 141 samples of bronchoalveolar lavage (BAL), from 4 different groups of patients (patients with COPD, with LC, with COPD and LC and without COPD or LC), from 2009 until 2011.

The samples were divided into two groups. The first group of samples from 60 patients was used to perform the study. A description of all the patients included may be found in Table 1. The second group, comprising 81 patients, was used to validate the results. (Table 2). All samples were taken at the Virgen del Rocio Hospital (Seville, Spain), from patients who had required flexible bronchoscopy for diagnostic purposes. The present study was approved by the Ethics Committee of the Hospital, and informed consent was obtained from all the patients before including them in the study.

The subjects were prepared with a combination of topical anesthesia (benzocaine 20 % aerosol in the pharynx plus topical lidocain 2 % as needed) and conscious sedation with midazolam and meperidine according to the institutional guidelines. The bronchoalveolar lavage (BAL) samples were obtained from the instillation and aspiration of 40 to 60 ml aliquots of sterile saline 0.9 % in the bronchopulmonary segment. The liquid recovered was passed immediately through a sterile 100-micron nylon filter (Becton Dickinson, San Jose, CA) in order to remove the mucus, and then was taken on ice to the laboratory. The total volume was centrifuged for 10 minutes at 1800 x g and 4 °C. The supernatant was divided in aliquots into 2 ml tubes and frozen at -80 °C until further use.

### Sample treatment

The experiments were carried out in approximately 4.8 ml of sample. Given its low protein content, the BAL samples were concentrated before use by means of a vacuum concentrator (Concentrator plus - Eppendorf, Hamburg, Germany). To this end, samples were thawed on ice, adding a protease inhibitor cocktail (Thermo Scientific, Franklin, MA, USA). The initial volume of the samples was reduced to 250-450 µl in 2.6 hours. Protein quantification was determined by means of the RCDC method (Bio-Rad, Hercules, CA, USA).

### Protein arrays

In order to study the protein profiles of the four groups of patients, an antibody array, a commercially available kit which analyzes the expression levels of 80 cytokines and growth factors (Quantibody ®, cytokine human antibody array 1000 - RayBiotech, Norcross, GA, USA), was used. The antibody analysis was performed according to the instructions provided by the manufacturer. Briefly, the microarrays were incubated with the blocking buffer at room temperature for 30 minutes and then with the sample for 120 min. The microarrays were washed with wash buffer I three times and with the wash buffer II for 2 hours at room temperature (5 min per wash). Then, the microarrays were incubated with an antibody cocktail at room temperature for 120 minutes. Finally, the microarrays were washed and incubated with Cy3 equivalent to conjugated streptavidin at room temperature for 120 minutes. The intensity measures equivalent to the expression of each protein were visualized by means of a GenePix 4100A Laser Scanner (Molecular Devices, Sunnyvale, CA, USA). The intensities of each protein were measured in quadruplicate for each sample.

### Western blot

50 µg of proteins from BAL were gel-separated at between 7 and 12 %, depending on the size of the protein, by polyacrilamide gel electrophoresis with sodium dodecil sulphate (SDS-PAGE) and then transferred to polyvinylidene fluoride (PVDF) membranes (Bio-Rad, Hercules, CA, USA), blocked with a 5 % BSA solution, and incubated overnight at 4 °C with primary antibodies according to the manufacturer's instructions: anti-EGF (1:500, Santa Cruz, CA, USA), anti VEGF, anti-IGFPB1, anti-IGFBP2, anti-eotaxin2 (CCL24), anti-MIP-1β (CCI4), anti-MIG, anti-GDF15 (CXCL9), anti-PDGFAA, anti-IL6R, anti-IL1R1, anti-IL11 y anti-I309 (1:200 Abcam, Cambridge , MA, USA), anti-TNFRI, anti TNFRII and anti-MCP-1 (1:1000 Cell signaling, Beverly, MA, USA). Consecutively, they were incubated with secondary antibodies, conjugated to anti-mouse peroxidase (GE Healthcare, Uppsala, Sweden) and anti-rabbit (Cell Signaling, Beverly, MA, USA), were applied to the individual membranes (1:2000) for 1 hour at room temperature. The bands corresponding to the proteins of interest were developed by chemoluminiscence using ECL (GE Healthcare, Uppsala, Sweden) and were visualized in an image analyzer (Mini LAS-3000, Fujifilm, Tokyo, Japan). The relative protein levels were calculated in comparison to the amount of β-actin (1:1000 Abcam, Cambridge, MA, USA). The experiments were repeated three times independently.

### ELISA

The ELISA assays were carried out by using specific antibodies in BALF samples from the second cohort of patients (validation cohort); ELISA sandwich assays were used for CCL4/MIP-1β, CXCL9/MIG, IGFBP-2, IGFBP-1, EGF, RI/TNFRSF1A sTNF, RI/TNFRSF1A sTNF, IL-1 RI, IL-6 Rα, CCL2/MCP-1, VEGF, GDF-15, CCL24/Eotaxin-2/MPIF-2, CCL1/I-309, IL-11, PDGF-AA (DuoSet, R & D Systems, Minneapolis, Minnesota, USA). All samples were analyzed in duplicate. The limits of detection for these assays were established between 25 and 200 µg of protein.

The protocol for performing the ELISA was as follows. First, the capture antibody was diluted in PBS and 100 µl thereof were added to each well of a 96-well plate, which was incubated overnight at 4 °C. Then, it was washed with TBS containing Tween-20 0.05 % (TBST 0.05 %). Next, it was blocked with 1 % bovine serum albumin for 1 hour at room temperature, and washed again with TBST 0.05 %. The samples and protein patterns were added consecutively and were incubated for 2 hours at room temperature. Upon completion of the foregoing steps, the detection antibody diluted in PBS was added for 2 hours at room temperature. Finally, streptavidin was added (DuoSet, R & D Systems) and the plate incubated for 30 minutes. Lastly, the coloring reagent o-phenylendiamine was added. The reaction was carried out in the dark for 20 minutes. After this time, H₂SO₄ 2 N was added to each well to quench the reaction, and the absorbance was read on a plate reader at 450 nm (Emax, Molecular Devices, Minneapolis, Minnesota, USA). The absorbance measurements are extrapolated to a standard curve obtained from the protein patterns in order to learn the measured concentration of the protein in the sample.

### Results obtained

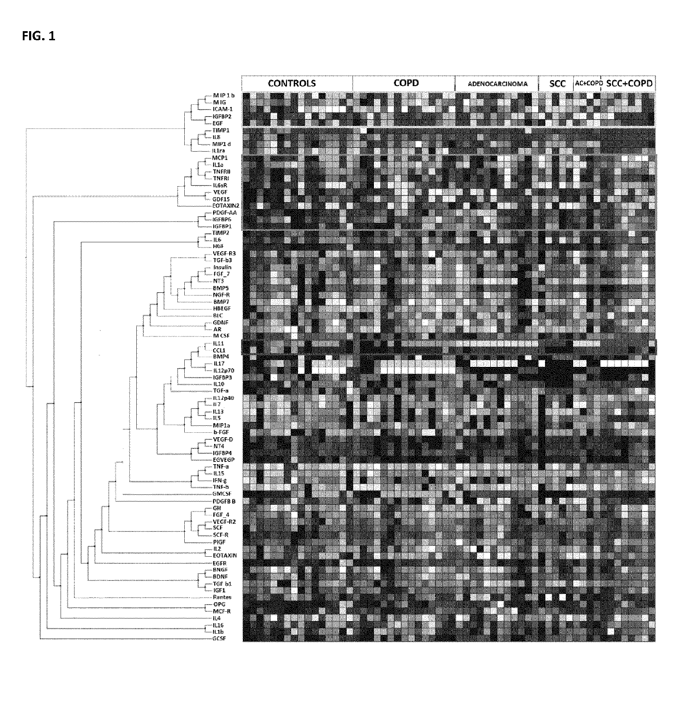

The first step to carry out the study was performing the antibody arrays (80 cytokines and growth factors involved in the inflammatory response) on the 60 patients summarized in Table 1. The data obtained from reading the arrays were analyzed by constructing a hierarchical supervised cluster using the Euclidean distance as a component-association method, and by using the program Babelomics, version 4.2 (Minguez P and J Dopazo) for statistical analysis (Figure 1). The expression level of each protein, relative to its mean expression level in all conditions, was represented in values from 0-100 %. From this analysis, 16 proteins of interest are highlighted (CCL4/MIP-1β, CXCL9/MIG, IGFBP-2, IGFBP-1, EGF, RI/TNFRSF1A sTNF, RI/TNFRSF1A sTNF, IL-1 RI, IL-6 Rα, CCL2/MCP-1, VEGF, GDF-15, CCL24/Eotaxin-2/MPIF-2, PDGF-AA CCL1/I-309, IL-11), which represent expression differences within the different groups of pathologies studied with respect to the control group (no COPD, no LC). For example, CCL1 and IL-11 proteins were only expressed in adenocarcinoma or adenocarinoma with COPD.

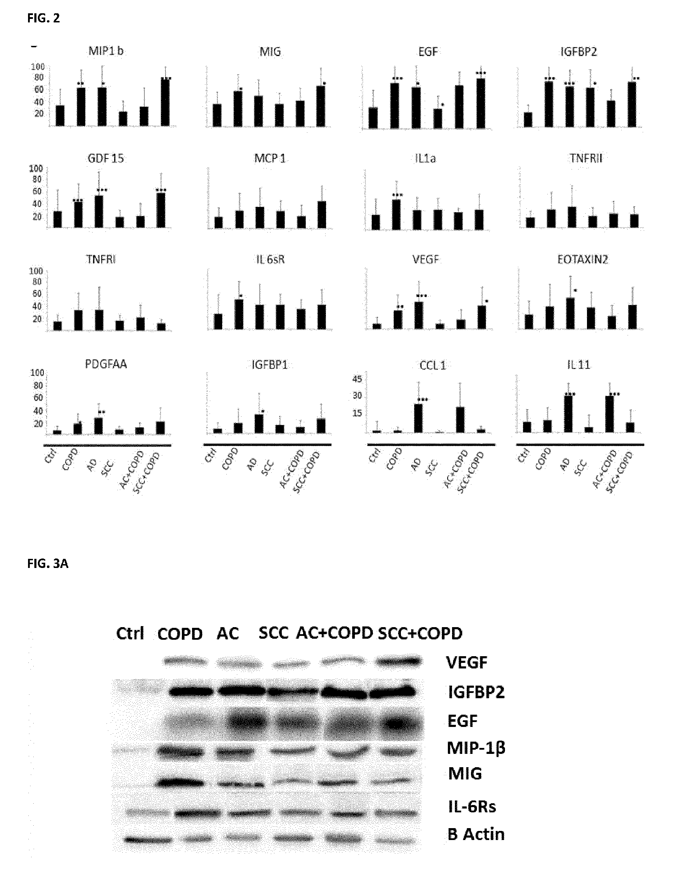

Meanwhile, the mean and standard deviation were analyzed for each of the proteins of interest for each of the pathologies, as well as for the control group. Where p<0.05 *, p<0.01**, p<0.001*** (Figure 2). Similar significant differences to those existing within the different pathological groups were observed with respect to the control group in the cluster analysis. This suggests that this selection of proteins may help discern between pathologies, since each protein has a different expression with respect to the control group depending on the pathology.

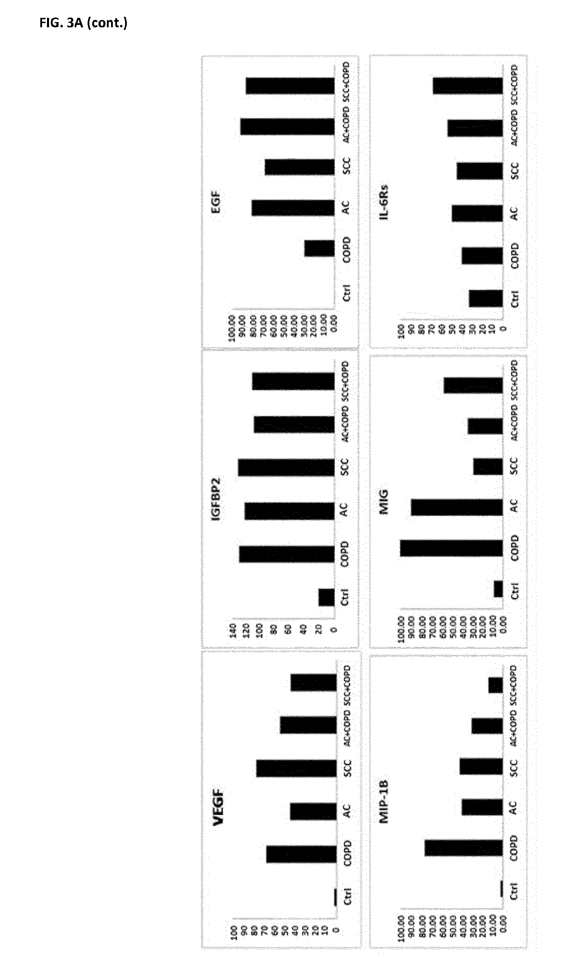

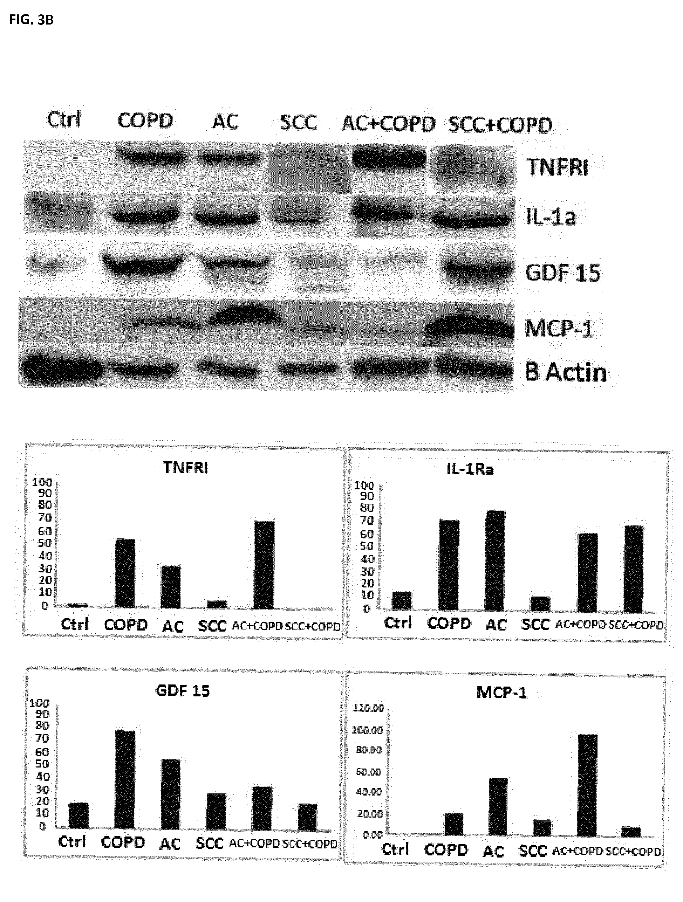

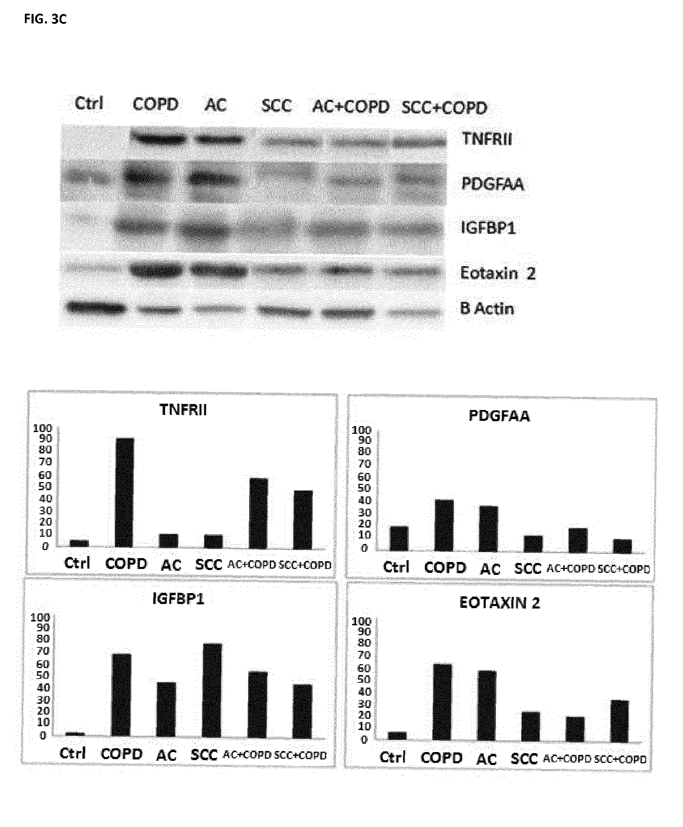

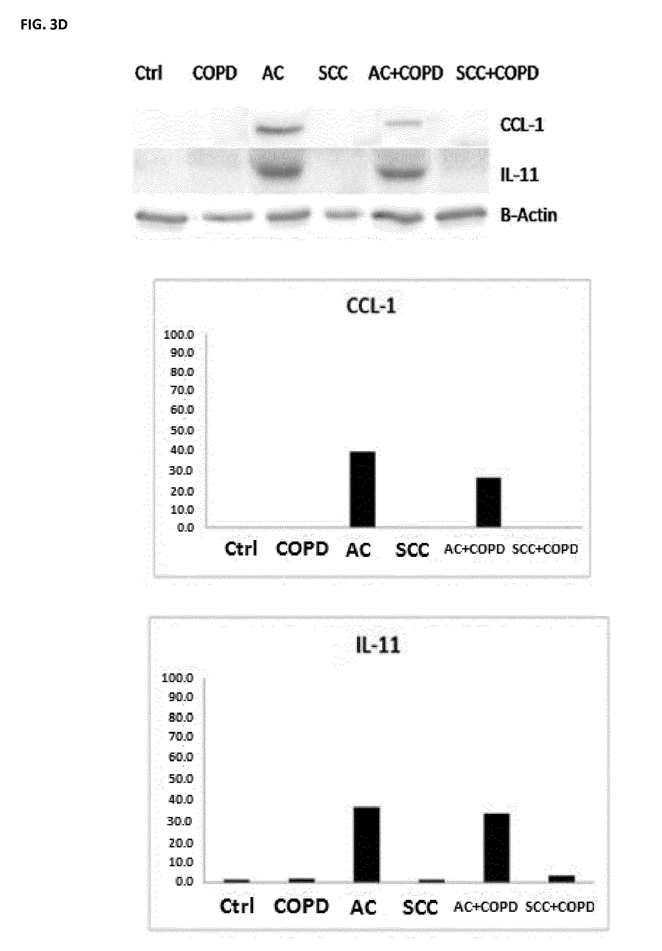

Next, we sought to examine the relationship of the selected proteins in greater depth, and their possible involvement in the studied pathologies. It was proposed to validate the results found by using another different methodology; to this end, the Western Blot methodology was used. The expression of each protein selected in the BAL samples from the patients selected in Table 1 were measured by means of this methodology (Figure 3). The results obtained were coherent with those previously obtained, so it has become increasingly apparent that the proteins selected are involved to some extent in the pathologies studied in this project.

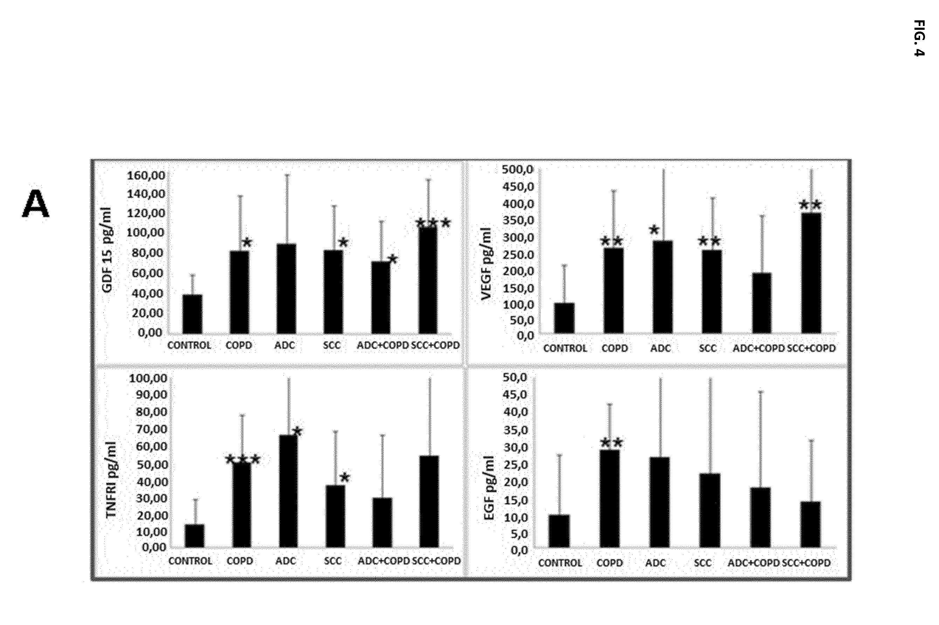

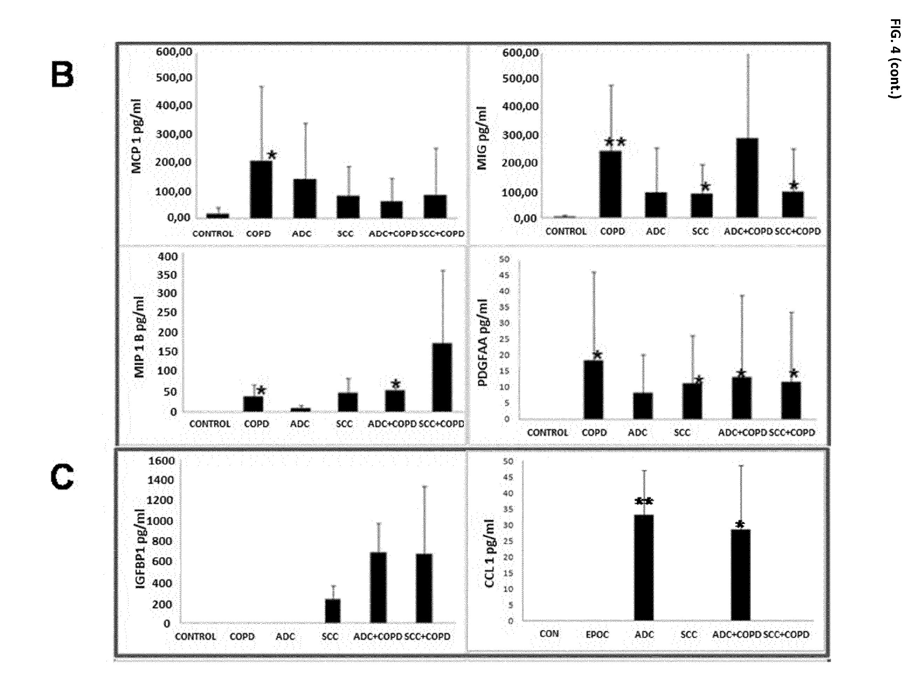

The next step was obtaining a sufficiently robust profile that was suitable for use in everyday clinical practice. To achieve this goal, a new cohort of patients was used, with characteristics similar to the previous cohort, with the only modification of a higher number of patients in the LC and LC/COPD group. This increase was due to the need to elucidate the differences observed in the previous experiments among the two types of LC (ADE and SCC). For the measurement of proteins the ELISA methodology was used, since its use is widespread in clinical practice (Figure 4). On the one hand, it was found through this analysis that not all the proteins participate in all the pathological groups, as had been observed in previous analysis, and that there is a group of 4 proteins (GDF15, VEGF, EGF, TNFR I) with significant expression differences with respect to the control group, another 4 proteins (MIG, MIP-1B, MCP1, PDGFAA) which are practically unexpressed in the control, and yet exhibit important differences within the various pathological groups, and two proteins, (IGFBP1, CCL1) which are only expressed in certain pathological groups; on the other hand, this analysis helped to establish a more robust protein profile than the previous one, suitable to be extrapolated to a kit for clinical practice (Figure 4).

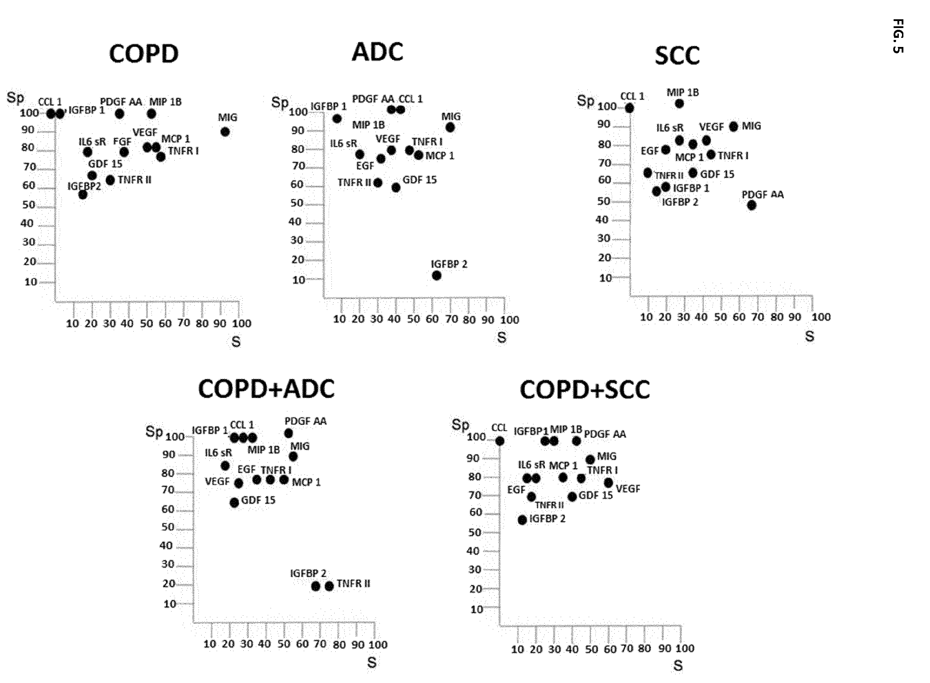

Later, the potential of each protein as a biomarker was analyzed in each pathology studied. To this end, the sensitivity, specificity, positive predictive value and negative predictive value was studied from the results obtained in the previous paragraph (Figure 5). This analysis makes clear that the MIG protein could be used as biomarker. MIG exhibits a sensitivity and specificity of about 90 % in BAL samples from patients with COPD, while CCL1 and IGFBP1 proteins exhibit a 0 % sensitivity and 100 % specificity, indicating that these three proteins could diagnose this pathology by means of a conventional ELISA. Likewise, CCL1 protein represents a 100 % specificity and a 40 % sensitivity, and so may be a discriminating biomarker for samples from adenocarcinoma. This analysis consolidates the protein profile obtained throughout the different analyses, helping to establish a possible kit capable of discriminating patients within the different groups of the study.

Lastly, a logistical regression model was used by calculating the odds ratio in order to define the possible diagnostic kit. This made it possible to associate whether the presence or absence of certain proteins was related with the likelihood of having a certain pathology (Table 4).

**Table 1. Characteristics of the first cohort of patients**

| | **Control n=16** | **COPD n=15** | **LC n=17** | **COPD with LC n=12** |
|---|---|---|---|---|
| **Gender** | | | | |
| Male | 100.0% (16) | 100.0 % (15) | 100.0% (17) | 100.0 % (12) |
| Female | 0.0% (0) | 0.0% (0) | 0.0% (0) | 0.0% (0) |
| **Mean age [range]** | **61.3 [41-80]** | **61.5 [45-78]** | **60.7 [46-69]** | **60.7 [49-68]** |

| **Status** | | | | |
|---|---|---|---|---|
| Smoker | 68.8 % **(11)** | 53.3% (8) | 52.9% (9) | 83.3% **(10)** |
| Ex-smoker | 31.2% **(5)** | 46.7% (7) | 47.1% (8) | 16.7% **(2)** |
| **Packs/year** | **21.82** | **32.2** | **35.21** | **30.78** |

| **COPD** | | | | |
|---|---|---|---|---|
| 1 | - | 20.0% (3) | - | 58.3% **(7)** |
| 2 | - | 33.3% (5) | - | 25.0% **(3)** |
| 3 | - | 26.7% (4) | - | **0.0% (0)** |
| 4 | - | 20.0% (3) | - | 16.7% **(2)** |

| **Lung Cancer** | | | | |
|---|---|---|---|---|
| ADC | - | - | 70.6% **(12)** | 33.3% **(14)** |
| SCC | - | - | 29.4% **(5)** | 66.7% **(8)** |

**Table 2. Characteristics of the second cohort of patients**

| | **Control n=10** | **COPD n=13** | **LC n=24** | **COPD with LC n=34** |
|---|---|---|---|---|
| **Gender** | | | | |
| Male | 60.0% (6) | 84.6% (11) | 83.3% (20) | 95.5% (32) |
| Female | 40.0% (4) | 15.4% (2) | 16.7% (4) | 4.5% (2) |
| **Mean age [range]** | 52.3 [42-58] | 65.2 [51-75] | 61.2 [48-75] | 64.3 [48-75] |

| **Status** | | | | |
|---|---|---|---|---|
| Smoker | 100.0% (10) | 53.8% (7) | 50.0% (12) | 53.0% (18) |
| Ex-smoker | 0.0% (0) | 46.2% (6) | 50.0% (12) | 47.0% (16) |
| **Packs/year** | 21.82 | 34.2 | 33.21 | 39.78 |

| **COPD** | | | | |
|---|---|---|---|---|
| 1 | - | 30.8% (4) | - | 26.5% (9) |
| 2 | - | 53.8% (7) | - | 50.0% (27) |
| 3 | - | 15.4% (2) | - | 23.5% (8) |
| 4 | - | 0.0% (0) | - | 0.0% (0) |

| **Lung Cancer** | | | | |
|---|---|---|---|---|
| ADC | - | - | 37.5% (9) | 47.0% (16) |
| SCC | - | - | 62.5% (15) | 53.0% (18) |

**Table 3. Amount of proteins used in the ELISA**

| **PROTEIN** | **µg** |
|---|---|
| **GDF-15** | 25 |
| **TNFRI** | |
| **VEGF** | |
| **EGF** | |
| **MIG** | 50 |
| **MCP-1** | |
| **IGFBP-1** | |
| **PDGFAA** | **75** |
| **MIP1β** | 125 |
| **CCL-1** | **200** |

**Table 4. Logistical regression analysis by calculating the odds ratio. Association between the potential condition and whether or not the pathological group is permanent as defined.**

| **Group** | **CONDITION** | **P-value** | **OR (95% CI)** |
|---|---|---|---|
| **Control (without COPD or LC)** | IGFP1 negative | P<0.001 | 50 (5.64-500) |
| | MIP1B negative | | |
| | CCL1 negative | | |
| | MIG negative | | |
| | PDGFAA negative | | |
| **COPD** | IGFP1 negative | 0.002 | 12.071 (2.485-58.629) |
| | CCL1 negative | | |
| | MIG positive | | |
| **Adenocarcinoma** | CCL1 positive | 0.002 | 20.83 (7.69-76.92) |
| | MIP1B negative | | |
| **Squamous carcinoma** | CCL1 negative | 0.009 | 5.88 (1.52-22.2) |
| | EGF negative | | |
| | PDFGAA or MIP1B positive | | |
| **Adenocarcinoma and COPD** | CCL1 positive | 0.001 | 5.31 (1.96-14.49) |
| | VEGF negative | | |
| **Squamous carcinoma and COPD** | GDF 15 positive | 0.002 | 9.34 (2.19-40) |
| | VEGF positive | | |
| | CCL-1 negative | | |
| | EGF negative | | |

### EXAMPLE 2. ADDITIONAL VALIDATION OF IL-11 AND CCL-1 CYTOKINES Materials and methods

### Patients and samples

In order to carry out this prospective study, a total of 359 patients who had required flexible bronchoscopy for diagnosis were chosen between 2009 and 2011. All the bronchoalveolar lavage (BAL) samples were collected from patients of the Virgen del Rocío Hospital (Seville, Spain). The selection criteria for the study were:
1) the patients having been screened by the pulmonology services because of hemoptysis or pulmonary nodules,
2) being smokers or ex-smokers of more than 20 packs per year,
3) being above 40 years old.

The exclusion criteria were:
1) presence of another carcinoma or sarcoma,
2) active pulmonary tuberculosis,
3) previous pulmonary resection,
4) history of drug abuse, and
5) presence of another acute or chronic inflammatory disease, besides chronic obstructive pulmonary disease (COPD). Approval for this study was given by the ethics committee of the institution. Furthermore, and according to the committee's regulations, informed consent was obtained from the participants.

The BAL protein profiles of the different groups of patients were analyzed by comparing the control group (patients without COPD or lung cancer) with the disease groups. The latter included a group with COPD, a group with lung cancer and a group with COPD and lung cancer. The samples were divided in three cohorts. A first group of samples from 60 patients was used to develop the initial study. The description of the patients included in the study shown in Table 5A. The second group of independent samples from 139 different patients (Table 5B) and a third group of independent samples from 160 different patients (Table 5C) were used.

**Table 5. (A) Characteristics of the control population used for the protein array. (B) Characteristics of the population used for validation. (C) Characteristics of the population used for additional validation.**

| **A** | | | | |
|---|---|---|---|---|
| | **Control n=16** | **COPD n=15** | **Lung Cancer N=17** | **COPD with Lung Cancer n=12** |
| **Gender** | | | | |
| Male | 100.0 % (16) | 100.0 % (15) | 100.0 % (17) | 100.0 % (12) |
| Female | 0.0% (0) | 0.0% (0) | 0.0% (0) | 0.0% (0) |
| **Mean age** | 61.3 [41-80] | 61.5 [45-78] | 60.7 [46-69] | 60.7 [49-68] |

| **Currently** | | | | |
|---|---|---|---|---|
| Smoker | 68.8% (11) | 53.3% (8) | 52.9% (9) | 83.3% (10) |
| Ex-smoker | 31.2% (5) | 46.7% (7) | 47.1% (8) | 16.7% (2) |
| **Age ranges** | 38.8 [31-53.2] | 50 [42-65] | 58.2 [41-65.7] | 52.3 [41-63.2] |

| **COPD** | | | | |
|---|---|---|---|---|
| Mild | - | 20.0% (3) | - | 58.3% (7) |
| Moderate | - | 33.3% (5) | - | 25.0% (3) |
| Severe | - | 26.7% (4) | - | 0.0% (0) |
| Very severe | - | 20.0% (3) | - | 16.7% (2) |

| **Lung Cancer** | | | | |
|---|---|---|---|---|
| Adenocarcinom | - | - | 70.6 % (12) | 33.3 % (4) |
| a Stage I-II | | | 16.6 % (2) | 0.0 % (0) |
| Stage III-IV | | | 83.4 % (10) | 100 % (4) |
| SCC | - | - | 29.4% (5) 20% (1) 80% (4) | 66.7% (8) |
| Stage I-II | | | | 12.5% (1) |
| Stage III-IV | | | | 87.5% (7) |

| **B** | | | | |
|---|---|---|---|---|
| | **Control n=20** | **COPD n=29** | **Lung Cancer n=40** | **COPD with Lung Cancer n=50** |
| **Gender** | | | | |
| Male | 60.0% (12) | 86.2% (25) | 82.5% (33) | 96.0% (48) |
| Female | 40.0% (8) | 13.8% (4) | 17.5% (7) | 4.0% (2) |
| **Mean age** | 52.3 [42-58] | 65.2 [51-75] | 61.2 [48-75] | 64.3 [48-75] |

| **Currently** | | | | |
|---|---|---|---|---|
| Smoker | 100.0 % (20) | 51.7% (15) | 50.0% (20) | 52.0% (26) |
| Ex-smoker | 0.0% (0) | 48.3% (14) | 50.0% (20) | 48.0% (24) |
| **Age ranges** | 41.8 [33-57.2] | 52.8 [41-69.2] | 55 [39-73.2] | 57 [43-75.2] |

| **COPD** | | | | |
|---|---|---|---|---|
| Mild | - | 27.6% (8) | - | 26.0% (13) |
| Moderate | - | 51.7% (15) | - | 50.0% (25) |
| Severe | - | 20.7% (6) | - | 24.0% (12) |
| Very severe | - | 0.0% (0) | - | 0.0% (0) |

| **Lung Cancer** | | | | |
|---|---|---|---|---|
| Adenocarcino | - | - | 37.5% (15) | 46.0% (23) |
| ma Stage I-II | | | 26.7% (4) | 21.7% (5) |
| Stage III-IV | | | 73.3% (11) | 78.3% (18) |
| SCC | - | - | 62.5% (25) | 54.0% (27) |
| Stage I-II | | | 40% (10) 60% (15) | 55.6% (15) |
| Stage III-IV | | | | 44.4% (12) |

| **C** | | | | |
|---|---|---|---|---|
| | **Control n=20** | **LC n=66** | **COPD with LC n=74** | |
| **Gender** | | | | |
| Male | 60.0% (12) | 83.3% (55) | 94.6% (70) | |
| Female | 40.0% (8) | 16.7% (11) | 5.4% (4) | |
| **Mean age [range]** | 52.3 [42-58] | 61.2 [48-75] | 64.3 [48-75] | |

| **Currently** | | | | |
|---|---|---|---|---|
| Smoker | 100.0 % (20) | 50.0% (33) | 52.7% (39) | |
| Ex-smoker | 0.0% (0) | 50.0% (33) | 47.3% (35) | |
| **Age ranges** | 36.6 [30-51.2] | 58.8 [41-77.2] | 62 [43-79.2] | |

| **Lung Cancer** | | | | |
|---|---|---|---|---|
| Adenocarcino ma | | 22.7% (15) | 16.2% (12) | |
| Stage I-II | - | 20% (3) | 16.7% (2) | |
| Stage III-IV | | 80% (12) | 83.3% (10) | |
| SCC | | 18.2% (12) | 13.5% (10) | |
| Stage I-II | - | 33.3% (4) | 60% (6) | |
| Stage III-IV | | 66.6% (8) | 30% (4) | |
| LCC | - | 12.1 % (8) | 18.9% (14) | |
| Stage I-II | | 37.5 % (3) | 42.85% (6) | |
| Stage III-IV | | 62.5 % (5) | 57.15% (8) | |
| SCLC | | 47.0% (31) | 51.4% (38) | |
| Limited stage | - | 41.9% (13) | 42.1% (16) | |
| Extensive stage | | 58.1% (18) | 57.9% (22) | |

The subjects were treated with a combination of topical anesthesia (benzocaine 20 % aerosol into the pharynx plus topical lidocaine 2 % as needed) and conscious sedation using midazolam and meperidine according to the institutional guidelines. The bronchoalveolar lavage samples were obtained by instillation and aspiration of 40-60 ml aliquots of sterile saline solution in the bronchopulmonary segment. The fluid recovered was immediately passed through an sterile 100-µm nylon filter (Becton Dickinson, San Jose, CA) to remove mucus, and was taken on ice to the laboratory. The total volume was centrifuged at 4 °C for 10 minutes at 1800 x g and was frozen at -80 °C until later use.

### Sample treatment

Approximately 4-8 ml of BAL sample were used in the experiments. Given its low protein content, the BAL samples had to be concentrated before use. The BAL samples were thawed on ice with a protease inhibitor kit (Thermo Scientific, Franklin, MA, USA). The samples were aliquoted in new tubes and were placed in a vacuum concentrator (Concentrator Plus - Eppendorf, Hamburg, Germany). The initial volume of the samples was reduced from 1.5 to 2 ml in 2-6 hours. The quantification of proteins was carried out by means of the RCDC method (Bio-Rad, Hercules, CA, USA).

### Protein arrays

In order to study the protein profiles of the four patient groups, a cytokine and growth-factor screening was carried out. The protein matrix used in the study is commercially available and analyzes the expression levels of 80 cytokines and growth factors (Quantibody ® cytokine human antibody array 1000 - RayBiotech, Norcross, GA, USA). A full list of the proteins analyzed in the study may be found in tables 6 and 7. The assay matrix for human cytokines was carried out according to the instructions provided by the manufacturer. The intensity of each signal was visualized by means of a laser scanner, model GenePix 4100 A (Molecular Devices, Sunnyvale, CA, USA). The intensity points for each cytokine (four spots per protein/positive control) were fused and expressed as the relative mean to the average signals of the positive controls in the microarrays analyzed with respect to the disease groups (patients with COPD, with lung cancer, with COPD and lung cancer) and the control group (patients without COPD or lung cancer).

**Table 6. Characteristics of the antibodies used in the study.**

| **Symbol of the cytokine** | **Name of the cytokine** |
|---|---|
| **BLC** | B-cell lymphoma |
| **Eotaxin** | Eotaxin |
| **Eotaxin 2** | Eotaxin 2 |
| **G-CSF** | Granulocyte colony-stimulating factor |
| **GM-CSF** | Granulocyte macrophage colony-stimulating factor |
| **I-309/CCL1** | Chemokine (C-C motif) ligand 1 |
| **ICAM1** | Intercellular adhesion molecule 1 |
| **IFNg** | Interferon-gamma |
| **IL-1a** | Interleukin-1 alpha |
| **IL-1b** | Interleukin-1 beta |
| **IL-1ra** | Interleukin antagonist receptor-1 |
| **IL-2** | Interleukin-2 |
| **IL-4** | Interleukin-4 |
| **IL-5** | Interleukin-5 |
| **IL-6** | Interleukin-6 |
| **IL-6Sr** | Interleukin soluble receptor-6 |
| **IL-7** | Interleukin-7 |
| **IL-8** | Interleukin-8 |
| **IL-10** | Interleukin-10 |
| **IL-11** | Interleukin-11 |
| **IL-12p40** | Interleukin-12 p40 |
| **IL-12p70** | Interleukin-12 p70 |
| **IL-13** | Interleukin-13 |
| **IL-15** | Interleukin-15 |
| **IL-16** | Interleukin-16 |
| **IL-17** | Interleukin-17 |
| **MCP-1/CCL2** | Monocyte chemotactic protein-1/chemokine (C-C motif) ligand 2 |
| **MCSF** | Mouse stem-cell factor |
| **MIG/CXCL9** | Monokine induced by IFN-Gamma/Chemokine (C-X-C motif) ligand 9 |
| **MIP 1 B-1a/CCL3** | Macrophage inflammatory protein 1 alpha/Chemokine (C-C motif) ligand 3 |
| **MIP 1 B-1b/CCL4** | Macrophage inflammatory protein 1 beta and/Chemokine (C-C motif) ligand 4 |
| **MIP 1 B-1d** | Macrophage inflammatory protein 1 delta |
| **PDGF-BB** | Platelet-derived growth factor BB |
| **Rantes/CCL5** | regulated on activation, normal T cell expressed and secreted/Chemokine (C-C motif) ligand 5 |
| **TIMP-1** | tissue inhibitor of metalloproteinases 1 |
| **TIMP-2** | tissue inhibitor of metalloproteinases 2 |
| **TNF a** | Tumor necrosis factor alpha |
| **TNF b** | Tumor necrosis factor beta |
| **TNF RI** | Tumor necrosis factor 1 |
| **TNF RII** | Tumor necrosis factor 2 |

**Table 7. Characteristics of the antibodies used in the study.**

| **Symbol of the growth factor** | **Name of the growth factor** |
|---|---|
| **AR** | Androgen receptor |
| **BNDF** | Brain-derived neurotrophic factor |
| **b FGF** | Basic fibroblast growth factor |
| **BMP4** | Bone morphogenetic protein 4 |
| **BMP5** | Bone morphogenetic protein 5 |
| **BMP7** | Bone morphogenetic protein 7 |
| **B-NGF** | Nerve growth factor beta |
| **EGF** | Epidermal growth factor |
| **EGFR** | Epidermal growth factor receptor |
| **EG-VEGF** | Endocrine-gland-derived vascular endothelial growth factor |
| **FGF 4** | Fibroblast growth factor 4 |
| **FGF 7** | Fibroblast growth factor 7 |
| **GDF-15** | Growth differentiation factor 15 |
| **GDNF** | Glial cell-derived neurotrophic factor |
| **GH** | Growth hormone |
| **HB-EGF** | Heparin-binding EGF-like growth factor |
| **HGF** | Hepatocyte growth factor |
| **IGFBP-1** | Insulin-like growth factor-binding protein 1 |
| **IGFBP-2** | Insulin-like growth factor-binding protein 2 |
| **IGFBP-3** | Insulin-like growth factor-binding protein 3 |
| **IGFBP-4** | Insulin-like growth factor-binding protein 4 |
| **IGFBP-6** | Insulin-like growth factor-binding protein 5 |
| **IGF-1** | Insulin-like growth factor 1 |
| **Insulin** | Insulin |
| **MCF R** | Macrophage chemotactic factor receptor |
| **NGF R** | Nerve growth-factor receptor |
| **NT-3** | Neurotrophin 3 |
| **NT-4** | Neurotrophin 4 |
| **OPG** | Osteoprotegerin |
| **PDGFAA** | Platelet-derived growth factor AA |
| **PIGF** | Phosphatidylinositol-glycan biosynthesis class F protein |
| **SCF** | Stem-cell factor |
| **SCF R** | Stem-cell-factor receptor |
| **TGFa** | Transforming growth factor alpha |
| **TGF b1** | Transforming growth factor beta 1 |
| **TGF b3** | Transforming growth factor beta 3 |
| **VEGF** | Vascular endothelial growth factor |
| **VEGF R2** | Vascular endothelial growth factor receptor 2 |
| **VEGF R3** | Vascular endothelial growth factor receptor 3 |
| **VEGF D** | Vascular endothelial growth factor D |

### Western blot

The proteins from the BAL (50 µg) were prepared as described above. Briefly, the SDS-PAGEs were transferred to PVDF membranes (Bio-Rad, Hercules, CA, USA). After blocking, the blots were incubated with the following primary antibodies: anti-IL11 and anti-CCL-1 (1:200 Abcam, Cambridge, MA, USA). The secondary antibodies were: anti-mouse conjugated to peroxidase (GE Healthcare, Uppsala, Sweden) and anti-rabbit (Cell Signaling, Beverly, MA, USA). The protein bands were developed using an increase of ECL chemoluminiscence (GE Healthcare, Uppsala, Sweden) and were visualized in an image analyzer (Mini LAS-3000, Fujifilm, Tokyo, Japan). The quantification of the expression levels was carried out by comparing the amount of β-actin protein (1:1000 Abcam, Cambridge, MA, USA). Densitometry was used in order to analyze the expression values of the proteins of interest obtained by Western Blot. The densitometry analysis of the scanned blots was carried out by using the Image J program (http://rsbweb.nih.gov/ij/) and the results were expressed as the relative changes with respect to the control protein (β-actin). The experiments were repeated three times independently.

### ELISA

The BAL samples were evaluated according to the manufacturer's instructions, using the ELISA sandwich kits for CCL-1/l-309 and IL-11 (DuoSet, R & D Systems, Minneapolis, Minnesota, USA). All the samples were assayed in duplicate and using the same plate. Briefly, the capture antibody (concentration as provided by the manufacturer) was diluted in PBS, was added to each well and was left overnight at 4 °C. The plate was washed 4 times in TBS with Tween-20 0.05 % (TBST 0.05 %). The plate was blocked with bovine serum albumin (BSA) 1 % for 1 hour at room temperature before being washed again four times with TBST 0.05 %. The samples and patterns were added to the plate and were incubated for 2 hours at room temperature. The plate was then washed, and the detection antibody diluted in PBS was added (concentration as provided by the manufacturer). The plate was incubated for 2 hours at room temperature. Once the plate was washed again, streptavidin (DuoSet, R & D Systems) was added and the plate was incubated for 30 minutes. Finally, the coloring reagent, o-phenylendiamine, was added to each well, and was allowed to react in the dark for 20 minutes. The reaction was quenched by adding H₂SO₄ 2N to each well. The absorbance was read on a plate reader at 450 nm (Emax, Molecular Devices, Minneapolis, Minnesota, USA).

### Statistical analysis

All continuous variables of patient characteristics were expressed as median values for each variable (interquartile range [IQR]) and categorical variables as the number of cases and percentage. The statistical analysis was carried out with the Statistical Package for the Social Sciences (SPSS 17, Chicago, Illinois, USA).

### Analysis of expression data from the protein arrays

The hierarchical cluster analysis was carried out using the UPGMA function (*Unweighted Pair Group Method with Arithmetic Mean*). Before the statistical analysis, the expression levels of the proteins were standardized, protein by protein, in all conditions, using the means and SD values. The conditions of the sample were clustered using Euclidian distance metric tests. The results were visualized and analyzed with Babelomics 4,2 (babelomics.bioinfo.cipf.es). The expression level of each protein, with respect to its mean expression levels under all the conditions, was represented by a color, where red represents higher expression than the median, blue represents lower expression than the median, and several intermediate color intensities represent the magnitude of the variance from the median.

### Analysis of validity of the diagnostic tests

The differences between two independent groups were analyzed by the U test of Mann-Whitney (continuous variables and non-parametric analysis). The ROC curves were constructed in order to evaluate the sensitivity, specificity and their respective areas under the curve (AUC) with a 95 % CI. The optimal cutoff value was investigated, which was chosen in order to reflect the best combination between sensitivity, specificity, positive predictive value, negative predictive value, probability ratio to predict a diagnosis of adenocarcinoma. In order to test the accuracy of the diagnosis when measuring both IL-11 and CCL-1, functions of the combined markers were estimated by binary logistic regression.

### Results

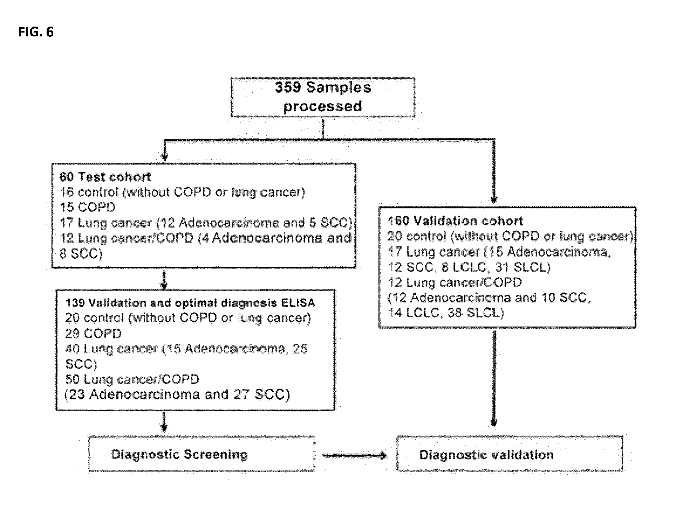

Generally, 359 patients were recruited, 60 in the screening cohort, 139 in the validation cohort and 160 in the additional validation cohort. The clinicopathological characteristics of the patients in the assay and validation cohorts are summarized in Figure 6.

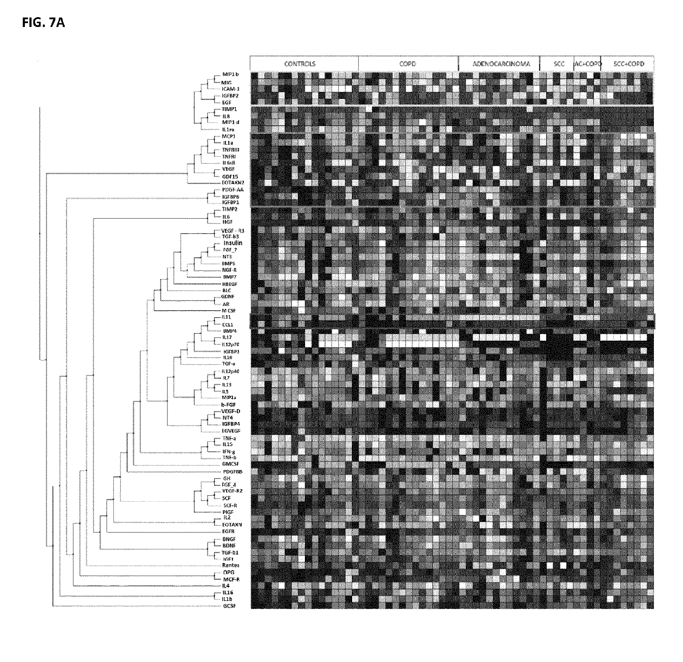

Firstly, the inflammatory protein expression profiles were analyzed using expression arrays, utilizing the BAL samples from patients in the test cohort. The characteristics of the patients were evenly distributed into four different groups: COPD, LC, both diseases or no disease (control group). The analysis revealed that a significant number (16) of the proteins (MIP1b, MIG, IGFBP2, IGFBP1, EGF, VEGF, TNFR-I, TNFR-II, IL6sR, GDF15, IL-1a, MCP-1, EOTAXIN2, PDGFAA, IL-11, and CCL-1) participating in the inflammatory pathways were overexpressed in the groups of patients with lung cancer and/or COPD compared to the control group. Interestingly, there were no clear differences in protein expression between the histological subtype of adenocarcinoma and the other remaining groups. More specifically, the expression of IL-11 and CCL-1 only appeared in the adenocarcinoma pathology (Figure 7A).

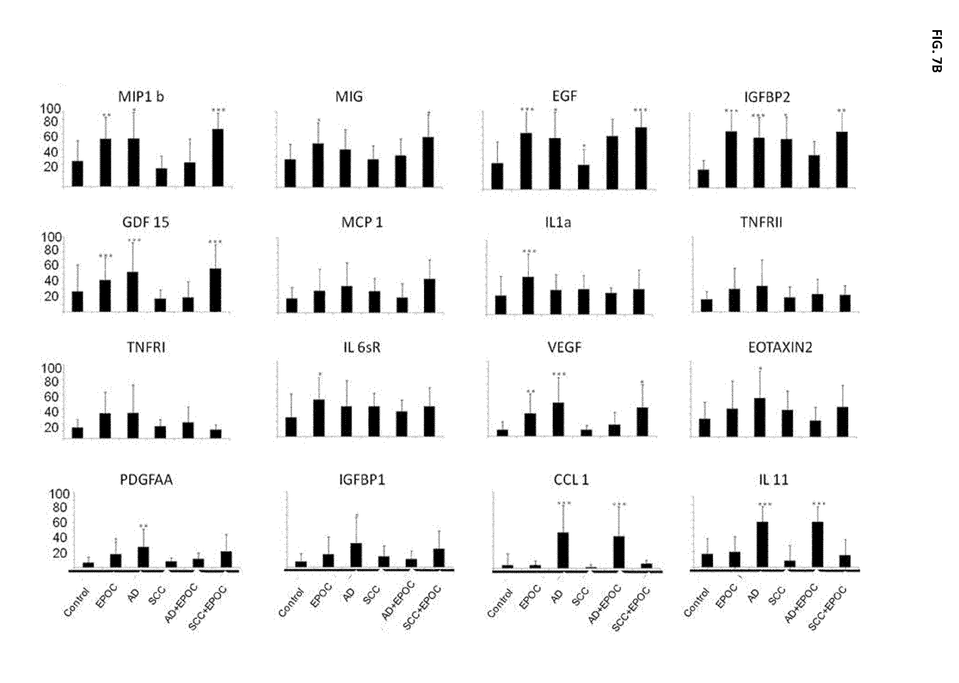

A formal statistical analysis (Figure 7B) confirmed the results previously obtained in the modelling heat map despite the dispersion present in the groups studied. Also, CCL-1 and IL-11 proteins showed statistically significant expression differences in patients with adenocarcinoma compared to the remaining groups of patients.

In order to validate the results observed in the protein arrays, a Western blot of the IL-11 and CCL-1 proteins exclusively associated with the adenocarinoma groups was carried out (Figure 8A). The results of the Western blot experiments indicated expression differences similar to those found in the heat map analysis.

In order to analyze the reliability of the profile obtained, the proteins previously identified were individually validated by ELISA methodology. This method was chosen for several reasons: validating the results with a different methodology, its high sensitivity and ease of clinical handling and use. The expression of IL-11 and CCL-1 was analyzed in two controlled independent cohorts. In the first validation cohorts, the 139 patients (Table 6) had similar clinicopathological characteristics to those of the test cohorts. On the other hand, the additional validation cohort of 160 patients (Table 7) differed from the assay cohort, given the inclusion of other lung cancer histological subtypes and the absence of a COPD group. The results demonstrated that IL-11 and CCL-1 were significantly overexpressed in patients with adenocarcinoma, compared to the remaining groups, in the first validation cohort (Figure 8B) and in the additional validation cohort (Figure 8C).

Subsequently, the diagnostic value for lung adenocarcinoma of both proteins was analyzed by ROC curves, in order to obtain a cutoff line which would enable classification of the patients into adenocarcinoma or no adenocarcinoma (Figure 9).

The ROC curves for the validation cohorts showed that the optimal cutoff value for IL-11 was 42 pg/ml (AUC: 0.935, CI 95 %: 0.896-0.975), with a 90 % sensitivity and an 86 % specificity (Figure 8A, Table 5A). The optimal cutoff value for CCL-1 was 39.5 pg/ml (AUC: 0.83, CI 95 %: 0.749-0.902), with a 83 % sensitivity and an 74 % specificity (Figure 8A, Table 5A). Also, the sensitivity and specificity of CCL-1 and IL-11 proteins as a whole and CCL-1 and/or IL-11 was analyzed (Table 8A). On the other hand, the ROC curves on the additional validation cohorts showed that the optimal cutoff value for the IL-11 diagnostic was 29.5 pg/ml (AUC: 0.95, CI 95 %: 0.92-0.98), with a 90.6 % sensitivity and an 83 % specificity (Figure 8B, Chart 1 b). The optimal cutoff value for CCL-1 was 24.25 pg/ml (AUC: 0.91, CI 95 %: 0.87-0.96), with a 91.7 % sensitivity and an 77.5 % specificity (Figure 8B, Chart 1 b). Also, the sensitivity and specificity of CCL-1 and IL-11 proteins as a whole and CCL-1 and/or IL-11 was analyzed for this cohort (Table 8B).

**Table 8.**

| **A.** Cohort validation test | | | | | | | |
|---|---|---|---|---|---|---|---|
| | **AUC (95 % CI)** | **Sensitivity (95% CI)** | **Specificity (95% CI)** | **PPV (95% CI)** | **NPV (95% CI)** | **Positive LR** | **Negative LR** |
| **IL**-**11** | 0.930 (0.896 - 0.975) | 90.2% (79.0 - 95.7 %) | 88.7% (80.6 - 93.5%) | 80.7% (68.7 - 88.9%) | 94.5% (87.8 - 97.6%) | 7.95 (4.53 - 13.98) | 0.11 (0.05 - 0.26) |
| **CCL-1** | 0.830 (0.794-0.902) | 80.0% (66.4 - 87.7%) | 74.1% (63.9 - 82.2%) | 72.1% (59.2 - 73.4%) | 86.3% (76.6 - 92.4%) | 3.02 (2.05 - 4.47) | 0.29 (0.17-0.52) |
| **IL-11 and CCL-1** | | 71.2% (57.7 - 81.7%) | 94.4% (88.4 - 97.4%) | 86.0% (72.7 - 93.4%) | 87.2% (79.9 - 92.1%) | 12.80 (5.77 - 28.41) | 0.31 (0.20 - 0.47) |
| **IL-11 and/or CCL-1** | | 94.3% (84.6-98.1%) | 74.1% (65.1 - 81.4%) | 64.1% (53.0 - 73.9%) | 96.4% (89.9 - 98.8%) | 3.64 (2.63 - 5.04) | 0.08 (0.03 - 0.23) |

| **B.** Additional cohort validation test | | | | | | | |
|---|---|---|---|---|---|---|---|
| | **AUC (95 % CI)** | **Sensitivity (95% CI)** | **Specificity (95% CI)** | **PPV (95% CI)** | **NPV (95% CI)** | **Positive LR** | **Negative LR** |
| **IL-11** | 0.95 (0.92 - 0.98) | 90.6% (79.7 - 95.9%) | 83.0% (86.8 - 87.7%) | 60.8% (49.7 - 70.8%) | 96.8% (92.7 - 98.6%) | 5.32 (3.81 - 7.41) | 0.11 (0.05 - 0.26) |
| **CCL-1** | 0.91 (0.87 - 0.96) | 91.7% (80.4 - 96.7%) | 77.5% (71.0 - 82.9%) | 51.2% (40.8 - 61.4%) | 97.3% (93.3 - 99.0%) | 4.08 (3.09 - 5.04) | 0.11 (0.04 - 0.28) |
| **IL-11 and CCL-1** | | 71.2% (57.7-81.7%) | 96.3% (92.5 - 98.2%) | 84.1% (70.6 - 92.1%) | 92.3% (87.7 - 95.3%) | 19.10 (9.00 - 41.13) | 0.30 (0.19-0.46) |
| **IL-11 and/or CCL-1** | | 92.3% (82.6 - 98.1%) | 84.0% (78.0 - 88.5%) | 62.5% (51.5 - 72.3%) | 98.1% (94.6 - 99.4%) | 5.88 (4.21 - 8.22) | 0.07 (0.02 - 0.20) |

| **C.** Validation of the first cohort in the second cutoff | | | | | | | |
|---|---|---|---|---|---|---|---|
| | **Sensitivity (95% CI)** | | **Specificity (95% CI)** | **PPV (95% CI)** | **NPV (95% CI)** | **Positive LR** | **Negative LR** |
| **IL-11** | 90.2% (79.7-95.9%) | | 91.4% (86.3 - 94.7%) | 75.4% (63.3 - 84.5%) | 97.0% (93.1 - 98.7%) | 10.52 (6.43 - 17.22) | 0.11 (0.05 - 0.25) |
| **CCL-1** | 78.3% (64.4 - 87.7%) | 85.4% (79.1 - 90.1%) | 61.0% (48.3 - 72.4%) | 93.1 (87.8 - 96.2%) | 5.38 (3.58 - 8.08) | 0.25 (0.15-0.44) | |
| **IL-11 and CCL-1** | 71.2% (57.7 - 81.7%) | 96.6% (92.8 - 98.4%) | 86.0% (72.7 - 93.4%) | 91.9% (87.1 - 95.0%) | 20.87 (9.33 - 46.69) | 0.30 (0.19-0.46) | |
| **IL-11 and/or CCL-1** | 93.0% (81.4-97.6%) | 77.8% (71.0-83.5%) | 51.9% (41.0 - 62.7%) | 97.7% (93.6 - 99.2%) | 4.20 (3.12-5.64) | 0.09 (0.03 - 0.27) | |

| | | | | | | | |
|---|---|---|---|---|---|---|---|
| AUC= area under the curve. PPV= positive predictive value. NPV= negative predictive value. LR= likelihood ratio. | | | | | | | |

As sensitivity and specificity were similar in both groups, a binary logistic regression was carried out in order to analyze the validity of the cutoff values obtained in the previous analysis. The cutoff value obtained in the first validation cohort had a diagnostic capacity for adenocarcinoma of 78.8 % and 81 % of patients with IL-11 and CCL-1, respectively (Figure 10A). This capacity was increased up to 90 % and 91 % in patients with IL-11 and CCL-1, respectively, in the additional validation cohort, detecting up to 96 % of patients when both biomarkers were used (Figure 10B). 40 pg/ml and 39.5 pg/ml were chosen as cutoff values for adenocarcinoma. The predictive values and probability ratios in the diagnosis of adenocarcinoma are shown in Table 9. Meanwhile, the differential diagnostic precision was analyzed by joining the two validation cohorts. Then, the AUC, sensitivity, specificity, VPP, VPN and the relationships in the initial stages, ex-smokers, packs smoked per year, number of cells in the BAL and affectation of the bronchial tract were analyzed (Table 9). The ROC analyses demonstrated that the tests for IL-11 and CCL-1 increased the accuracy of the early stage adenocarcinoma diagnosis; the AUC of IL-11 was 0.95 (CI 95 %: 0.91-0.99) with 100 % sensitivity and 93 % specificity, and the AUC of CCL-1 was 0.93 (CI 95 %: 0.85-1), with 91.7 % sensitivity and 81.6 % specificity (Table 9). The AUC, sensitivity and specificity of both proteins in adenocarcinoma were also proven to be higher in ex-smokers, and increased with packets smoked per year (> 30 p; Table 9B, 9C).

**Table 9.**

| **A.** Early stages | | | | | | | |
|---|---|---|---|---|---|---|---|
| | **AUC (95% CI)** | **Sensitivity (95% CI)** | **Specificity (95% CI)** | **PPV (95% CI)** | **NPV (95% CI)** | **Positive LR** | **Negative LR** |
| **IL-11** | 0.95 (0.91 - 0.99) | 100.0% (83.9-100.0%) | 93.0% (81.4-97.6%) | 87.0% (67.9 - 95.5%) | 100.0% (91.2-100.0%) | 14.33 (4.81 - 42.69) | 0 |
| **CCL-1** | 0.93 (0.85-1.00) | 91.7% (64.6-98.5%) | 81.6% (66.6 - 90.8%) | 61.1% (38.6 - 79.7%) | 96.9% (84.3 - 99.4%) | 4.98 (2.49 - 9.93) | 0.10 (0.02 - 0.68) |
| **IL-11 and** | | 76.9% | 95.3% | 83.3% | 93.2% | 16.54 | 0.24 |
| **CCL-1** | | (49.7-91.8%) | (84.5 - 98.7%) | (55.2 - 95.3%) | (81.8-97.7%) | (4,14 - 86,11) | (0.09 - 0.66) |
| **IL-11 and/or CCL-1** | | 100.0% (77.2-100.0%) | 69.8% (54.9 - 81.4%) | 50.0% (32.1 - 67.9%) | 100.0% (88.6 - 100.0%) | 3.31 (2.10-5.21) | 0 |

| **B. Ex-smokers** | | | | | | | |
|---|---|---|---|---|---|---|---|
| | **AUC (95% CI)** | **Sensitivity (95% CI)** | **Specificity (95% CI)** | **PPV (95% CI)** | **NPV (95% CI)** | **Positive LR** | **Negative LR** |
| **IL-11** | 0.97 (0.95-1.00) | 100.0% (81.6-100.0%) | 93.2% (85.1 - 97.1%) | 77.3% (56.6 - 89.9%) | 100.0% (94.7 - 100.0%) | 14.80 (6.35 - 35.40) | 0 |
| **CCL-1** | 0.97 (0.94-1.00) | 88.9% (67.2-96.2%) | 87.8% (78.5 - 93.5%) | 64.0% (44.5 - 79.8%) | 97.0% (89.8 - 99.2%) | 7.31 (3.88 - 13.77) | 0.13 (0.03 - 0.47) |
| **IL-11 and CCL-1** | | 94.1% (73.0 - 99.0%) | 92.6% (82.4 - 97.1%) | 80.0% (58.4 - 91.9%) | 98.0% (89.7 - 99.7%) | 12.71 (4.91 - 32.87) | 0.06 (0.01 - 0.43) |
| **IL-11 and/or CCL-1** | | 100.0% (81.6 - 100.0%) | 85.0% (75.6 - 91.2%) | 58.6% (40.7 - 75.4%) | 100.0% (94.7 - 100.0%) | 6.67 (3.96 - 11.23) | 0 |

| **C.** Smokers of less than 30 packs per year | | | | | | | |
|---|---|---|---|---|---|---|---|
| | **AUC (95% CI)** | **Sensitivity (95% CI)** | **Specificity (95% CI)** | **PPV (95% CI)** | **NPV (95% CI)** | **Positive LR** | **Negative LR** |
| **IL-11** | 0.94 (0.90 - 0.98) | 89.5% (68.6 - 97.1%) | 96.8% (83.8 - 99.4%) | 94.4% (74.2 - 99.0%) | 93.8% (79.9 - 98.3%) | 27.74 (4.01 - 191.91) | 0.11 (0.03 - 0.40) |
| **CCL-1** | 0.90 (0.84 - 0.97) | 92.3% (66.7 - 96.6%) | 96.6% (82.8 - 99.4%) | 92.3% (66.7 - 98.6%) | 96.6% (82.8 - 99.4%) | 26.77 (3.88 - 184.85) | 0.08 (0.01 - 0.53) |
| **IL-11 and CCL-1** | | 92.3% (66.7 - 98.6%) | 95.8% (79.8 - 99.3%) | 92.3% (66.7 - 98.6%) | 95.8% (79.8 - 99.3%) | 22.15 (3.23 - 15.189) | 0.08 (0.01 - 0.53) |
| **IL-11 and/or CCL-1** | | 100.0% (77.2 - 100.0%) | 83.9% (67.4 - 92.9%) | 72.2% (49.1 - 87.5%) | 100.0% (87.1 - 100.0%) | 6.20 (2.78 - 13.84) | 0 |

The comparative analyses of number of cells in BAL from patients with adenocarcinoma demonstrated that the patients with lower cell number than the median exhibited similar AUC, sensitivity and specificity results than patients above the median cell number (Table 10).

**Table 10.**

| **A.** Number of cells per million above the median | | | | | | | |
|---|---|---|---|---|---|---|---|
| | **AUC (95% CI)** | **Sensitivity (95% CI)** | **Specificity (95% CI)** | **PPV (95% CI)** | **NPV (95% CI)** | **Positive LR** | **Negative LR** |
| **IL**-**11** | 0.90 (0.83 - 0.96) | 91.7% (74.2 - 97.7%) | 95.5% (84.9 - 98.7%) | 91.7% (74.2 - 97.7%) | 95.5% (84.9 - 98.7%) | 20.17 (5.18-78.53) | 0.09 (0.02 - 0.13) |
| **CCL-1** | 0.91 (0.86 - 0.96) | 86.4% (66.7 - 95.3%) | 85.7% (74.3 - 92.6%) | 70.4% (51.5-84.1%) | 94.1% (84.1 - 98.0%) | 6.05 (3.12-11.73) | 0.16 (0.05 - 0.46) |
| **IL**-**11 and CCL-1** | | 87.0% (67.9 - 95.5%) | 100.0% (92.6 - 100.0%) | 100.0% (83.9 - 100.0%) | 94.1% (84.0 - 98.0%) | 0 | 0.13 (0.05 - 0.37) |
| **IL-11 and/or** | | 100.0% | 84.9% | 75.0% | 100.0% | 6.63 | 0 |
| **CCL-1** | | (86.2 - 100.0%) | (72.9 - 92.1%) | (57.9 - 86.7%) | (92.1 - 100.0%) | (3.50 - 12.55) | |

| **B.** Number of cells per million above the median | | | | | | | |
|---|---|---|---|---|---|---|---|
| | **AUC (95% CI)** | **Sensitivity (95% CI)** | **Sensitivity (95% CI)** | **PPV (95% CI)** | **NPV (95% CI)** | **Positive LR** | **Negative LR** |
| **IL-11** | 0.90 (0.84 - 0.98) | 86.4% (66.7-95.3%) | 86.8% (75.2 - 93.5%) | 73.1% (53.9 - 86.3%) | 93.9% (83.5 - 97.9%) | 6.54 (3.22 - 13.30) | 0.16 (0.05 - 0.45) |
| **CCL-1** | 0.91 (0.85 - 0.96) | 75.0% (53.1 - 88.8%) | 82.9% (67.3 - 91.9%) | 71.4% (50.0 - 86.2%) | 85.3% (69.9 - 93.6%) | 4.38 (2.02 - 9.46) | 0.30 (0.14-0.66) |
| **IL-11 and CCL-1** | | 80.0% (58.4 - 91.9%) | 98.1% (89.9 - 99.7%) | 94.1% (73.0 - 99.0%) | 92.7% (82.7 - 97.1%) | 41.60 (5.90 - 293.39) | 0.20 (0.08 - 0.49) |
| **IL-11 and/or CCL-1** | | 90.5% (71.1 - 97.3%) | 73.6% (60.4 - 83.6%) | 57.6% (40.8 - 72.8%) | 95.1% (83.9 - 98.7%) | 3.43 (2.14-5.84) | 0.13 (0.03 - 0.49) |

With respect to tumor location, there were no differences between patients. The patients with proximal location adenocarcinoma exhibited better AUC, sensitivity and specificity than those with distal location. The AUC for **IL-11** in patients with proximal location was 0.98 (CI 95 %: 0.96-1) with 100 % sensitivity and 91.4 % specificity, whereas the AUC for **IL-11** in patients with distal location was 0.92 (CI 95 %: 0.87-0.94), with 89.3 % sensitivity and 91.5 % specificity. Similar results were obtained for CCL-1 (Table 11).

**Table 11.**

| **A.** Proximal location | | | | | | | |
|---|---|---|---|---|---|---|---|
| | **AUC (95% CI)** | **Sensitivity (95% CI)** | **Specificity (95% CI)** | **PPV (95% CI)** | **NPV (95% CI)** | **Positive LR** | **Negative LR** |
| **IL-11** | 0.92 (0.88 - 0.94) | 100.0% (85.7-100.0%) | 91.4% (86.3 - 94.7%) | 60.5% (44.7-74.4%) | 100.0% (97.6 - 100.0%) | 11.60 (7.15-18.82) | 0 |
| **CCL-1** | 0.89 (0.83 - 0.96) | 100.0% (85.7 - 100.0%) | 82.4% (75.0 - 88.0%) | 50.0% (36.1 - 63.9%) | 100.0% (96.6 - 100.0%) | 5.70 (3.93 - 8.25) | 0 |
| **IL-11 and CCL-1** | | 82.6% (62.9 - 93.0%) | 96.2% (92.4 - 98.2%) | 73.1% (53.9 - 86.3%) | 97.8% (94.5 - 99.1%) | 21.95 (10.36-46.49) | 0.18 (0.17-0.44) |
| **IL-11 and/or CCL-1** | | 100.0% (85.7 - 100.0%) | 78.5% (72.0 - 88.8%) | 36.5% (25.7 - 48.9%) | 100.0% (97.4 - 100.0%) | 4.65 (3.53 - 12.00) | 0 |

| **B.** Distal location | | | | | | | |
|---|---|---|---|---|---|---|---|
| | **AUC (95% CI)** | **Sensitivity (95% CI)** | **Specificity (95% CI)** | **PPV (95% CI)** | **NPV (95% CI)** | **Positive LR** | **Negative LR** |
| **IL-11** | 0.98 (0.96 - 1.00) | 89.3% (72.8 - 96.3%) | 91.4% (86.3 - 94.7%) | 62.5% (47.0 - 66.3%) | 98.1% (94.7 - 99.4%) | 10.36 (6.28 - 17.08) | 0.12 (0.04 - 0.37) |
| **CCL-1** | 0.94 (0.88 - 0.98) | 89.7% (73.6 - 96.4%) | 82.4% (75.0 - 88.0%) | 53.1% (39.4 - 66.3%) | 97.3% (92.4 - 99.1%) | 5.11 (3.45 - 7.55) | 0.13 (0.04 - 0.37) |
| **IL-11 and CCL-1** | | 67.9% (49.3 - 82.1%) | 96.2% (92.4 - 98.2%) | 73.1% (53.9 - 86.2%) | 95.2% (91.2 - 97.5%) | 18.03 (8.35 - 38.95) | 0.33 (0.19 - 0.58) |
| **IL-11 and/or CCL-1** | | 92.6% (76.6 - 97.9%) | 78.5% (72.0 - 83.8%) | 38.5% (27.6 - 50.6%) | 98.6% (95.2 - 99.6%) | 4.31 (3.25 - 5.78) | 0.09 (0.02 - 0.36) |

Finally, the direct and indirect signal and the signal without alterations were analyzed in patients with adenocarcinoma. The diagnostic accuracy in these groups did not show important differences between their AUC, sensitivity and specificity (Table 12).

**Table 12.**

| **A. Direct signal** | | | | | | | |
|---|---|---|---|---|---|---|---|
| | **AUC (95% CI)** | **Sensitivity (95% CI)** | **Specificity (95% CI)** | **PPV (95% CI)** | **NPV (95% CI)** | **Positive LR** | **Negative LR** |
| **IL-11** | 0.94 (0.89 - 0.99) | 93.3% (70.2 - 98.8%) | 91.4% (86.3 - 94.7%) | 48.3% (31.4 - 65.6%) | 99.4% (96.5 - 99.9%) | 10.83 (6.55 - 17.89) | 0.07 (0.01 - 0.49) |
| **CCL-1** | 0.90 (0.84 - 0.96) | 81.8% (52.3 - 94.9%) | 82.4% (75.0 - 88.0%) | 28.1% (15.6-45.4%) | 98.2% (93.6-99.5%) | 4.66 (2.93 - 7.41) | 0.22 (0.06 - 0.78) |
| **IL-11 and CCL-1** | | 62.5% (38.6 - 81.5%) | 96.2% (92.4 - 98.2%) | 58.8% (36.0 - 78.4%) | 96.8% (93.1 - 98.5%) | 16.61 (7.32 - 37.70) | 0.39 (0.21 - 0.74) |
| **IL-11 and/or CCL-1** | | 100.0% (80.6 - 100.0%) | 78.5% (72.0 - 83.8%) | 23.1% (13.7-36.01%) | 98.6% (95.2-99.6%) | 3.99 (2.81 - 5.64) | 0.18 (0.05 - 0.66) |

| **B. Indirect signal** | | | | | | | |
|---|---|---|---|---|---|---|---|
| | **AUC (95% CI)** | **Sensitivity (95% CI)** | **Specificity (95% CI)** | **PPV (95% CI)** | **NPV (95% CI)** | **Positive LR** | **Negative LR** |
| **IL-11** | 0.95 (0.89 - 0.99) | 85.7% (60.1 - 96.0%) | 91.4% (86.3 - 94.7%) | 44.4% (27.6 - 62.7%) | 98.8% (95.6 - 99.7%) | 9.94 (5.86 - 16.87) | 0.16 (0.04 - 0.57) |
| **CCL-1** | 0.89 (0.78 - 1.00) | 78.6% (52.4 - 92.4%) | 82.4% (75.0 - 88.0%) | 32.4% (19.1 - 49.2%) | 97.3% (92.4-99.1%) | 4.48 (2.82 - 7.10) | 0.26 (0.09 - 0.72) |
| **IL-11 and CCL-1** | | 80.0% (54.8 - 93.0%) | 96.2% (92.4 - 98.2%) | 63.2% (41.0-80.9%) | 98.4% (95.3-94.4%) | 21.26 (9.85 - 45.89) | 0.21 (0.08 - 0.57) |
| **IL-11 and/or CCL-1** | | 85.7% (60.1 - 96.0%) | 78.5% (72.0 - 83.8%) | 23.1% (13.7 - 36.1%) | 98.6% (95.2 - 99.6%) | 3.99 (2.81 - 5.64) | 0.18 (0.05 - 0.66) |

| **C. Signal without abnormalities** | | | | | | | |
|---|---|---|---|---|---|---|---|
| | **AUC (95% CI)** | **Sensitivity (95% CI)** | **Specificity (95% CI)** | **PPV (95% CI)** | **NPV (95% CI)** | **Positive LR** | **Negative LR** |
| **IL-11** | 0.98 (0.95 - 1.00) | 95.0% (76.4 - 99.1%) | 91.4% (86.3 - 94.7%) | 55.9% (39.5 - 71.1%) | 99.4% (96.5 - 99.9%) | 11.02 (6.72 - 18.03) | 0.05 (0.01 - 0.37) |
| **CCL-1** | 0.93 (0.88 - 0.98) | 81.0% (60.0 - 92.3%) | 82.4% (75.0 - 88.0%) | 42.5% (28.5-57.8%) | 96.4% (91.2 - 98.6%) | 4.61 (3.01 - 7.05) | 0.23 (0.09 - 0.56) |
| **IL-11 and CCL-1** | | 85.0% (64.0 - 94.8%) | 96.2% (92.4 - 98.2%) | 70.8% (50.8-85.1%) | 98.4% (95.3 - 99.4%) | 22.59 (10.67 - 24.78) | 0.16 (0.05 - 0.44) |
| **IL-11 and/or CCL-1** | | 100.0% (84.5 - 100.0%) | 78.5% (72.0 - 83.8%) | 34.4% (23.7 - 47.0%) | 100.0% (97.4 - 100.0%) | 4.65 (3.53 - 6.12) | 0 |

### Conclusion

Several non-invasive lung cancer screening techniques have been studied. The imaging techniques, such as thoracic radiography, low-dose spiral computed tomography, sputum cytology and molecular biomarkers in different biological samples, have been investigated to determine their diagnostic value for early detection of lung cancer (Patz et al., 2010. J Thorac Oncol 5, 1502-1506; Hoffman et al., 2000, Lancet 355, 479-485). Although these tests vary in their sensitivity and specificity, only low-dose computed tomography of the thorax has been shown to reduce lung-cancer-specific mortality (Manser et al., 2003. Thorax 58, 784-789; Manser et al., 2004. Cochrane Database Syst Rev CD001991). The present invention localizes protein markers involved in inflammation, which participate in the pathogenesis of the two most frequent and devastating respiratory diseases associated with smokers, lung cancer and COPD.

In the validation study, and using similar control groups, the ROC 20 curves showed that the optimal cutoff levels for the diagnosis in BAL were 42 pg/ml for IL-11 and 39.5 pg/ml for CCL-1. Diagnostic precision for adenocarcinoma was confirmed for each biomarker. There was even a positive correlation between IL-11 and CCL-1 levels in BAL; the measurement of both proteins optimized the sensitivity and specificity of the diagnosis up to levels of 90 % and 89 %, respectively. Surprisingly, both proteins were similarly predictive of adenocarcinoma, without concurrent COPD being present. The results of the present invention strongly suggest differences at the inflammatory level between both histological subtypes (SCC and adenocarcinoma).

Also, there is a correlation between elevated expression levels of IL-11 and cell proliferation, invasiveness, metastasis and poor prognosis.

CCL-1 acts as a potent chemoatractant for monocytes and lymphocytes and is thought to play an important role in inflammatory processes (Harpel et al., 2002. Isr. Med. Assoc. J. 4, 1025-1027). Recently, multiple-reaction monitoring, a high-performance liquid chromatography following the method of tandem mass spectrometry, may enable validation of biomarkers. The function of these proteins in the differential diagnosis of pleural effusions (for example, lung adenocarcinoma, mesothelioma and adenocarcinoma from other sources and non-tumor) would also be of value.

The present invention indicates that the determination of the levels of CCL-1 and IL-11 in the BAL samples by means of an easy assay, such as ELISA, may improve the diagnosis of lung adenocarcinoma in high-risk smokers, despite the presence of absence of COPD.

## Claims

1. The simultaneous use of the cytokines and growth factors selected from the list consisting of IGFBP1, MIP1β, CCL-1, MIG, PDGFAA, GDF-15, VEGF and EGF, for the prediction, diagnosis, prognosis and classification of individuals into:
a) individuals without COPD or lung cancer,
b) individuals with COPD,
c) individuals with adenocarcinoma,
d) individuals with squamous carcinoma,
e) individuals with COPD and adenocarcinoma, or
f) individuals with COPD and squamous carcinoma.

2. The use according to the previous claim, further comprising the cytokines and growth factors IL-6sR, IL-1a, IL-11, EOTAXIN-2, TNFRI, TNFRII, IGFBP2, MCP1 and GDF-15.

3. The use of IL-11 and CCL-1 according to claim 2 for predicting or prognosticating, or for early diagnosis of lung adenocarcinoma in an individual.

4. The use of IL-11 and CCL-1, according to the previous claim, wherein the individual is a smoker.

5. A method for obtaining useful data for the diagnosis, prognosis and classification of the individuals into a) individuals without COPD or lung cancer, b) individuals with COPD, c) individuals with adenocarcinoma, d) individuals with COPD and adenocarcinoma, or e) individuals with COPD and squamous carcinoma, comprising:
i) obtaining an isolated biological sample from an individual, and
ii) quantifying the expression products of cytokines and growth factors selected from the list consisting of: *IGFBP1, MIP1β*, *CCL-1, MIG*, *PDGFAA, GDF-15, VEGF and EGF.*

6. The method according to the previous claim, further comprising:
iii) comparing the amounts obtained in step (ii) to a reference amount.

7. The method according to any of the claims 5-6, further comprising quantifying the expression products of the cytokines and growth factors IL-6sR, IL-1a, IL-11, EOTAXIN-2, TNFRI, TNFRII, IGFBP2, and MCP-1.

8. The method according to any of the claims 5-7, wherein steps (ii) and/or (iii) of the methods described above may be totally or partially automated.

9. The method according to any of the claims 5-8, wherein the biological sample is bronchoalveolar fluid.

10. The method according to any of the claims 5-9, wherein the quantification is performed by means of an immunoassay.

11. The method according to any of the claims 5-10, wherein the immunoassay is an ELISA.

12. A method for the diagnosis, prognosis and classification of individuals comprising steps (i)-(iii) according to any of the claims 5-11, further comprising assigning an individual from step (i) to the group of individuals without COPD or lung cancer when the individual does not exhibit expression of the *IGFBP1, MIP1β*, *CCL-1, MIG* and *PDGFAA* genes.

13. A method for the diagnosis, prognosis and classification of individuals comprising steps (i)-(iii) according to any of the claims 5-12, further comprising assigning the individual from step (i) to the group of individuals with adenocarcinoma, when the individual exhibits an increase in the expression product of the *IL-11* and/or *CCL-1* genes relative to a reference amount.

14. A method for the diagnosis, prognosis and classification of individuals, comprising the steps (i)-(iii) according to any of the claims 5-13, further comprising assigning the individual from step (i) to the group of individuals with COPD when the expression of *MIG* is detected and the expression of *CCL-1* and *IGFBP1* is not detected.

15. A method for the diagnosis, prognosis and classification of individuals, comprising steps (i)-(iii) according to any of the claims 5-14, further comprising assigning the individual from step (i) to the group of individuals with adenocarcinoma when the expression of *CCL-1* is detected and the amount of expression of *MIP1β* is lower than 20 pg/ml.

16. A method for the diagnosis, prognosis and classification of individuals, comprising the steps (i)-(iii) according to any of the claims 5-15, further comprising assigning the individual from step (i) to the group of individuals with squamous carcinoma when any expression level of *PDGFAA* or *MIP1β* is detected and the expression of *CCL-1* is not detected.

17. A method for the diagnosis, prognosis and classification of individuals, comprising steps (i)-(iii) according to any of the claims 5-16, further comprising assigning the individual from step (i) to the group of individuals with COPD and adenocarcinoma when any expression level of *CCL-1* is detected and the expression of *VEGF* is lower than 200 pg/ml.

18. A method for the diagnosis, prognosis and classification of individuals, comprising steps (i)-(iii) according to any of the claims 5-17, further comprising assigning the individual from step (i) to the group of individuals with COPD and squamous carcinoma when the expression of *GDF-15* and of *VEGF* is detected above 50 pg/ml and 200 pg/ml, respectively, and no expression of *CCL-1* or *EGF* is detected.

19. The use of a pharmaceutical composition comprising an active principle selected among a β2 agonist, an anticholinergic, a compound of the group of corticosteroids, a phosphodiesterase inhibitor and an immune system suppressor, in the manufacture of a medicament for the treatment of an individual with COPD identifiable by the method according to any of the claims 5-18.

20. The use of a pharmaceutical composition comprising an active principle selected among platinum coordination complexes (cisplatin or carboplatin), gemcitabine, paclitaxel, docetaxel, etoposide, vinorelbin, pemetrexed, gefitinib, erlotinib, bevacizumab or any combinations thereof, in the manufacture of a medicament for the treatment of an individual with adenocarcinoma and squamous carcinoma associated or not with COPD, identifiable by the method according to any of the claims 5-18.

21. A kit or device, hereinafter the kit of the invention, comprising the necessary elements to quantify the expression product of the cytokines and growth factors selected among *IGFBP1, MIP1β*, *CCL-1, MIG*, *PDGFAA, GDF-15, VEGF* and *EGF.*

22. The kit or device according to the previous claim, further comprising the necessary tools to quantify the expression product of the cytokines and growth factors *IL-6sR, IL-1a, IL-11, EOTAXIN-2, TNFRI, TNFRII, IGFBP2, and MCP1.*

23. The kit or device according to any of the claims 21-22, comprising the necessary tools to quantify the expression product of the cytokines and growth factors CCL-1 and/or IL-11.

24. The kit or device according to any of the claims 21-23, comprising the anti-CCL-1 and anti-IL-11 antibodies.

25. The kit or device according to any of the claims 21-24, comprising the anti-IGFBP1, anti-MIP-1B, anti-CCL-1, anti-MIG, anti-PDGFAA, anti-GDF-15, anti-VEGF or anti-EGF antibodies.

26. The kit or device according to claim 25, further comprising the anti-IL-6sR, anti-IL-1a, anti-IL-11, anti-EOTAXIN-2, anti-TNFRI, anti-TNFRII, anti-IGFBP2, and anti-MCP1 antibodies.

27. The use of a kit or device according to any of the claims 21-26 to carry out a method as described in any of the claims 5-18.

28. A computer-readable storage means comprising program instructions capable of making a computer perform the steps of the method according to any of the claims 5-18.

29. The storage means according to the previous claim, comprising the anti-IGFBP1, anti-MIP-1B, anti-CCL-1, anti-MIG, anti-PDGFAA, anti-GDF-15, anti-VEGF or anti-EGF antibodies.

30. The storage means according to claim 29, further comprising the anti-IL-6sR, anti-IL-1a, anti-IL-11, anti-EOTAXIN-2, anti-TNFRI, anti-TNFRII, anti-IGFBP2, and anti-MCP1 antibodies.

31. The storage means according to claim 28, comprising oligonucleotides or single-channel microarrays designed from a known sequence or an mRNA of the *IGFBP1, MIP1β, CCL-1, MIG*, *PDGFAA, GDF-15, VEGF* and *EGF* genes.

32. The storage means according to claim 31, comprising oligonucleotides or single-channel microarrays designed from a known sequence or an mRNA of the *IL-6sR, IL-1a, IL-11, EOTAXIN-2, TNFRI, TNFRII, IGFBP2,* and *MCP1* genes.

33. A transmissible signal comprising program instructions capable of making a computer perform the steps of the method according to any of the claims 5-18.


SEQUENCE LISTING

<110> Servicio Andaluz de Salud
<110> Universidad de Sevilla
<110> Consejo Superior de Investigaciones Cientificas

<120> Method for obtaining useful data for the diagnosis, prognosis and
classification of patients with chronic obstructive pulmonary disease (COPD)
and/or lung cancer

<130> PCT-06449

<160> 32

<170> PatentIn version 3.5

<210> 1
<211> 5928
<212> DNA
<213> Homo sapiens

<400> 1

ggcggtcccc tgttctcccc gctcaggtgc ggcgctgtgg caggaagcca ccccctcggt 60

cggccggtgc gcggggctgt tgcgccatcc gctccggctt tcgtaaccgc accctgggac 120

ggcccagaga cgctccagcg cgagttcctc aaatgttttc ctgcgttgcc aggaccgtcc 180

gccgctctga gtcatgtgcg agtgggaagt cgcactgaca ctgagccggg ccagagggag 240

aggagccgag cgcggcgcgg ggccgaggga ctcgcagtgt gtgtagagag ccgggctcct 300

gcggatgggg gctgcccccg gggcctgagc ccgcctgccc gcccaccgcc ccgccccgcc 360

cctgccaccc ctgccgcccg gttcccatta gcctgtccgc ctctgcggga ccatggagtg 420

gtagccgagg aggaagcatg ctggccgtcg gctgcgcgct gctggctgcc ctgctggccg 480

cgccgggagc ggcgctggcc ccaaggcgct gccctgcgca ggaggtggcg agaggcgtgc 540

tgaccagtct gccaggagac agcgtgactc tgacctgccc gggggtagag ccggaagaca 600

atgccactgt tcactgggtg ctcaggaagc cggctgcagg ctcccacccc agcagatggg 660

ctggcatggg aaggaggctg ctgctgaggt cggtgcagct ccacgactct ggaaactatt 720

catgctaccg ggccggccgc ccagctggga ctgtgcactt gctggtggat gttccccccg 780

aggagcccca gctctcctgc ttccggaaga gccccctcag caatgttgtt tgtgagtggg 840

gtcctcggag caccccatcc ctgacgacaa aggctgtgct cttggtgagg aagtttcaga 900

acagtccggc cgaagacttc caggagccgt gccagtattc ccaggagtcc cagaagttct 960

cctgccagtt agcagtcccg gagggagaca gctctttcta catagtgtcc atgtgcgtcg 1020

ccagtagtgt cgggagcaag ttcagcaaaa ctcaaacctt tcagggttgt ggaatcttgc 1080

agcctgatcc gcctgccaac atcacagtca ctgccgtggc cagaaacccc cgctggctca 1140

gtgtcacctg gcaagacccc cactcctgga actcatcttt ctacagacta cggtttgagc 1200

tcagatatcg ggctgaacgg tcaaagacat tcacaacatg gatggtcaag gacctccagc 1260
atcactgtgt catccacgac gcctggagcg gcctgaggca cgtggtgcag cttcgtgccc 1320
aggaggagtt cgggcaaggc gagtggagcg agtggagccc ggaggccatg ggcacgcctt 1380
ggacagaatc caggagtcct ccagctgaga acgaggtgtc cacccccatg caggcactta 1440
ctactaataa agacgatgat aatattctct tcagagattc tgcaaatgcg acaagcctcc 1500
cagtgcaaga ttcttcttca gtaccactgc ccacattcct ggttgctgga gggagcctgg 1560
ccttcggaac gctcctctgc attgccattg ttctgaggtt caagaagacg tggaagctgc 1620
gggctctgaa ggaaggcaag acaagcatgc atccgccgta ctctttgggg cagctggtcc 1680
cggagaggcc tcgacccacc ccagtgcttg ttcctctcat ctccccaccg gtgtccccca 1740
gcagcctggg gtctgacaat acctcgagcc acaaccgacc agatgccagg gacccacgga 1800
gcccttatga catcagcaat acagactact tcttccccag atagctggct gggtggcacc 1860
agcagcctgg accctgtgga tgataaaaca caaacgggct cagcaaaaga tgcttctcac 1920
tgccatgcca gcttatctca ggggtgtgcg gcctttggct tcacggaaga gccttgcgga 1980
aggttctacg ccaggggaaa atcagcctgc tccagctgtt cagctggttg aggtttcaaa 2040
cctccctttc caaatgccca gcttaaaggg gctagagtga acttgggcca ctgtgaagag 2100
aaccatatca agactctttg gacactcaca cggacactca aaagctgggc aggttggtgg 2160
gggcctcggt gtggagaagc ggctggcagc ccacccctca acacctctgc acaagctgca 2220
ccctcaggca ggtgggatgg atttccagcc aaagcctcct ccagccgcca tgctcctggc 2280
ccactgcatc gtttcatctt ccaactcaaa ctcttaaaac ccaagtgcct tagcaaattc 2340
tgtttttcta ggcctgggga cggcttttac ttaaaccgcc aaggctgggg gaagaagctc 2400
tctcctccct ttcttcccta cagttgaaaa acagctgagg gtgagtgggt gaataataca 2460
gtatctcagg gcctggtcgt tttcaacaga attataatta gttcctcatt agcattttgc 2520
taaatgtgaa tgatgatcct aggcatttgc tgaatacaga ggcaactgca ttggctttgg 2580
gttgcaggac ctcaggtgag aagcagagga aggagaggag aggggcacag ggtctctacc 2640
atcccctgta gagtgggagc tgagtggggg atcacagcct ctgaaaacca atgttctctc 2700
ttctccacct cccacaaagg agagctagca gcagggaggg cttctgccat ttctgagatc 2760
aaaacggttt tactgcagct ttgtttgttg tcagctgaac ctgggtaact agggaagata 2820
atattaagga agacaatgtg aaaagaaaaa tgagcctggc aagaatgtgt ttaaacttgg 2880
tttttaaaaa actgctgact gttttctctt gagagggtgg aatatccaat attcgctgtg 2940
tcagcataga agtaacttac ttaggtgtgg gggaagcacc ataactttgt ttagcccaaa 3000

accaagtcaa gtgaaaaagg aggaagagaa aaaatatttt cctgccaggc atggtggccc 3060

acgcacttcg ggaggtcgag gcaggaggat cacttgagtc cagaagtttg agatcagcct 3120

gggcaatgtg ataaaacccc atctctacaa aaagcataaa aattagccaa gtgtggtaga 3180

gtgtgcctga agtcccagat acttgggggg ctgaggtggg aggatctctt gagcctggga 3240

ggtcaaggct gcagtgagcc gagattgcac cactgcactc cagcctgggt gacagagcaa 3300

gtgagaccct gtctcaaaaa aagaaaaaga aaaagaaaaa atattttccc tattagagaa 3360

gagattgtgg tttcattctg tattttgttt ttgtcttaaa aagtggaaaa atagcctgcc 3420

tcttctctac tctagggaaa aaccagcgtg tgactactcc cccaggtggt tatggagagg 3480

gtgtccggtc cctgtcccag tgccgagaag gaagcctccc acgactgccc ggcagggtcc 3540

tagaaattcc ccaccctgaa agccctgagc tttctgctat caaagaggtt ttaaaaaaat 3600

cccatttaaa aaaaatccct tacctcggtg ccttcctctt tttatttagt tccttgagtt 3660

gattcagctc tgcaagaatt gaagcaggac taaatgtcta gttgtaacac catgattaac 3720

cacttcagct gacttttctg tccgagcttt gaaaattcag tggtgttagt ggttacccag 3780

ttagctctca agttatcagg gtattccaga gtggggatat gatttaaatc agccgtgtaa 3840

ccatggaccc aatatttacc agaccacaaa acttttctaa tactctaccc tcttagaaaa 3900

accaccacca tcaccagaca ggtgcgaaag gatgaaagtg accatgtttt gtttacggtt 3960

ttccaggttt aagctgttac tgtcttcagt aagccgtgat tttcattgct gggcttgtct 4020

gtagatttta gaccctattg ctgcttgagg caactcatct taggttggca aaaaggcagg 4080

atggccgggc gcggtggctc acgcctgtaa tcctagcact ttgggaggcc aaggtgggag 4140

gattgcttga gctcaggagt ttgagaccaa cctgggtaac atagtgagac accatctcta 4200

ttatgaacaa taacagttaa gaaaaaaaaa ggcaggcagg cggttatggt ggttccctcc 4260

catcccacca cataaagttt ctgagacttg agaacagcaa aatgctgtta aagggaaata 4320

ttaagaatga gaatctgcag taagggtgat tctgtgccca cagttcttca attctttata 4380

ccgttttacc cacatgtggt gttaccaaag ccgggcagaa ccatgctagc ggaagatgtg 4440

aaatccagat agctcattat tgccaagagc taggcagctt tgatctccaa attgttattg 4500

ctttcatttt tattgtaatg gaattgcttt gttttgtttt tttgtttttg tattgaagag 4560

ggttgttttc cctttatttt tcataagcta atgtaaatga agaaaaaatg tcttctctgg 4620

gctgtaggcc tggctcagcg tacacaggta tacatcctaa gctctctatg ttctctaatc 4680

tgtggtgact gaacatgtgt ctcaatgcac ggggcatttc tacctgtgtt tctgcagcac 4740

ccccactgcc ttgagtcccc agcagtgctg ttatttgcct aacacctgta gccatctgcc 4800

acgcagccag acgtgaaacg ctgagacaga gaccatttag gttaaatacg acagcttatc 4860

ctgctgggtg gggaaagtaa aaaatatgct ggttcaaggc ctaaagtaaa atgatcaata 4920
atgtttgtag cattaatgaa atattttcaa gaaatgtgtc caggggtagc actggctatg 4980
ttgacgaggc ctttggtaac tcagagagct cttggccctg atggggactt gcccttacgc 5040
tttctttatc aggctctgag ttcacacgga gcctctggca cttccctgct gtcttgggag 5100
aaaggaaact ggttgccgcg gcaggttgtg gaatctgttg ctggaaccag gctggaagcc 5160
cacctggtag tgaacagggc ccagtggggc aggctgggca tgttgtggtc tatgggtttg 5220
tttcctggag aatgttcagg aatgtcttcc cagctgcttt ggtgctgagc tctattatct 5280
cacagcacgt ccagaaggct aacccaggtg gggaggatgc tgacaccagc tccaggtgga 5340
gttggtggtc ttaatttgga gatgcagggg caacctgtga ccctttgagg caagagccct 5400
gcacccagct gtcccgtgca gccgtgggca ggggctgcac acggaggggc aggcgggcca 5460
gttcagggtc cgtgccaggc cctcctcagt gccctgtgaa ggcctcctgt cctccgtgcg 5520
gctgggcacc agcaccaggg agtttctatg gcaaccttag tgattattaa ggaacactgt 5580
cagttttatg aacatatgct caaatgaaat tctactttag gaggaaagga ttggaacagc 5640
atgtcacaag gctgttaatt aacagagaga ccttattgga tggagatcac atctgttaaa 5700
tagaatacct caactctacg ttgttttctt ggagataaat aatagtttca agtttttgtt 5760
tgtttgtttt acctaattac ctgaaagcaa ataccaaagg ctgatgtctg tatatggggc 5820
aaagggtcag tatatttttc agtgtttttt tttctaccag ctattttgca tttaaagtga 5880
acattgtgtt tggaataaat actcttaaaa aataaaaaaa aaaaaaaa 5928

<210> 2
<211> 468
<212> PRT
<213> Homo sapiens

<400> 2

Met Leu Ala Val Gly Cys Ala Leu Leu Ala Ala Leu Leu Ala Ala Pro
1               5                   10                  15

Gly Ala Ala Leu Ala Pro Arg Arg Cys Pro Ala Gln Glu Val Ala Arg
            20                  25                  30

Gly Val Leu Thr Ser Leu Pro Gly Asp Ser Val Thr Leu Thr Cys Pro
        35                  40                  45

Gly Val Glu Pro Glu Asp Asn Ala Thr Val His Trp Val Leu Arg Lys
    50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
            100                 105                 110

Pro Pro Glu Glu Pro Gln Leu Ser Cys Phe Arg Lys Ser Pro Leu Ser
        115                 120                 125

Asn Val Val Cys Glu Trp Gly Pro Arg Ser Thr Pro Ser Leu Thr Thr
    130                 135                 140

Lys Ala Val Leu Leu Val Arg Lys Phe Gln Asn Ser Pro Ala Glu Asp
145                 150                 155                 160

Phe Gln Glu Pro Cys Gln Tyr Ser Gln Glu Ser Gln Lys Phe Ser Cys
                165                 170                 175

Gln Leu Ala Val Pro Glu Gly Asp Ser Ser Phe Tyr Ile Val Ser Met
            180                 185                 190

Cys Val Ala Ser Ser Val Gly Ser Lys Phe Ser Lys Thr Gln Thr Phe
        195                 200                 205

Gln Gly Cys Gly Ile Leu Gln Pro Asp Pro Pro Ala Asn Ile Thr Val
    210                 215                 220

Thr Ala Val Ala Arg Asn Pro Arg Trp Leu Ser Val Thr Trp Gln Asp
225                 230                 235                 240

Pro His Ser Trp Asn Ser Ser Phe Tyr Arg Leu Arg Phe Glu Leu Arg
                245                 250                 255

Tyr Arg Ala Glu Arg Ser Lys Thr Phe Thr Thr Trp Met Val Lys Asp
            260                 265                 270

Leu Gln His His Cys Val Ile His Asp Ala Trp Ser Gly Leu Arg His
        275                 280                 285

Val Val Gln Leu Arg Ala Gln Glu Glu Phe Gly Gln Gly Glu Trp Ser
    290                 295                 300

Glu Trp Ser Pro Glu Ala Met Gly Thr Pro Trp Thr Glu Ser Arg Ser

305                 310                 315                 320

Pro Pro Ala Glu Asn Glu Val Ser Thr Pro Met Gln Ala Leu Thr Thr
                325                 330                 335

Asn Lys Asp Asp Asp Asn Ile Leu Phe Arg Asp Ser Ala Asn Ala Thr
            340                 345                 350

Ser Leu Pro Val Gln Asp Ser Ser Ser Val Pro Leu Pro Thr Phe Leu
        355                 360                 365

Val Ala Gly Gly Ser Leu Ala Phe Gly Thr Leu Leu Cys Ile Ala Ile
    370                 375                 380

Val Leu Arg Phe Lys Lys Thr Trp Lys Leu Arg Ala Leu Lys Glu Gly
385                 390                 395                 400

Lys Thr Ser Met His Pro Pro Tyr Ser Leu Gly Gln Leu Val Pro Glu
                405                 410                 415

Arg Pro Arg Pro Thr Pro Val Leu Val Pro Leu Ile Ser Pro Pro Val
            420                 425                 430

Ser Pro Ser Ser Leu Gly Ser Asp Asn Thr Ser Ser His Asn Arg Pro
        435                 440                 445

Asp Ala Arg Asp Pro Arg Ser Pro Tyr Asp Ile Ser Asn Thr Asp Tyr
    450                 455                 460

Phe Phe Pro Arg
465

<210> 3
<211> 2943
<212> DNA
<213> Homo sapiens

<400> 3

accaggcaac accattgaag gctcatatgt aaaaatccat gccttccttt ctcccaatct 60

ccattcccaa acttagccac tggcttctgg ctgaggcctt acgcatacct cccggggctt 120

gcacacacct tcttctacag aagacacacc ttgggcatat cctacagaag accaggcttc 180

tctctggtcc ttggtagagg gctactttac tgtaacaggg ccagggtgga gagttctctc 240

ctgaagctcc atcccctcta taggaaatgt gttgacaata ttcagaagag taagaggatc 300

aagacttctt tgtgctcaaa taccactgtt ctcttctcta ccctgcccta accaggagct 360

tgtcacccca aactctgagg tgatttatgc cttaatcaag caaacttccc tcttcagaaa 420

agatggctca ttttccctca aaagttgcca ggagctgcca agtattctgc caattcaccc 480

tggagcacaa tcaacaaatt cagccagaac acaactacag ctactattag aactattatt 540

attaataaat tcctctccaa atctagcccc ttgacttcgg atttcacgat ttctcccttc 600

ctcctagaaa cttgataagt ttcccgcgct tccctttttc taagactaca tgtttgtcat 660

cttataaagc aaaggggtga ataaatgaac caaatcaata acttctggaa tatctgcaaa 720

caacaataat atcagctatg ccatctttca ctattttagc cagtatcgag ttgaatgaac 780

atagaaaaat acaaaactga attcttccct gtaaattccc cgttttgacg acgcacttgt 840

agccacgtag ccacgcctac ttaagacaat tacaaaaggc gaagaagact gactcaggct 900

taagctgcca gccagagagg gagtcatttc attggcgttt gagtcagcaa agaagtcaag 960

atggccaaag ttccagacat gtttgaagac ctgaagaact gttacagtga aaatgaagaa 1020

gacagttcct ccattgatca tctgtctctg aatcagaaat ccttctatca tgtaagctat 1080

ggcccactcc atgaaggctg catggatcaa tctgtgtctc tgagtatctc tgaaacctct 1140

aaaacatcca agcttacctt caaggagagc atggtggtag tagcaaccaa cgggaaggtt 1200

ctgaagaaga gacggttgag tttaagccaa tccatcactg atgatgacct ggaggccatc 1260

gccaatgact cagaggaaga aatcatcaag cctaggtcag caccttttag cttcctgagc 1320

aatgtgaaat acaactttat gaggatcatc aaatacgaat tcatcctgaa tgacgccctc 1380

aatcaaagta taattcgagc caatgatcag tacctcacgg ctgctgcatt acataatctg 1440

gatgaagcag tgaaatttga catgggtgct tataagtcat caaaggatga tgctaaaatt 1500

accgtgattc taagaatctc aaaaactcaa ttgtatgtga ctgcccaaga tgaagaccaa 1560

ccagtgctgc tgaaggagat gcctgagata cccaaaacca tcacaggtag tgagaccaac 1620

ctcctcttct tctgggaaac tcacggcact aagaactatt tcacatcagt tgcccatcca 1680

aacttgttta ttgccacaaa gcaagactac tgggtgtgct tggcaggggg gccaccctct 1740

atcactgact ttcagatact ggaaaaccag gcgtaggtct ggagtctcac ttgtctcact 1800

tgtgcagtgt tgacagttca tatgtaccat gtacatgaag aagctaaatc ctttactgtt 1860

agtcatttgc tgagcatgta ctgagccttg taattctaaa tgaatgttta cactctttgt 1920

aagagtggaa ccaacactaa catataatgt tgttatttaa agaacaccct atattttgca 1980

tagtaccaat cattttaatt attattcttc ataacaattt taggaggacc agagctactg 2040

actatggcta ccaaaaagac tctacccata ttacagatgg gcaaattaag gcataagaaa 2100

actaagaaat atgcacaata gcagttgaaa caagaagcca cagacctagg atttcatgat 2160

ttcatttcaa ctgtttgcct tctactttta agttgctgat gaactcttaa tcaaatagca 2220

taagtttctg ggacctcagt tttatcattt tcaaaatgga gggaataata cctaagcctt 2280

cctgccgcaa cagtttttta tgctaatcag ggaggtcatt ttggtaaaat acttcttgaa 2340

gccgagcctc aagatgaagg caaagcacga aatgttattt tttaattatt atttatatat 2400

gtatttataa atatatttaa gataattata atatactata tttatgggaa ccccttcatc 2460

ctctgagtgt gaccaggcat cctccacaat agcagacagt gttttctggg ataagtaagt 2520

ttgatttcat taatacaggg cattttggtc caagttgtgc ttatcccata gccaggaaac 2580

tctgcattct agtacttggg agacctgtaa tcatataata aatgtacatt aattaccttg 2640

agccagtaat tggtccgatc tttgactctt ttgccattaa acttacctgg gcattcttgt 2700

ttcaattcca cctgcaatca agtcctacaa gctaaaatta gatgaactca actttgacaa 2760

ccatgagacc actgttatca aaactttctt ttctggaatg taatcaatgt ttcttctagg 2820

ttctaaaaat tgtgatcaga ccataatgtt acattattat caacaatagt gattgataga 2880

gtgttatcag tcataactaa ataaagcttg caacaaaatt ctctgacaaa aaaaaaaaaa 2940

aaa 2943

<210> 4
<211> 271
<212> PRT
<213> Homo sapiens

<400> 4

Met Ala Lys Val Pro Asp Met Phe Glu Asp Leu Lys Asn Cys Tyr Ser
1               5                   10                  15

Glu Asn Glu Glu Asp Ser Ser Ser Ile Asp His Leu Ser Leu Asn Gln
            20                  25                  30

Lys Ser Phe Tyr His Val Ser Tyr Gly Pro Leu His Glu Gly Cys Met
        35                  40                  45

Asp Gln Ser Val Ser Leu Ser Ile Ser Glu Thr Ser Lys Thr Ser Lys
    50                  55                  60

Leu Thr Phe Lys Glu Ser Met Val Val Val Ala Thr Asn Gly Lys Val
65                  70                  75                  80

Leu Lys Lys Arg Arg Leu Ser Leu Ser Gln Ser Ile Thr Asp Asp Asp
                85                  90                  95

Leu Glu Ala Ile Ala Asn Asp Ser Glu Glu Glu Ile Ile Lys Pro Arg
            100                 105                 110

Ser Ala Pro Phe Ser Phe Leu Ser Asn Val Lys Tyr Asn Phe Met Arg
        115                 120                 125

Ile Ile Lys Tyr Glu Phe Ile Leu Asn Asp Ala Leu Asn Gln Ser Ile
    130                 135                 140

Ile Arg Ala Asn Asp Gln Tyr Leu Thr Ala Ala Ala Leu His Asn Leu
145                 150                 155                 160

Asp Glu Ala Val Lys Phe Asp Met Gly Ala Tyr Lys Ser Ser Lys Asp
                165                 170                 175

Asp Ala Lys Ile Thr Val Ile Leu Arg Ile Ser Lys Thr Gln Leu Tyr
            180                 185                 190

Val Thr Ala Gln Asp Glu Asp Gln Pro Val Leu Leu Lys Glu Met Pro
        195                 200                 205

Glu Ile Pro Lys Thr Ile Thr Gly Ser Glu Thr Asn Leu Leu Phe Phe
    210                 215                 220

Trp Glu Thr His Gly Thr Lys Asn Tyr Phe Thr Ser Val Ala His Pro
225                 230                 235                 240

Asn Leu Phe Ile Ala Thr Lys Gln Asp Tyr Trp Val Cys Leu Ala Gly
                245                 250                 255

Gly Pro Pro Ser Ile Thr Asp Phe Gln Ile Leu Glu Asn Gln Ala
            260                 265                 270

<210> 5
<211> 2354
<212> DNA
<213> Homo sapiens

<400> 5

gctcagggca catgcctccc ctccccaggc cgcggcccag ctgaccctcg gggctccccc 60

ggcagcggac agggaagggt taaaggcccc cggctccctg ccccctgccc tggggaaccc 120

ctggccctgt ggggacatga actgtgtttg ccgcctggtc ctggtcgtgc tgagcctgtg 180

gccagataca gctgtcgccc ctgggccacc acctggcccc cctcgagttt ccccagaccc 240

tcgggccgag ctggacagca ccgtgctcct gacccgctct ctcctggcgg acacgcggca 300

gctggctgca cagctgaggg acaaattccc agctgacggg gaccacaacc tggattccct 360

gcccaccctg gccatgagtg cgggggcact gggagctcta cagctcccag gtgtgctgac 420

aaggctgcga gcggacctac tgtcctacct gcggcacgtg cagtggctgc gccgggcagg 480
tggctcttcc ctgaagaccc tggagcccga gctgggcacc ctgcaggccc gactggaccg 540
gctgctgcgc cggctgcagc tcctgatgtc ccgcctggcc ctgccccagc cacccccgga 600
cccgccggcg cccccgctgg cgcccccctc ctcagcctgg gggggcatca gggccgccca 660
cgccatcctg ggggggctgc acctgacact tgactgggcc gtgaggggac tgctgctgct 720
gaagactcgg ctgtgacccg gggcccaaag ccaccaccgt ccttccaaag ccagatctta 780
tttatttatt tatttcagta ctgggggcga aacagccagg tgatcccccc gccattatct 840
ccccctagtt agagacagtc cttccgtgag gcctgggggg catctgtgcc ttatttatac 900
ttatttattt caggagcagg ggtgggaggc aggtggactc ctgggtcccc gaggaggagg 960
ggactggggt cccggattct tgggtctcca agaagtctgt ccacagactt ctgccctggc 1020
tcttccccat ctaggcctgg gcaggaacat atattattta tttaagcaat tacttttcat 1080
gttggggtgg ggacggaggg gaaagggaag cctgggtttt tgtacaaaaa tgtgagaaac 1140
ctttgtgaga cagagaacag ggaattaaat gtgtcataca tatccacttg agggcgattt 1200
gtctgagagc tggggctgga tgcttgggta actggggcag ggcaggtgga ggggagacct 1260
ccattcaggt ggaggtcccg agtgggcggg gcagcgactg ggagatgggt cggtcaccca 1320
gacagctctg tggaggcagg gtctgagcct tgcctggggc cccgcactgc atagggcctt 1380
ttgtttgttt tttgagatgg agtctcgctc tgttgcctag gctggagtgc agtgaggcaa 1440
tctgaggtca ctgcaacctc cacctcccgg gttcaagcaa ttctcctgcc tcagcctccc 1500
gattagctgg gatcacaggt gtgcaccacc atgcccagct aattatttat ttcttttgta 1560
tttttagtag agacagggtt tcaccatgtt ggccaggctg gtttcgaact cctgacctca 1620
ggtgatcctc ctgcctcggc ctcccaaagt gctgggatta caggtgtgag ccaccacacc 1680
tgacccatag gtcttcaata aatatttaat ggaaggttcc acaagtcacc ctgtgatcaa 1740
cagtacccgt atgggacaaa gctgcaaggt caagatggtt cattatggct gtgttcacca 1800
tagcaaactg gaaacaatct agatatccaa cagtgagggt taagcaacat ggtgcatctg 1860
tggatagaac gccacccagc cgcccggagc agggactgtc attcagggag gctaaggaga 1920
gaggcttgct tgggatatag aaagatatcc tgacattggc caggcatggt ggctcacgcc 1980
tgtaatcctg gcactttggg aggacgaagc gagtggatca ctgaagtcca agagttcgag 2040
accggcctgc gagacatggc aaaaccctgt ctcaaaaaag aaagaatgat gtcctgacat 2100
gaaacagcag gctacaaaac cactgcatgc tgtgatccca attttgtgtt tttctttcta 2160
tatatggatt aaaacaaaaa tcctaaaggg aaatacgcca aaatgttgac aatgactgtc 2220

tccaggtcaa aggagagagg tgggattgtg ggtgactttt aatgtgtatg attgtctgta 2280

ttttacagaa tttctgccat gactgtgtat tttgcatgac acattttaaa aataataaac 2340

actattttta gaat 2354

<210> 6
<211> 199
<212> PRT
<213> Homo sapiens

<400> 6

Met Asn Cys Val Cys Arg Leu Val Leu Val Val Leu Ser Leu Trp Pro
1               5                   10                  15

Asp Thr Ala Val Ala Pro Gly Pro Pro Pro Gly Pro Pro Arg Val Ser
            20                  25                  30

Pro Asp Pro Arg Ala Glu Leu Asp Ser Thr Val Leu Leu Thr Arg Ser
        35                  40                  45

Leu Leu Ala Asp Thr Arg Gln Leu Ala Ala Gln Leu Arg Asp Lys Phe
    50                  55                  60

Pro Ala Asp Gly Asp His Asn Leu Asp Ser Leu Pro Thr Leu Ala Met
65                  70                  75                  80

Ser Ala Gly Ala Leu Gly Ala Leu Gln Leu Pro Gly Val Leu Thr Arg
                85                  90                  95

Leu Arg Ala Asp Leu Leu Ser Tyr Leu Arg His Val Gln Trp Leu Arg
            100                 105                 110

Arg Ala Gly Gly Ser Ser Leu Lys Thr Leu Glu Pro Glu Leu Gly Thr
        115                 120                 125

Leu Gln Ala Arg Leu Asp Arg Leu Leu Arg Arg Leu Gln Leu Leu Met
    130                 135                 140

Ser Arg Leu Ala Leu Pro Gln Pro Pro Pro Asp Pro Pro Ala Pro Pro
145                 150                 155                 160

Leu Ala Pro Pro Ser Ser Ala Trp Gly Gly Ile Arg Ala Ala His Ala
                165                 170                 175

Ile Leu Gly Gly Leu His Leu Thr Leu Asp Trp Ala Val Arg Gly Leu
            180                 185                 190

Leu Leu Leu Lys Thr Arg Leu
        195

<210> 7
<211> 542

<212> DNA
<213> Homo sapiens

<400> 7

acagtggtga gctcttagct tcaccaggct catcaaagct gctccaggaa ggcccaagcc 60
agaccagaag acatgcagat catcaccaca gccctggtgt gcttgctgct agctgggatg 120
tggccggaag atgtggacag caagagcatg caggtaccct tctccagatg ttgcttctca 180
tttgcggagc aagagattcc cctgagggca atcctgtgtt acagaaatac cagctccatc 240
tgctccaatg agggcttaat attcaagctg aagagaggca aagaggcctg cgccttggac 300
acagttggat gggttcagag gcacagaaaa atgctgaggc actgcccgtc aaaaagaaaa 360
tgagcagatt tctttccatt gtgggctctg gaaaccacat ggcttcacct gtccccgaaa 420
ctaccagccc tacaccattc cttctgccct gcttttgcta ggtcacagag gatctgcttg 480
gtcttgataa gctatgttgt tgcactttaa acatttaaat tatacaatca tcaaccccca 540
ac 542

<210> 8
<211> 96
<212> PRT
<213> Homo sapiens

<400> 8

Met Gln Ile Ile Thr Thr Ala Leu Val Cys Leu Leu Leu Ala Gly Met
1               5                   10                  15

Trp Pro Glu Asp Val Asp Ser Lys Ser Met Gln Val Pro Phe Ser Arg
            20                  25                  30

Cys Cys Phe Ser Phe Ala Glu Gln Glu Ile Pro Leu Arg Ala Ile Leu
        35                  40                  45

Cys Tyr Arg Asn Thr Ser Ser Ile Cys Ser Asn Glu Gly Leu Ile Phe
    50                  55                  60

Lys Leu Lys Arg Gly Lys Glu Ala Cys Ala Leu Asp Thr Val Gly Trp
65                  70                  75                  80

Val Gln Arg His Arg Lys Met Leu Arg His Cys Pro Ser Lys Arg Lys
                85                  90                  95

<210> 9
<211> 360
<212> DNA
<213> Homo sapiens

<400> 9

atggcaggcc tgatgaccat agtaaccagc cttctgttcc ttggtgtctg tgcccaccac      60

atcatcccta cgggctctgt ggtcatcccc tctccctgct gcatgttctt tgtttccaag     120

agaattcctg agaaccgagt ggtcagctac cagctgtcca gcaggagcac atgcctcaag     180

gcaggagtga tcttcaccac caagaagggc cagcagttct gtggcgaccc caagcaggag     240

tgggtccaga ggtacatgaa gaacctggac gccaagcaga agaaggcttc ccctagggcc     300

agggcagtgg ctgtcaaggg ccctgtccag agatatcctg gcaaccaaac cacctgctaa     360

<210> 10
<211> 119
<212> PRT
<213> Homo sapiens

<400> 10

Met Ala Gly Leu Met Thr Ile Val Thr Ser Leu Leu Phe Leu Gly Val
1               5                   10                  15

Cys Ala His His Ile Ile Pro Thr Gly Ser Val Val Ile Pro Ser Pro
            20                  25                  30

Cys Cys Met Phe Phe Val Ser Lys Arg Ile Pro Glu Asn Arg Val Val
        35                  40                  45

Ser Tyr Gln Leu Ser Ser Arg Ser Thr Cys Leu Lys Ala Gly Val Ile
    50                  55                  60

Phe Thr Thr Lys Lys Gly Gln Gln Phe Cys Gly Asp Pro Lys Gln Glu
65                  70                  75                  80

Trp Val Gln Arg Tyr Met Lys Asn Leu Asp Ala Lys Gln Lys Lys Ala
                85                  90                  95

Ser Pro Arg Ala Arg Ala Val Ala Val Lys Gly Pro Val Gln Arg Tyr
            100                 105                 110

Pro Gly Asn Gln Thr Thr Cys
        115

<210> 11
<211> 2809
<212> DNA
<213> Homo sapiens

<400> 11

acgcgcgccc tgcggagccc gcccaactcc ggcgagccgg gcctgcgcct actcctcctc 60

ctcctctccc ggcggcggct gcggcggagg cgccgactcg gccttgcgcc cgccctcagg 120

cccgcgcggg cggcgcagcg aggccccggg cggcgggtgg tggctgccag gcggctcggc 180

cgcgggcgct gcccggcccc ggcgagcgga gggcggagcg cggcgccgga gccgagggcg 240

cgccgcggag ggggtgctgg gccgcgctgt gcccggccgg gcggcggctg caagaggagg 300

ccggaggcga gcgcggggcc ggcggtgggc gcgcagggcg gctcgcagct cgcagccggg 360

gccgggccag gcgtccaggc aggtgatcgg tgtggcggcg gcggcggcgg cggccccaga 420

ctccctccgg agttcttctt ggggctgatg tccgcaaata tgcagaatta ccggccgggt 480

cgctcctgaa gccagcgcgg ggagcgagcg cggcggcggc cagcaccggg aacgcaccga 540

ggaagaagcc cagcccccgc cctccgcccc ttccgtcccc accccctacc cggcggccca 600

ggaggctccc cgcgctgcgg gcgcgcactc cctgtttctc ctcctcctgg ctggcgctgc 660

ctgcctctcc gcactcactg ctcgcgccgg gcgcgctccg ccagctccgt gctccccgcg 720

ccaccctcct ccgggccgcg ctccctaagg gatggtactg aatttcgccg ccacaggaga 780

ccggctggag cgcccgcccc gcggcctcgc ctctcctccg agcagccagc gcctcgggac 840

gcgatgagga ccttggcttg cctgctgctc ctcggctgcg gatacctcgc ccatgttctg 900

gccgaggaag ccgagatccc ccgcgaggtg atcgagaggc tggcccgcag tcagatccac 960

agcatccggg acctccagcg actcctggag atagactccg tagggagtga ggattctttg 1020

gacaccagcc tgagagctca cggggtccat gccactaagc atgtgcccga gaagcggccc 1080

ctgcccattc ggaggaagag aagcatcgag gaagctgtcc ccgctgtctg caagaccagg 1140

acggtcattt acgagattcc tcggagtcag gtcgacccca cgtccgccaa cttcctgatc 1200

tggcccccgt gcgtggaggt gaaacgctgc accggctgct gcaacacgag cagtgtcaag 1260

tgccagccct cccgcgtcca ccaccgcagc gtcaaggtgg ccaaggtgga atacgtcagg 1320

aagaagccaa aattaaaaga agtccaggtg aggttagagg agcatttgga gtgcgcctgc 1380

gcgaccacaa gcctgaatcc ggattatcgg gaagaggaca cgggaaggcc tagggagtca 1440

ggtaaaaaac ggaaaagaaa aaggttaaaa cccacctaaa gcagccaacc agatgtgagg 1500

tgaggatgag ccgcagccct ttcctgggac atggatgtac atggcgtgtt acattcctga 1560

acctactatg tacggtgctt tattgccagt gtgcggtctt tgttctcctc cgtgaaaaac 1620

tgtgtccgag aacactcggg agaacaaaga gacagtgcac atttgtttaa tgtgacatca 1680

aagcaagtat tgtagcactc ggtgaagcag taagaagctt ccttgtcaaa aagagagaga 1740

gagaaagaga gagagaaaac aaaaccacaa atgacaaaaa caaaacggac tcacaaaaat 1800

atctaaactc gatgagatgg agggtcgccc cgtgggatgg aagtgcagag gtctcagcag 1860

actggatttc tgtccgggtg gtcacaggtg cttttttgcc gaggatgcag agcctgcttt 1920

gggaacgact ccagaggggt gctggtgggc tctgcagggg cccgcaggaa gcaggaatgt 1980

cttggaaacc gccacgcgaa ctttagaaac cacacctcct cgctgtagta tttaagccca 2040

tacagaaacc ttcctgagag ccttaagtgg tttttttttt tgtttttgtt ttgttttttt 2100

tttttttgtt tttttttttt tttttttaca ccataaagtg attattaagc tttccttttt 2160

actctttggc tagctttttt tttttttttt tttttttaat tatctcttgg atgacattta 2220

caccgataac acacaggctg ctgtaactgt caggacagtg cgacggtatt tttcctagca 2280

agatgcaaac taatgagatg tattaaaata aacatggtat acctacctat gcatcatttc 2340

ctaaatgttt ctggctttgt gtttctccct taccctgctt tatttgttaa tttaagccat 2400

tttgaaagaa ctatgcgtca accaatcgta cgccgtccct gcggcacctg ccccagagcc 2460

cgtttgtggc tgagtgacaa cttgttcccc gcagtgcaca cctagaatgc tgtgttccca 2520

cgcggcacgt gagatgcatt gccgcttctg tctgtgttgt tggtgtgccc tggtgccgtg 2580

gtggcggtca ctccctctgc tgccagtgtt tggacagaac ccaaattctt tatttttggt 2640

aagatattgt gctttacctg tattaacaga aatgtgtgtg tgtggtttgt ttttttgtaa 2700

aggtgaagtt tgtatgttta cctaatatta cctgttttgt atacctgaga gcctgctatg 2760

ttcttttttt gttgatccaa aattaaaaaa aaaaatacca ccaacaaaa 2809

<210> 12
<211> 211
<212> PRT
<213> Homo sapiens

<400> 12

<210> 13
<211> 2258
<212> DNA
<213> Homo sapiens

<400> 13

ctcctccagc tcttcctgtc ccgctgttgc aacactgcct cactcttccc ctcccacctt 60
ctctcccctc ctctctgctt taattttctc agaattctct ggactgaggc tccagttctg 120
gcctttgggg ttcaagatca ctgggaccag gccgtgatct ctatgcccga gtctcaaccc 180
tcaactgtca ccccaaggca cttgggacgt cctggacaga ccgagtcccg ggaagcccca 240
gcactgccgc tgccacactg ccctgagccc aaatggggga gtgagaggcc atagctgtct 300
ggcatgggcc tctccaccgt gcctgacctg ctgctgccac tggtgctcct ggagctgttg 360
gtgggaatat acccctcagg ggttattgga ctggtccctc acctagggga cagggagaag 420

agagatagtg tgtgtcccca aggaaaatat atccaccctc aaaataattc gatttgctgt 480
accaagtgcc acaaaggaac ctacttgtac aatgactgtc caggcccggg gcaggatacg 540
gactgcaggg agtgtgagag cggctccttc accgcttcag aaaaccacct cagacactgc 600
ctcagctgct ccaaatgccg aaaggaaatg ggtcaggtgg agatctcttc ttgcacagtg 660
gaccgggaca ccgtgtgtgg ctgcaggaag aaccagtacc ggcattattg gagtgaaaac 720
cttttccagt gcttcaattg cagcctctgc ctcaatggga ccgtgcacct ctcctgccag 780
gagaaacaga acaccgtgtg cacctgccat gcaggtttct ttctaagaga aaacgagtgt 840
gtctcctgta gtaactgtaa gaaaagcctg gagtgcacga agttgtgcct accccagatt 900
gagaatgtta agggcactga ggactcaggc accacagtgc tgttgcccct ggtcattttc 960
tttggtcttt gccttttatc cctcctcttc attggtttaa tgtatcgcta ccaacggtgg 1020
aagtccaagc tctactccat tgtttgtggg aaatcgacac ctgaaaaaga gggggagctt 1080
gaaggaacta ctactaagcc cctggcccca aacccaagct tcagtcccac tccaggcttc 1140
acccccaccc tgggcttcag tcccgtgccc agttccacct tcacctccag ctccacctat 1200
acccccggtg actgtcccaa ctttgcggct ccccgcagag aggtggcacc accctatcag 1260
ggggctgacc ccatccttgc gacagccctc gcctccgacc ccatccccaa cccccttcag 1320
aagtgggagg acagcgccca caagccacag agcctagaca ctgatgaccc cgcgacgctg 1380
tacgccgtgg tggagaacgt gcccccgttg cgctggaagg aattcgtgcg gcgcctaggg 1440
ctgagcgacc acgagatcga tcggctggag ctgcagaacg ggcgctgcct gcgcgaggcg 1500
caatacagca tgctggcgac ctggaggcgg cgcacgccgc ggcgcgaggc cacgctggag 1560
ctgctgggac gcgtgctccg cgacatggac ctgctgggct gcctggagga catcgaggag 1620
gcgctttgcg gccccgccgc cctcccgccc gcgcccagtc ttctcagatg aggctgcgcc 1680
cctgcgggca gctctaagga ccgtcctgcg agatcgcctt ccaaccccac ttttttctgg 1740
aaaggagggg tcctgcaggg gcaagcagga gctagcagcc gcctacttgg tgctaacccc 1800
tcgatgtaca tagcttttct cagctgcctg cgcgccgccg acagtcagcg ctgtgcgcgc 1860
ggagagaggt gcgccgtggg ctcaagagcc tgagtgggtg gtttgcgagg atgagggacg 1920
ctatgcctca tgcccgtttt gggtgtcctc accagcaagg ctgctcgggg gcccctggtt 1980
cgtccctgag cctttttcac agtgcataag cagttttttt tgtttttgtt ttgttttgtt 2040
ttgtttttaa atcaatcatg ttacactaat agaaacttgg cactcctgtg ccctctgcct 2100
ggacaagcac atagcaagct gaactgtcct aaggcagggg cgagcacgga acaatggggc 2160
cttcagctgg agctgtggac ttttgtacat acactaaaat tctgaagtta aagctctgct 2220
cttggaaaaa aaaaaaaaaa aaaaaaaaaa aaaaaaaa 2258

<210> 14
<211> 455
<212> PRT
<213> Homo sapiens

<400> 14

<210> 15
<211> 3682
<212> DNA
<213> Homo sapiens

<400> 15

gcgagcgcag cggagcctgg agagaaggcg ctgggctgcg agggcgcgag ggcgcgaggg 60
cagggggcaa ccggaccccg cccgcaccca tggcgcccgt cgccgtctgg gccgcgctgg 120
ccgtcggact ggagctctgg gctgcggcgc acgccttgcc cgcccaggtg gcatttacac 180
cctacgcccc ggagcccggg agcacatgcc ggctcagaga atactatgac cagacagctc 240
agatgtgctg cagcaaatgc tcgccgggcc aacatgcaaa agtcttctgt accaagacct 300
cggacaccgt gtgtgactcc tgtgaggaca gcacatacac ccagctctgg aactgggttc 360
ccgagtgctt gagctgtggc tcccgctgta gctctgacca ggtggaaact caagcctgca 420
ctcgggaaca gaaccgcatc tgcacctgca ggcccggctg gtactgcgcg ctgagcaagc 480
aggaggggtg ccggctgtgc gcgccgctgc gcaagtgccg cccgggcttc ggcgtggcca 540
gaccaggaac tgaaacatca gacgtggtgt gcaagccctg tgccccgggg acgttctcca 600
acacgacttc atccacggat atttgcaggc cccaccagat ctgtaacgtg gtggccatcc 660
ctgggaatgc aagcatggat gcagtctgca cgtccacgtc ccccacccgg agtatggccc 720
caggggcagt acacttaccc cagccagtgt ccacacgatc ccaacacacg cagccaactc 780
cagaacccag cactgctcca agcacctcct tcctgctccc aatgggcccc agccccccag 840
ctgaagggag cactggcgac ttcgctcttc cagttggact gattgtgggt gtgacagcct 900
tgggtctact aataatagga gtggtgaact gtgtcatcat gacccaggtg aaaaagaagc 960
ccttgtgcct gcagagagaa gccaaggtgc ctcacttgcc tgccgataag gcccggggta 1020
cacagggccc cgagcagcag cacctgctga tcacagcgcc gagctccagc agcagctccc 1080
tggagagctc ggccagtgcg ttggacagaa gggcgcccac tcggaaccag ccacaggcac 1140
caggcgtgga ggccagtggg gccggggagg cccgggccag caccgggagc tcagattctt 1200
cccctggtgg ccatgggacc caggtcaatg tcacctgcat cgtgaacgtc tgtagcagct 1260
ctgaccacag ctcacagtgc tcctcccaag ccagctccac aatgggagac acagattcca 1320
gcccctcgga gtccccgaag gacgagcagg tccccttctc caaggaggaa tgtgcctttc 1380
ggtcacagct ggagacgcca gagaccctgc tggggagcac cgaagagaag cccctgcccc 1440
ttggagtgcc tgatgctggg atgaagccca gttaaccagg ccggtgtggg ctgtgtcgta 1500
gccaaggtgg gctgagccct ggcaggatga ccctgcgaag gggccctggt ccttccaggc 1560

ccccaccact aggactctga ggctctttct gggccaagtt cctctagtgc cctccacagc 1620
cgcagcctcc ctctgacctg caggccaaga gcagaggcag cgagttgtgg aaagcctctg 1680
ctgccatggc gtgtccctct cggaaggctg gctgggcatg gacgttcggg gcatgctggg 1740
gcaagtccct gactctctgt gacctgcccc gcccagctgc acctgccagc ctggcttctg 1800
gagcccttgg gttttttgtt tgtttgtttg tttgtttgtt tgtttctccc cctgggctct 1860
gccccagctc tggcttccag aaaaccccag catccttttc tgcagagggg ctttctggag 1920
aggagggatg ctgcctgagt cacccatgaa gacaggacag tgcttcagcc tgaggctgag 1980
actgcgggat ggtcctgggg ctctgtgcag ggaggaggtg gcagccctgt agggaacggg 2040
gtccttcaag ttagctcagg aggcttggaa agcatcacct caggccaggt gcagtggctc 2100
acgcctatga tcccagcact ttgggaggct gaggcgggtg gatcacctga ggttaggagt 2160
tcgagaccag cctggccaac atggtaaaac cccatctcta ctaaaaatac agaaattagc 2220
cgggcgtggt ggcgggcacc tatagtccca gctactcaga agcctgaggc tgggaaatcg 2280
tttgaacccg ggaagcggag gttgcaggga gccgagatca cgccactgca ctccagcctg 2340
ggcgacagag cgagagtctg tctcaaaaga aaaaaaaaag caccgcctcc aaatgccaac 2400
ttgtcctttt gtaccatggt gtgaaagtca gatgcccaga gggcccaggc aggccaccat 2460
attcagtgct gtggcctggg caagataacg cacttctaac tagaaatctg ccaatttttt 2520
aaaaaagtaa gtaccactca ggccaacaag ccaacgacaa agccaaactc tgccagccac 2580
atccaacccc ccacctgcca tttgcaccct ccgccttcac tccggtgtgc ctgcagcccc 2640
gcgcctcctt ccttgctgtc ctaggccaca ccatctcctt tcagggaatt tcaggaacta 2700
gagatgactg agtcctcgta gccatctctc tactcctacc tcagcctaga ccctcctcct 2760
cccccagagg ggtgggttcc tcttccccac tccccacctt caattcctgg gccccaaacg 2820
ggctgccctg ccactttggt acatggccag tgtgatccca agtgccagtc ttgtgtctgc 2880
gtctgtgttg cgtgtcgtgg gtgtgtgtag ccaaggtcgg taagttgaat ggcctgcctt 2940
gaagccactg aagctgggat tcctccccat tagagtcagc cttccccctc ccagggccag 3000
ggccctgcag aggggaaacc agtgtagcct tgcccggatt ctgggaggaa gcaggttgag 3060
gggctcctgg aaaggctcag tctcaggagc atggggataa aggagaaggc atgaaattgt 3120
ctagcagagc aggggcaggg tgataaattg ttgataaatt ccactggact tgagcttggc 3180
agctgaacta ttggagggtg ggagagccca gccattacca tggagacaag aagggttttc 3240
caccctggaa tcaagatgtc agactggctg gctgcagtga cgtgcacctg tactcaggag 3300
gctgagggga ggatcactgg agcccaggag tttgaggctg cagcgagcta tgatcgcgcc 3360
actacactcc agcctgagca acagagtgag accctgtctc ttaaagaaaa aaaaagtcag 3420

actgctggga ctggccaggt ttctgcccac attggaccca catgaggaca tgatggagcg 3480

cacctgcccc ctggtggaca gtcctgggag aacctcaggc ttccttggca tcacagggca 3540

gagccgggaa gcgatgaatt tggagactct gtggggcctt ggttcccttg tgtgtgtgtg 3600

ttgatcccaa gacaatgaaa gtttgcactg tatgctggac ggcattcctg cttatcaata 3660

aacctgtttg ttttaaaaaa aa 3682

<210> 16
<211> 461
<212> PRT
<213> Homo sapiens

<400> 16

Met Ala Pro Val Ala Val Trp Ala Ala Leu Ala Val Gly Leu Glu Leu
1               5                   10                  15

Trp Ala Ala Ala His Ala Leu Pro Ala Gln Val Ala Phe Thr Pro Tyr
            20                  25                  30

Ala Pro Glu Pro Gly Ser Thr Cys Arg Leu Arg Glu Tyr Tyr Asp Gln
        35                  40                  45

Thr Ala Gln Met Cys Cys Ser Lys Cys Ser Pro Gly Gln His Ala Lys
    50                  55                  60

Val Phe Cys Thr Lys Thr Ser Asp Thr Val Cys Asp Ser Cys Glu Asp
65                  70                  75                  80

Ser Thr Tyr Thr Gln Leu Trp Asn Trp Val Pro Glu Cys Leu Ser Cys
                85                  90                  95

Gly Ser Arg Cys Ser Ser Asp Gln Val Glu Thr Gln Ala Cys Thr Arg
            100                 105                 110

Glu Gln Asn Arg Ile Cys Thr Cys Arg Pro Gly Trp Tyr Cys Ala Leu
        115                 120                 125

Ser Lys Gln Glu Gly Cys Arg Leu Cys Ala Pro Leu Arg Lys Cys Arg
    130                 135                 140

Pro Gly Phe Gly Val Ala Arg Pro Gly Thr Glu Thr Ser Asp Val Val
145                 150                 155                 160

Cys Lys Pro Cys Ala Pro Gly Thr Phe Ser Asn Thr Thr Ser Ser Thr
                165                 170                 175

Asp Ile Cys Arg Pro His Gln Ile Cys Asn Val Val Ala Ile Pro Gly
            180                 185                 190

Asn Ala Ser Met Asp Ala Val Cys Thr Ser Thr Ser Pro Thr Arg Ser
        195                 200                 205

Met Ala Pro Gly Ala Val His Leu Pro Gln Pro Val Ser Thr Arg Ser
    210                 215                 220

Gln His Thr Gln Pro Thr Pro Glu Pro Ser Thr Ala Pro Ser Thr Ser
225                 230                 235                 240

Phe Leu Leu Pro Met Gly Pro Ser Pro Pro Ala Glu Gly Ser Thr Gly
                245                 250                 255

Asp Phe Ala Leu Pro Val Gly Leu Ile Val Gly Val Thr Ala Leu Gly
            260                 265                 270

Leu Leu Ile Ile Gly Val Val Asn Cys Val Ile Met Thr Gln Val Lys
        275                 280                 285

Lys Lys Pro Leu Cys Leu Gln Arg Glu Ala Lys Val Pro His Leu Pro
    290                 295                 300

Ala Asp Lys Ala Arg Gly Thr Gln Gly Pro Glu Gln Gln His Leu Leu
305                 310                 315                 320

Ile Thr Ala Pro Ser Ser Ser Ser Ser Ser Leu Glu Ser Ser Ala Ser
                325                 330                 335

Ala Leu Asp Arg Arg Ala Pro Thr Arg Asn Gln Pro Gln Ala Pro Gly
            340                 345                 350

Val Glu Ala Ser Gly Ala Gly Glu Ala Arg Ala Ser Thr Gly Ser Ser
        355                 360                 365

Asp Ser Ser Pro Gly Gly His Gly Thr Gln Val Asn Val Thr Cys Ile
    370                 375                 380

Val Asn Val Cys Ser Ser Ser Asp His Ser Ser Gln Cys Ser Ser Gln
385                 390                 395                 400

Ala Ser Ser Thr Met Gly Asp Thr Asp Ser Ser Pro Ser Glu Ser Pro
                405                 410                 415

Lys Asp Glu Gln Val Pro Phe Ser Lys Glu Glu Cys Ala Phe Arg Ser
            420                 425                 430

Gln Leu Glu Thr Pro Glu Thr Leu Leu Gly Ser Thr Glu Glu Lys Pro
        435                 440                 445

Leu Pro Leu Gly Val Pro Asp Ala Gly Met Lys Pro Ser
    450                 455                 460

<210> 17
<211> 5600
<212> DNA
<213> Homo sapiens

<400> 17

aaaaagagaa actgttggga gaggaatcgt atctccatat ttcttctttc agccccaatc 60
caagggttgt agctggaact ttccatcagt tcttcctttc tttttcctct ctaagccttt 120
gccttgctct gtcacagtga agtcagccag agcagggctg ttaaactctg tgaaatttgt 180
cataagggtg tcaggtattt cttactggct tccaaagaaa catagataaa gaaatctttc 240
ctgtggcttc ccttggcagg ctgcattcag aaggtctctc agttgaagaa agagcttgga 300
ggacaacagc acaacaggag agtaaaagat gccccagggc tgaggcctcc gctcaggcag 360
ccgcatctgg ggtcaatcat actcaccttg cccgggccat gctccagcaa aatcaagctg 420
ttttcttttg aaagttcaaa ctcatcaaga ttatgctgct cactcttatc attctgttgc 480
cagtagtttc aaaatttagt tttgttagtc tctcagcacc gcagcactgg agctgtcctg 540
aaggtactct cgcaggaaat gggaattcta cttgtgtggg tcctgcaccc ttcttaattt 600
tctcccatgg aaatagtatc tttaggattg acacagaagg aaccaattat gagcaattgg 660
tggtggatgc tggtgtctca gtgatcatgg attttcatta taatgagaaa agaatctatt 720
gggtggattt agaaagacaa cttttgcaaa gagtttttct gaatgggtca aggcaagaga 780
gagtatgtaa tatagagaaa aatgtttctg gaatggcaat aaattggata aatgaagaag 840
ttatttggtc aaatcaacag gaaggaatca ttacagtaac agatatgaaa ggaaataatt 900
cccacattct tttaagtgct ttaaaatatc ctgcaaatgt agcagttgat ccagtagaaa 960
ggtttatatt ttggtcttca gaggtggctg gaagccttta tagagcagat ctcgatggtg 1020
tgggagtgaa ggctctgttg gagacatcag agaaaataac agctgtgtca ttggatgtgc 1080
ttgataagcg gctgttttgg attcagtaca acagagaagg aagcaattct cttatttgct 1140
cctgtgatta tgatggaggt tctgtccaca ttagtaaaca tccaacacag cataatttgt 1200
ttgcaatgtc cctttttggt gaccgtatct tctattcaac atggaaaatg aagacaattt 1260

ggatagccaa caaacacact ggaaaggaca tggttagaat taacctccat tcatcatttg 1320
taccacttgg tgaactgaaa gtagtgcatc cacttgcaca acccaaggca gaagatgaca 1380
cttgggagcc tgagcagaaa ctttgcaaat tgaggaaagg aaactgcagc agcactgtgt 1440
gtgggcaaga cctccagtca cacttgtgca tgtgtgcaga gggatacgcc ctaagtcgag 1500
accggaagta ctgtgaagat gttaatgaat gtgctttttg gaatcatggc tgtactcttg 1560
ggtgtaaaaa cacccctgga tcctattact gcacgtgccc tgtaggattt gttctgcttc 1620
ctgatgggaa acgatgtcat caacttgttt cctgtccacg caatgtgtct gaatgcagcc 1680
atgactgtgt tctgacatca gaaggtccct tatgtttctg tcctgaaggc tcagtgcttg 1740
agagagatgg gaaaacatgt agcggttgtt cctcacccga taatggtgga tgtagccagc 1800
tctgcgttcc tcttagccca gtatcctggg aatgtgattg ctttcctggg tatgacctac 1860
aactggatga aaaaagctgt gcagcttcag gaccacaacc atttttgctg tttgccaatt 1920
ctcaagatat tcgacacatg cattttgatg gaacagacta tggaactctg ctcagccagc 1980
agatgggaat ggtttatgcc ctagatcatg accctgtgga aaataagata tactttgccc 2040
atacagccct gaagtggata gagagagcta atatggatgg ttcccagcga gaaaggctta 2100
ttgaggaagg agtagatgtg ccagaaggtc ttgctgtgga ctggattggc cgtagattct 2160
attggacaga cagagggaaa tctctgattg gaaggagtga tttaaatggg aaacgttcca 2220
aaataatcac taaggagaac atctctcaac cacgaggaat tgctgttcat ccaatggcca 2280
agagattatt ctggactgat acagggatta atccacgaat tgaaagttct tccctccaag 2340
gccttggccg tctggttata gccagctctg atctaatctg gcccagtgga ataacgattg 2400
acttcttaac tgacaagttg tactggtgcg atgccaagca gtctgtgatt gaaatggcca 2460
atctggatgg ttcaaaacgc cgaagactta cccagaatga tgtaggtcac ccatttgctg 2520
tagcagtgtt tgaggattat gtgtggttct cagattgggc tatgccatca gtaatgagag 2580
taaacaagag gactggcaaa gatagagtac gtctccaagg cagcatgctg aagccctcat 2640
cactggttgt ggttcatcca ttggcaaaac caggagcaga tccctgctta tatcaaaacg 2700
gaggctgtga acatatttgc aaaaagaggc ttggaactgc ttggtgttcg tgtcgtgaag 2760
gttttatgaa agcctcagat gggaaaacgt gtctggctct ggatggtcat cagctgttgg 2820
caggtggtga agttgatcta aagaaccaag taacaccatt ggacatcttg tccaagacta 2880
gagtgtcaga agataacatt acagaatctc aacacatgct agtggctgaa atcatggtgt 2940
cagatcaaga tgactgtgct cctgtgggat gcagcatgta tgctcggtgt atttcagagg 3000
gagaggatgc cacatgtcag tgtttgaaag gatttgctgg ggatggaaaa ctatgttctg 3060
atatagatga atgtgagatg ggtgtcccag tgtgcccccc tgcctcctcc aagtgcatca 3120

acaccgaagg tggttatgtc tgccggtgct cagaaggcta ccaaggagat gggattcact 3180

gtcttgatat tgatgagtgc caactggggg agcacagctg tggagagaat gccagctgca 3240

caaatacaga gggaggctat acctgcatgt gtgctggacg cctgtctgaa ccaggactga 3300

tttgccctga ctctactcca ccccctcacc tcagggaaga tgaccaccac tattccgtaa 3360

gaaatagtga ctctgaatgt cccctgtccc acgatgggta ctgcctccat gatggtgtgt 3420

gcatgtatat tgaagcattg gacaagtatg catgcaactg tgttgttggc tacatcgggg 3480

agcgatgtca gtaccgagac ctgaagtggt gggaactgcg ccacgctggc cacgggcagc 3540

agcagaaggt catcgtggtg gctgtctgcg tggtggtgct tgtcatgctg ctcctcctga 3600

gcctgtgggg ggcccactac tacaggactc agaagctgct atcgaaaaac ccaaagaatc 3660

cttatgagga gtcgagcaga gatgtgagga gtcgcaggcc tgctgacact gaggatggga 3720

tgtcctcttg ccctcaacct tggtttgtgg ttataaaaga acaccaagac ctcaagaatg 3780

ggggtcaacc agtggctggt gaggatggcc aggcagcaga tgggtcaatg caaccaactt 3840

catggaggca ggagccccag ttatgtggaa tgggcacaga gcaaggctgc tggattccag 3900

tatccagtga taagggctcc tgtccccagg taatggagcg aagctttcat atgccctcct 3960

atgggacaca gacccttgaa gggggtgtcg agaagcccca ttctctccta tcagctaacc 4020

cattatggca acaaagggcc ctggacccac cacaccaaat ggagctgact cagtgaaaac 4080

tggaattaaa aggaaagtca agaagaatga actatgtcga tgcacagtat cttttctttc 4140

aaaagtagag caaaactata ggttttggtt ccacaatctc tacgactaat cacctactca 4200

atgcctggag acagatacgt agttgtgctt ttgtttgctc ttttaagcag tctcactgca 4260

gtcttatttc caagtaagag tactgggaga atcactaggt aacttattag aaacccaaat 4320

tgggacaaca gtgctttgta aattgtgttg tcttcagcag tcaatacaaa tagatttttg 4380

tttttgttgt tcctgcagcc ccagaagaaa ttaggggtta aagcagacag tcacactggt 4440

ttggtcagtt acaaagtaat ttctttgatc tggacagaac atttatatca gtttcatgaa 4500

atgattggaa tattacaata ccgttaagat acagtgtagg catttaactc ctcattggcg 4560

tggtccatgc tgatgatttt gcaaaatgag ttgtgatgaa tcaatgaaaa atgtaattta 4620

gaaactgatt tcttcagaat tagatggctt attttttaaa atatttgaat gaaaacattt 4680

tatttttaaa atattacaca ggaggcttcg gagtttctta gtcattactg tccttttccc 4740

ctacagaatt ttccctcttg gtgtgattgc acagaatttg tatgtatttt cagttacaag 4800

attgtaagta aattgcctga tttgttttca ttatagacaa cgatgaattt cttctaatta 4860

tttaaataaa atcaccaaaa acataaacat tttattgtat gcctgattaa gtagttaatt 4920

atagtctaag gcagtactag agttgaacca aaatgatttg tcaagcttgc tgatgtttct 4980

gtttttcgtt tttttttttt ttccggagag aggataggat ctcactctgt tatccaggct 5040

ggagtgtgca atggcacaat catagctcag tgcagcctca aactcctggg ctcaagcaat 5100

cctcctgcct cagcctcccg agtaactagg accacaggca caggccacca tgcctggcta 5160

aggtttttat ttttattttt tgtagacatg gggatcacac aatgttgccc aggctggtct 5220

tgaactcctg gcctcaagca aggtcgtgct ggtaattttg caaaatgaat tgtgattgac 5280

tttcagcctc ccaacgtatt agattatagg cattagccat ggtgcccagc cttgtaactt 5340

ttaaaaaaat tttttaatct acaactctgt agattaaaat ttcacatggt gttctaatta 5400

aatatttttc ttgcagccaa gatattgtta ctacagataa cacaacctga tatggtaact 5460

ttaaattttg ggggctttga atcattcagt ttatgcatta actagtccct ttgtttatct 5520

ttcatttctc aaccccttgt actttggtga taccagacat cagaataaaa agaaattgaa 5580

gtaaaaaaaa aaaaaaaaaa 5600

<210> 18
<211> 1207
<212> PRT
<213> Homo sapiens

<400> 18

Met Leu Leu Thr Leu Ile Ile Leu Leu Pro Val Val Ser Lys Phe Ser
1               5                   10                  15

Phe Val Ser Leu Ser Ala Pro Gln His Trp Ser Cys Pro Glu Gly Thr
            20                  25                  30

Leu Ala Gly Asn Gly Asn Ser Thr Cys Val Gly Pro Ala Pro Phe Leu
        35                  40                  45

Ile Phe Ser His Gly Asn Ser Ile Phe Arg Ile Asp Thr Glu Gly Thr
    50                  55                  60

Asn Tyr Glu Gln Leu Val Val Asp Ala Gly Val Ser Val Ile Met Asp
65                  70                  75                  80

Phe His Tyr Asn Glu Lys Arg Ile Tyr Trp Val Asp Leu Glu Arg Gln
                85                  90                  95

Leu Leu Gln Arg Val Phe Leu Asn Gly Ser Arg Gln Glu Arg Val Cys
            100                 105                 110

Asn Ile Glu Lys Asn Val Ser Gly Met Ala Ile Asn Trp Ile Asn Glu

        115                 120                 125

Glu Val Ile Trp Ser Asn Gln Gln Glu Gly Ile Ile Thr Val Thr Asp
    130                 135                 140

Met Lys Gly Asn Asn Ser His Ile Leu Leu Ser Ala Leu Lys Tyr Pro
145                 150                 155                 160

Ala Asn Val Ala Val Asp Pro Val Glu Arg Phe Ile Phe Trp Ser Ser
                165                 170                 175

Glu Val Ala Gly Ser Leu Tyr Arg Ala Asp Leu Asp Gly Val Gly Val
            180                 185                 190

Lys Ala Leu Leu Glu Thr Ser Glu Lys Ile Thr Ala Val Ser Leu Asp
        195                 200                 205

Val Leu Asp Lys Arg Leu Phe Trp Ile Gln Tyr Asn Arg Glu Gly Ser
    210                 215                 220

Asn Ser Leu Ile Cys Ser Cys Asp Tyr Asp Gly Gly Ser Val His Ile
225                 230                 235                 240

Ser Lys His Pro Thr Gln His Asn Leu Phe Ala Met Ser Leu Phe Gly
                245                 250                 255

Asp Arg Ile Phe Tyr Ser Thr Trp Lys Met Lys Thr Ile Trp Ile Ala
            260                 265                 270

Asn Lys His Thr Gly Lys Asp Met Val Arg Ile Asn Leu His Ser Ser
        275                 280                 285

Phe Val Pro Leu Gly Glu Leu Lys Val Val His Pro Leu Ala Gln Pro
    290                 295                 300

Lys Ala Glu Asp Asp Thr Trp Glu Pro Glu Gln Lys Leu Cys Lys Leu
305                 310                 315                 320

Arg Lys Gly Asn Cys Ser Ser Thr Val Cys Gly Gln Asp Leu Gln Ser
                325                 330                 335

His Leu Cys Met Cys Ala Glu Gly Tyr Ala Leu Ser Arg Asp Arg Lys
            340                 345                 350

Tyr Cys Glu Asp Val Asn Glu Cys Ala Phe Trp Asn His Gly Cys Thr
        355                 360                 365

Leu Gly Cys Lys Asn Thr Pro Gly Ser Tyr Tyr Cys Thr Cys Pro Val
    370                 375                 380

Gly Phe Val Leu Leu Pro Asp Gly Lys Arg Cys His Gln Leu Val Ser
385                 390                 395                 400

Cys Pro Arg Asn Val Ser Glu Cys Ser His Asp Cys Val Leu Thr Ser
                405                 410                 415

Glu Gly Pro Leu Cys Phe Cys Pro Glu Gly Ser Val Leu Glu Arg Asp
            420                 425                 430

Gly Lys Thr Cys Ser Gly Cys Ser Ser Pro Asp Asn Gly Gly Cys Ser
        435                 440                 445

Gln Leu Cys Val Pro Leu Ser Pro Val Ser Trp Glu Cys Asp Cys Phe
    450                 455                 460

Pro Gly Tyr Asp Leu Gln Leu Asp Glu Lys Ser Cys Ala Ala Ser Gly
465                 470                 475                 480

Pro Gln Pro Phe Leu Leu Phe Ala Asn Ser Gln Asp Ile Arg His Met
                485                 490                 495

His Phe Asp Gly Thr Asp Tyr Gly Thr Leu Leu Ser Gln Gln Met Gly
            500                 505                 510

Met Val Tyr Ala Leu Asp His Asp Pro Val Glu Asn Lys Ile Tyr Phe
        515                 520                 525

Ala His Thr Ala Leu Lys Trp Ile Glu Arg Ala Asn Met Asp Gly Ser
    530                 535                 540

Gln Arg Glu Arg Leu Ile Glu Glu Gly Val Asp Val Pro Glu Gly Leu
545                 550                 555                 560

Ala Val Asp Trp Ile Gly Arg Arg Phe Tyr Trp Thr Asp Arg Gly Lys
                565                 570                 575

Ser Leu Ile Gly Arg Ser Asp Leu Asn Gly Lys Arg Ser Lys Ile Ile
            580                 585                 590

Thr Lys Glu Asn Ile Ser Gln Pro Arg Gly Ile Ala Val His Pro Met
        595                 600                 605

Ala Lys Arg Leu Phe Trp Thr Asp Thr Gly Ile Asn Pro Arg Ile Glu
    610                 615                 620

Ser Ser Ser Leu Gln Gly Leu Gly Arg Leu Val Ile Ala Ser Ser Asp
625                 630                 635                 640

Leu Ile Trp Pro Ser Gly Ile Thr Ile Asp Phe Leu Thr Asp Lys Leu
                645                 650                 655

Tyr Trp Cys Asp Ala Lys Gln Ser Val Ile Glu Met Ala Asn Leu Asp
            660                 665                 670

Gly Ser Lys Arg Arg Arg Leu Thr Gln Asn Asp Val Gly His Pro Phe
        675                 680                 685

Ala Val Ala Val Phe Glu Asp Tyr Val Trp Phe Ser Asp Trp Ala Met
    690                 695                 700

Pro Ser Val Met Arg Val Asn Lys Arg Thr Gly Lys Asp Arg Val Arg
705                 710                 715                 720

Leu Gln Gly Ser Met Leu Lys Pro Ser Ser Leu Val Val Val His Pro
                725                 730                 735

Leu Ala Lys Pro Gly Ala Asp Pro Cys Leu Tyr Gln Asn Gly Gly Cys
            740                 745                 750

Glu His Ile Cys Lys Lys Arg Leu Gly Thr Ala Trp Cys Ser Cys Arg
        755                 760                 765

Glu Gly Phe Met Lys Ala Ser Asp Gly Lys Thr Cys Leu Ala Leu Asp
    770                 775                 780

Gly His Gln Leu Leu Ala Gly Gly Glu Val Asp Leu Lys Asn Gln Val
785                 790                 795                 800

Thr Pro Leu Asp Ile Leu Ser Lys Thr Arg Val Ser Glu Asp Asn Ile
                805                 810                 815

Thr Glu Ser Gln His Met Leu Val Ala Glu Ile Met Val Ser Asp Gln
            820                 825                 830

Asp Asp Cys Ala Pro Val Gly Cys Ser Met Tyr Ala Arg Cys Ile Ser
        835                 840                 845

Glu Gly Glu Asp Ala Thr Cys Gln Cys Leu Lys Gly Phe Ala Gly Asp
    850                 855                 860

Gly Lys Leu Cys Ser Asp Ile Asp Glu Cys Glu Met Gly Val Pro Val
865                 870                 875                 880

Cys Pro Pro Ala Ser Ser Lys Cys Ile Asn Thr Glu Gly Gly Tyr Val
                885                 890                 895

Cys Arg Cys Ser Glu Gly Tyr Gln Gly Asp Gly Ile His Cys Leu Asp
            900                 905                 910

Ile Asp Glu Cys Gln Leu Gly Glu His Ser Cys Gly Glu Asn Ala Ser
        915                 920                 925

Cys Thr Asn Thr Glu Gly Gly Tyr Thr Cys Met Cys Ala Gly Arg Leu
    930                 935                 940

Ser Glu Pro Gly Leu Ile Cys Pro Asp Ser Thr Pro Pro Pro His Leu
945                 950                 955                 960

Arg Glu Asp Asp His His Tyr Ser Val Arg Asn Ser Asp Ser Glu Cys
                965                 970                 975

Pro Leu Ser His Asp Gly Tyr Cys Leu His Asp Gly Val Cys Met Tyr
            980                 985                 990

Ile Glu Ala Leu Asp Lys Tyr Ala Cys Asn Cys Val Val Gly Tyr Ile
        995                 1000                1005

Gly Glu Arg Cys Gln Tyr Arg Asp Leu Lys Trp Trp Glu Leu Arg
    1010                1015                1020

His Ala Gly His Gly Gln Gln Gln Lys Val Ile Val Val Ala Val
    1025                1030                1035

Cys Val Val Val Leu Val Met Leu Leu Leu Leu Ser Leu Trp Gly
    1040                1045                1050

Ala His Tyr Tyr Arg Thr Gln Lys Leu Leu Ser Lys Asn Pro Lys
    1055                1060                1065

Asn Pro Tyr Glu Glu Ser Ser Arg Asp Val Arg Ser Arg Arg Pro
    1070                1075                1080

Ala Asp Thr Glu Asp Gly Met Ser Ser Cys Pro Gln Pro Trp Phe

1085                1090                1095

Val Val Ile Lys Glu His Gln Asp Leu Lys Asn Gly Gly Gln Pro
    1100                1105                1110

Val Ala Gly Glu Asp Gly Gln Ala Ala Asp Gly Ser Met Gln Pro
    1115                1120                1125

Thr Ser Trp Arg Gln Glu Pro Gln Leu Cys Gly Met Gly Thr Glu
    1130                1135                1140

Gln Gly Cys Trp Ile Pro Val Ser Ser Asp Lys Gly Ser Cys Pro
    1145                1150                1155

Gln Val Met Glu Arg Ser Phe His Met Pro Ser Tyr Gly Thr Gln
    1160                1165                1170

Thr Leu Glu Gly Gly Val Glu Lys Pro His Ser Leu Leu Ser Ala
    1175                1180                1185

Asn Pro Leu Trp Gln Gln Arg Ala Leu Asp Pro Pro His Gln Met
    1190                1195                1200

Glu Leu Thr Gln
    1205

<210> 19
<211> 667
<212> DNA
<213> Homo sapiens

<400> 19

agcacaggac acagctgggt tctgaagctt ctgagttctg cagcctcacc tctgagaaaa 60

cctctttgcc accaatacca tgaagctctg cgtgactgtc ctgtctctcc tcatgctagt 120

agctgccttc tgctctccag cgctctcagc accaatgggc tcagaccctc ccaccgcctg 180

ctgcttttct tacactgcga ggaagcttcc tcgcaacttt gtggtagatt actatgagac 240

cagcagcctc tgctcccagc cagctgtggt attccaaacc aaaagaagca agcaagtctg 300

tgctgatccc agtgaatcct gggtccagga gtacgtgtat gacctggaac tgaactgagc 360

tgctcagaga caggaagtct tcagggaagg tcacctgagc ccggatgctt ctccatgaga 420

cacatctcct ccatactcag gactcctctc cgcagttcct gtcccttctc ttaatttaat 480

cttttttatg tgccgtgtta ttgtattagg tgtcatttcc attatttata ttagtttagc 540

caaaggataa gtgtccccta tggggatggt ccactgtcac tgtttctctg ctgttgcaaa 600

tacatggata acacatttga ttctgtgtgt tttcataata aaactttaaa ataaaatgca 660

gacagtt 667

<210> 20
<211> 92
<212> PRT
<213> Homo sapiens

<400> 20

Met Lys Leu Cys Val Thr Val Leu Ser Leu Leu Met Leu Val Ala Ala
1               5                   10                  15

Phe Cys Ser Pro Ala Leu Ser Ala Pro Met Gly Ser Asp Pro Pro Thr
            20                  25                  30

Ala Cys Cys Phe Ser Tyr Thr Ala Arg Lys Leu Pro Arg Asn Phe Val
        35                  40                  45

Val Asp Tyr Tyr Glu Thr Ser Ser Leu Cys Ser Gln Pro Ala Val Val
    50                  55                  60

Phe Gln Thr Lys Arg Ser Lys Gln Val Cys Ala Asp Pro Ser Glu Ser
65                  70                  75                  80

Trp Val Gln Glu Tyr Val Tyr Asp Leu Glu Leu Asn
                85                  90

<210> 21
<211> 2545
<212> DNA
<213> Homo sapiens

<400> 21

atccaataca ggagtgactt ggaactccat tctatcacta tgaagaaaag tggtgttctt 60
ttcctcttgg gcatcatctt gctggttctg attggagtgc aaggaacccc agtagtgaga 120
aagggtcgct gttcctgcat cagcaccaac caagggacta tccacctaca atccttgaaa 180
gaccttaaac aatttgcccc aagcccttcc tgcgagaaaa ttgaaatcat tgctacactg 240
aagaatggag ttcaaacatg tctaaaccca gattcagcag atgtgaagga actgattaaa 300
aagtgggaga aacaggtcag ccaaaagaaa aagcaaaaga atgggaaaaa acatcaaaaa 360
aagaaagttc tgaaagttcg aaaatctcaa cgttctcgtc aaaagaagac tacataagag 420
accacttcac caataagtat tctgtgttaa aaatgttcta ttttaattat accgctatca 480
ttccaaagga ggatggcata taatacaaag gcttattaat ttgactagaa aatttaaaac 540
attactctga aattgtaact aaagttagaa agttgatttt aagaatccaa acgttaagaa 600

ttgttaaagg ctatgattgt ctttgttctt ctaccaccca ccagttgaat ttcatcatgc 660

ttaaggccat gattttagca atacccatgt ctacacagat gttcacccaa ccacatccca 720

ctcacaacag ctgcctggaa gagcagccct aggcttccac gtactgcagc ctccagagag 780

tatctgaggc acatgtcagc aagtcctaag cctgttagca tgctggtgag ccaagcagtt 840

tgaaattgag ctggacctca ccaagctgct gtggccatca acctctgtat ttgaatcagc 900

ctacaggcct cacacacaat gtgtctgaga gattcatgct gattgttatt gggtatcacc 960

actggagatc accagtgtgt ggctttcaga gcctcctttc tggctttgga agccatgtga 1020

ttccatcttg cccgctcagg ctgaccactt tatttctttt tgttcccctt tgcttcattc 1080

aagtcagctc ttctccatcc taccacaatg cagtgccttt cttctctcca gtgcacctgt 1140

catatgctct gatttatctg agtcaactcc tttctcatct tgtccccaac accccacaga 1200

agtgctttct tctcccaatt catcctcact cagtccagct tagttcaagt cctgcctctt 1260

aaataaacct ttttggacac acaaattatc ttaaaactcc tgtttcactt ggttcagtac 1320

cacatgggtg aacactcaat ggttaactaa ttcttgggtg tttatcctat ctctccaacc 1380

agattgtcag ctccttgagg gcaagagcca cagtatattt ccctgtttct tccacagtgc 1440

ctaataatac tgtggaacta ggttttaata attttttaat tgatgttgtt atgggcagga 1500

tggcaaccag accattgtct cagagcaggt gctggctctt tcctggctac tccatgttgg 1560

ctagcctctg gtaacctctt acttattatc ttcaggacac tcactacagg gaccagggat 1620

gatgcaacat ccttgtcttt ttatgacagg atgtttgctc agcttctcca acaataagaa 1680

gcacgtggta aaacacttgc ggatattctg gactgttttt aaaaaatata cagtttaccg 1740

aaaatcatat aatcttacaa tgaaaaggac tttatagatc agccagtgac caaccttttc 1800

ccaaccatac aaaaattcct tttcccgaag gaaaagggct ttctcaataa gcctcagctt 1860

tctaagatct aacaagatag ccaccgagat ccttatcgaa actcatttta ggcaaatatg 1920

agttttattg tccgtttact tgtttcagag tttgtattgt gattatcaat taccacacca 1980

tctcccatga agaaagggaa cggtgaagta ctaagcgcta gaggaagcag ccaagtcggt 2040

tagtggaagc atgattggtg cccagttagc ctctgcagga tgtggaaacc tccttccagg 2100

ggaggttcag tgaattgtgt aggagaggtt gtctgtggcc agaatttaaa cctatactca 2160

ctttcccaaa ttgaatcact gctcacactg ctgatgattt agagtgctgt ccggtggaga 2220

tcccacccga acgtcttatc taatcatgaa actccctagt tccttcatgt aacttccctg 2280

aaaaatctaa gtgtttcata aatttgagag tctgtgaccc acttaccttg catctcacag 2340

gtagacagta tataactaac aaccaaagac tacatattgt cactgacaca cacgttataa 2400

tcatttatca tatatataca tacatgcata cactctcaaa gcaaataatt tttcacttca 2460

aaacagtatt gacttgtata ccttgtaatt tgaaatattt tctttgttaa aatagaatgg 2520

tatcaataaa tagaccatta atcag 2545

<210> 22
<211> 125
<212> PRT
<213> Homo sapiens

<400> 22

Met Lys Lys Ser Gly Val Leu Phe Leu Leu Gly Ile Ile Leu Leu Val
1               5                   10                  15

Leu Ile Gly Val Gln Gly Thr Pro Val Val Arg Lys Gly Arg Cys Ser
            20                  25                  30

Cys Ile Ser Thr Asn Gln Gly Thr Ile His Leu Gln Ser Leu Lys Asp
        35                  40                  45

Leu Lys Gln Phe Ala Pro Ser Pro Ser Cys Glu Lys Ile Glu Ile Ile
    50                  55                  60

Ala Thr Leu Lys Asn Gly Val Gln Thr Cys Leu Asn Pro Asp Ser Ala
65                  70                  75                  80

Asp Val Lys Glu Leu Ile Lys Lys Trp Glu Lys Gln Val Ser Gln Lys
                85                  90                  95

Lys Lys Gln Lys Asn Gly Lys Lys His Gln Lys Lys Lys Val Leu Lys
            100                 105                 110

Val Arg Lys Ser Gln Arg Ser Arg Gln Lys Lys Thr Thr
        115                 120                 125

<210> 23
<211> 760
<212> DNA
<213> Homo sapiens

<400> 23

gaggaaccga gaggctgaga ctaacccaga aacatccaat tctcaaactg aagctcgcac 60

tctcgcctcc agcatgaaag tctctgccgc ccttctgtgc ctgctgctca tagcagccac 120

cttcattccc caagggctcg ctcagccaga tgcaatcaat gccccagtca cctgctgtta 180

taacttcacc aataggaaga tctcagtgca gaggctcgcg agctatagaa gaatcaccag 240

cagcaagtgt cccaaagaag ctgtgatctt caagaccatt gtggccaagg agatctgtgc 300

tgaccccaag cagaagtggg ttcaggattc catggaccac ctggacaagc aaacccaaac 360

tccgaagact tgaacactca ctccacaacc caagaatctg cagctaactt attttcccct 420

agctttcccc agacaccctg ttttatttta ttataatgaa ttttgtttgt tgatgtgaaa 480

cattatgcct taagtaatgt taattcttat ttaagttatt gatgttttaa gtttatcttt 540

catggtacta gtgtttttta gatacagaga cttggggaaa ttgcttttcc tcttgaacca 600

cagttctacc cctgggatgt tttgagggtc tttgcaagaa tcattaatac aaagaatttt 660

ttttaacatt ccaatgcatt gctaaaatat tattgtggaa atgaatattt tgtaactatt 720

acaccaaata aatatatttt tgtacaaaaa aaaaaaaaaa 760

<210> 24
<211> 99
<212> PRT
<213> Homo sapiens

<400> 24

Met Lys Val Ser Ala Ala Leu Leu Cys Leu Leu Leu Ile Ala Ala Thr
1               5                   10                  15

Phe Ile Pro Gln Gly Leu Ala Gln Pro Asp Ala Ile Asn Ala Pro Val
            20                  25                  30

Thr Cys Cys Tyr Asn Phe Thr Asn Arg Lys Ile Ser Val Gln Arg Leu
        35                  40                  45

Ala Ser Tyr Arg Arg Ile Thr Ser Ser Lys Cys Pro Lys Glu Ala Val
    50                  55                  60

Ile Phe Lys Thr Ile Val Ala Lys Glu Ile Cys Ala Asp Pro Lys Gln
65                  70                  75                  80

Lys Trp Val Gln Asp Ser Met Asp His Leu Asp Lys Gln Thr Gln Thr
                85                  90                  95

Pro Lys Thr

<210> 25
<211> 1439
<212> DNA
<213> Homo sapiens

<400> 25

tgcggcggcg agggaggagg aagaagcgga ggaggcggct cccgcgctcg cagggccgtg        60

ccacctgccc gcccgcccgc tcgctcgctc gcccgccgcg ccgcgctgcc gaccgccagc 120

atgctgccga gagtgggctg ccccgcgctg ccgctgccgc cgccgccgct gctgccgctg 180

ctgccgctgc tgctgctgct actgggcgcg agtggcggcg gcggcggggc gcgcgcggag 240

gtgctgttcc gctgcccgcc ctgcacaccc gagcgcctgg ccgcctgcgg gcccccgccg 300

gttgcgccgc ccgccgcggt ggccgcagtg gccggaggcg cccgcatgcc atgcgcggag 360

ctcgtccggg agccgggctg cggctgctgc tcggtgtgcg cccggctgga gggcgaggcg 420

tgcggcgtct acaccccgcg ctgcggccag gggctgcgct gctatcccca cccgggctcc 480

gagctgcccc tgcaggcgct ggtcatgggc gagggcactt gtgagaagcg ccgggacgcc 540

gagtatggcg ccagcccgga gcaggttgca gacaatggcg atgaccactc agaaggaggc 600

ctggtggaga accacgtgga cagcaccatg aacatgttgg gcgggggagg cagtgctggc 660

cggaagcccc tcaagtcggg tatgaaggag ctggccgtgt tccgggagaa ggtcactgag 720

cagcaccggc agatgggcaa gggtggcaag catcaccttg gcctggagga gcccaagaag 780

ctgcgaccac cccctgccag gactccctgc caacaggaac tggaccaggt cctggagcgg 840

atctccacca tgcgccttcc ggatgagcgg ggccctctgg agcacctcta ctccctgcac 900

atccccaact gtgacaagca tggcctgtac aacctcaaac agtgcaagat gtctctgaac 960

gggcagcgtg gggagtgctg gtgtgtgaac cccaacaccg ggaagctgat ccagggagcc 1020

cccaccatcc ggggggaccc cgagtgtcat ctcttctaca atgagcagca ggaggctcgc 1080

ggggtgcaca cccagcggat gcagtagacc gcagccagcc ggtgcctggc gcccctgccc 1140

cccgcccctc tccaaacacc ggcagaaaac ggagagtgct tgggtggtgg gtgctggagg 1200

attttccagt tctgacacac gtatttatat ttggaaagag accagcaccg agctcggcac 1260

ctccccggcc tctctcttcc cagctgcaga tgccacacct gctccttctt gctttccccg 1320

ggggaggaag ggggttgtgg tcggggagct ggggtacagg tttggggagg gggaagagaa 1380

atttttattt ttgaacccct gtgtcccttt tgcataagat taaaggaagg aaaagtaaa 1439

<210> 26
<211> 328
<212> PRT
<213> Homo sapiens

<400> 26

Met Leu Pro Arg Val Gly Cys Pro Ala Leu Pro Leu Pro Pro Pro Pro
1               5                   10                  15

Leu Leu Pro Leu Leu Pro Leu Leu Leu Leu Leu Leu Gly Ala Ser Gly
            20                  25                  30

Gly Gly Gly Gly Ala Arg Ala Glu Val Leu Phe Arg Cys Pro Pro Cys
        35                  40                  45

Thr Pro Glu Arg Leu Ala Ala Cys Gly Pro Pro Pro Val Ala Pro Pro
    50                  55                  60

Ala Ala Val Ala Ala Val Ala Gly Gly Ala Arg Met Pro Cys Ala Glu
65                  70                  75                  80

Leu Val Arg Glu Pro Gly Cys Gly Cys Cys Ser Val Cys Ala Arg Leu
                85                  90                  95

Glu Gly Glu Ala Cys Gly Val Tyr Thr Pro Arg Cys Gly Gln Gly Leu
            100                 105                 110

Arg Cys Tyr Pro His Pro Gly Ser Glu Leu Pro Leu Gln Ala Leu Val
        115                 120                 125

Met Gly Glu Gly Thr Cys Glu Lys Arg Arg Asp Ala Glu Tyr Gly Ala
    130                 135                 140

Ser Pro Glu Gln Val Ala Asp Asn Gly Asp Asp His Ser Glu Gly Gly
145                 150                 155                 160

Leu Val Glu Asn His Val Asp Ser Thr Met Asn Met Leu Gly Gly Gly
                165                 170                 175

Gly Ser Ala Gly Arg Lys Pro Leu Lys Ser Gly Met Lys Glu Leu Ala
            180                 185                 190

Val Phe Arg Glu Lys Val Thr Glu Gln His Arg Gln Met Gly Lys Gly
        195                 200                 205

Gly Lys His His Leu Gly Leu Glu Glu Pro Lys Lys Leu Arg Pro Pro
    210                 215                 220

Pro Ala Arg Thr Pro Cys Gln Gln Glu Leu Asp Gln Val Leu Glu Arg
225                 230                 235                 240

Ile Ser Thr Met Arg Leu Pro Asp Glu Arg Gly Pro Leu Glu His Leu
                245                 250                 255

Tyr Ser Leu His Ile Pro Asn Cys Asp Lys His Gly Leu Tyr Asn Leu
            260                 265                 270

Lys Gln Cys Lys Met Ser Leu Asn Gly Gln Arg Gly Glu Cys Trp Cys
        275                 280                 285

Val Asn Pro Asn Thr Gly Lys Leu Ile Gln Gly Ala Pro Thr Ile Arg
    290                 295                 300

Gly Asp Pro Glu Cys His Leu Phe Tyr Asn Glu Gln Gln Glu Ala Arg
305                 310                 315                 320

Gly Val His Thr Gln Arg Met Gln
                325

<210> 27
<211> 1660
<212> DNA
<213> Homo sapiens

<400> 27

ggtgcactag caaaacaaac ttattttgaa cactcagctc ctagcgtgcg gcgctgccaa 60
tcattaacct cctggtgcaa gtggcgcggc ctgtgccctt tataaggtgc gcgctgtgtc 120
cagcgagcat cggccaccgc catcccatcc agcgagcatc tgccgccgcg ccgccgccac 180
cctcccagag agcactggcc accgctccac catcacttgc ccagagtttg ggccaccgcc 240
cgccgccacc agcccagaga gcatcggccc ctgtctgctg ctcgcgcctg gagatgtcag 300
aggtccccgt tgctcgcgtc tggctggtac tgctcctgct gactgtccag gtcggcgtga 360
cagccggcgc tccgtggcag tgcgcgccct gctccgccga gaagctcgcg ctctgcccgc 420
cggtgtccgc ctcgtgctcg gaggtcaccc ggtccgccgg ctgcggctgt tgcccgatgt 480
gcgccctgcc tctgggcgcc gcgtgcggcg tggcgactgc acgctgcgcc cggggactca 540
gttgccgcgc gctgccgggg gagcagcaac ctctgcacgc cctcacccgc ggccaaggcg 600
cctgcgtgca ggagtctgac gcctccgctc cccatgctgc agaggcaggg agccctgaaa 660
gcccagagag cacggagata actgaggagg agctcctgga taatttccat ctgatggccc 720
cttctgaaga ggatcattcc atcctttggg acgccatcag tacctatgat ggctcgaagg 780
ctctccatgt caccaacatc aaaaaatgga aggagccctg ccgaatagaa ctctacagag 840
tcgtagagag tttagccaag gcacaggaga catcaggaga agaaatttcc aaattttacc 900
tgccaaactg caacaagaat ggattttatc acagcagaca gtgtgagaca tccatggatg 960
gagaggcggg actctgctgg tgcgtctacc cttggaatgg gaagaggatc cctgggtctc 1020
cagagatcag gggagacccc aactgccaga tatattttaa tgtacaaaac tgaaaccaga 1080
tgaaataatg ttctgtcacg tgaaatattt aagtatatag tatatttata ctctagaaca 1140
tgcacattta tatatatatg tatatgtata tatatatagt aactactttt tatactccat 1200
acataacttg atatagaaag ctgtttattt attcactgta agtttatttt ttctacacag 1260
taaaaacttg tactatgtta ataacttgtc ctatgtcaat ttgtatatca tgaaacactt 1320
ctcatcatat tgtatgtaag taattgcatt tctgctcttc caaagctcct gcgtctgttt 1380
ttaaagagca tggaaaaata ctgcctagaa aatgcaaaat gaaataagag agagtagttt 1440
ttcagctagt ttgaaggagg acggttaact tgtatattcc accattcaca tttgatgtac 1500
atgtgtaggg aaagttaaaa gtgttgatta cataatcaaa gctacctgtg gtgatgttgc 1560
cacctgttaa aatgtacact ggatatgttg ttaaacacgt gtctataatg gaaacattta 1620
caataaatat tctgcatgga aatactgtta aaaaaaaaaa 1660

<210> 28
<211> 259

<212> PRT
<213> Homo sapiens

<400> 28

Met Ser Glu Val Pro Val Ala Arg Val Trp Leu Val Leu Leu Leu Leu
1               5                   10                  15

Thr Val Gln Val Gly Val Thr Ala Gly Ala Pro Trp Gln Cys Ala Pro
            20                  25                  30

Cys Ser Ala Glu Lys Leu Ala Leu Cys Pro Pro Val Ser Ala Ser Cys
        35                  40                  45

Ser Glu Val Thr Arg Ser Ala Gly Cys Gly Cys Cys Pro Met Cys Ala
    50                  55                  60

Leu Pro Leu Gly Ala Ala Cys Gly Val Ala Thr Ala Arg Cys Ala Arg
65                  70                  75                  80

Gly Leu Ser Cys Arg Ala Leu Pro Gly Glu Gln Gln Pro Leu His Ala
                85                  90                  95

Leu Thr Arg Gly Gln Gly Ala Cys Val Gln Glu Ser Asp Ala Ser Ala
            100                 105                 110

Pro His Ala Ala Glu Ala Gly Ser Pro Glu Ser Pro Glu Ser Thr Glu
        115                 120                 125

Ile Thr Glu Glu Glu Leu Leu Asp Asn Phe His Leu Met Ala Pro Ser
    130                 135                 140

Glu Glu Asp His Ser Ile Leu Trp Asp Ala Ile Ser Thr Tyr Asp Gly
145                 150                 155                 160

Ser Lys Ala Leu His Val Thr Asn Ile Lys Lys Trp Lys Glu Pro Cys
                165                 170                 175

Arg Ile Glu Leu Tyr Arg Val Val Glu Ser Leu Ala Lys Ala Gln Glu
            180                 185                 190

Thr Ser Gly Glu Glu Ile Ser Lys Phe Tyr Leu Pro Asn Cys Asn Lys
        195                 200                 205

Asn Gly Phe Tyr His Ser Arg Gln Cys Glu Thr Ser Met Asp Gly Glu
    210                 215                 220

Ala Gly Leu Cys Trp Cys Val Tyr Pro Trp Asn Gly Lys Arg Ile Pro
225                 230                 235                 240

Gly Ser Pro Glu Ile Arg Gly Asp Pro Asn Cys Gln Ile Tyr Phe Asn
                245                 250                 255

Val Gln Asn

<210> 29
<211> 1220
<212> DNA
<213> Homo sapiens

<400> 29

agtcccagct cagagccgca acctgcacag ccatgcccgg gcaagaactc aggacggtga 60

atggctctca gatgctcctg gtgttgctgg tgctctcgtg gctgccgcat gggggcgccc 120

tgtctctggc cgaggcgagc cgcgcaagtt tcccgggacc ctcagagttg cactccgaag 180

actccagatt ccgagagttg cggaaacgct acgaggacct gctaaccagg ctgcgggcca 240

accagagctg ggaagattcg aacaccgacc tcgtcccggc ccctgcagtc cggatactca 300

cgccagaagt gcggctggga tccggcggcc acctgcacct gcgtatctct cgggccgccc 360

ttcccgaggg gctccccgag gcctcccgcc ttcaccgggc tctgttccgg ctgtccccga 420

cggcgtcaag gtcgtgggac gtgacacgac cgctgcggcg tcagctcagc cttgcaagac 480

cccaggcgcc cgcgctgcac ctgcgactgt cgccgccgcc gtcgcagtcg gaccaactgc 540

tggcagaatc ttcgtccgca cggccccagc tggagttgca cttgcggccg caagccgcca 600

gggggcgccg cagagcgcgt gcgcgcaacg gggaccactg tccgctcggg cccgggcgtt 660

gctgccgtct gcacacggtc cgcgcgtcgc tggaagacct gggctgggcc gattgggtgc 720

tgtcgccacg ggaggtgcaa gtgaccatgt gcatcggcgc gtgcccgagc cagttccggg 780

cggcaaacat gcacgcgcag atcaagacga gcctgcaccg cctgaagccc gacacggtgc 840

cagcgccctg ctgcgtgccc gccagctaca atcccatggt gctcattcaa aagaccgaca 900

ccggggtgtc gctccagacc tatgatgact tgttagccaa agactgccac tgcatatgag 960

cagtcctggt ccttccactg tgcacctgcg cggaggacgc gacctcagtt gtcctgccct 1020

gtggaatggg ctcaaggttc ctgagacacc cgattcctgc ccaaacagct gtatttatat 1080

aagtctgtta tttattatta atttattggg gtgaccttct tggggactcg ggggctggtc 1140

tgatggaact gtgtatttat ttaaaactct ggtgataaaa ataaagctgt ctgaactgtt 1200

aaaaaaaaaa aaaaaaaaaa 1220

<210> 30
<211> 308
<212> PRT
<213> Homo sapiens

<400> 30

Met Pro Gly Gln Glu Leu Arg Thr Val Asn Gly Ser Gln Met Leu Leu
1               5                   10                  15

Val Leu Leu Val Leu Ser Trp Leu Pro His Gly Gly Ala Leu Ser Leu
            20                  25                  30

Ala Glu Ala Ser Arg Ala Ser Phe Pro Gly Pro Ser Glu Leu His Ser
        35                  40                  45

Glu Asp Ser Arg Phe Arg Glu Leu Arg Lys Arg Tyr Glu Asp Leu Leu
    50                  55                  60

Thr Arg Leu Arg Ala Asn Gln Ser Trp Glu Asp Ser Asn Thr Asp Leu
65                  70                  75                  80

Val Pro Ala Pro Ala Val Arg Ile Leu Thr Pro Glu Val Arg Leu Gly
                85                  90                  95

Ser Gly Gly His Leu His Leu Arg Ile Ser Arg Ala Ala Leu Pro Glu
            100                 105                 110

Gly Leu Pro Glu Ala Ser Arg Leu His Arg Ala Leu Phe Arg Leu Ser
        115                 120                 125

Pro Thr Ala Ser Arg Ser Trp Asp Val Thr Arg Pro Leu Arg Arg Gln
    130                 135                 140

Leu Ser Leu Ala Arg Pro Gln Ala Pro Ala Leu His Leu Arg Leu Ser
145                 150                 155                 160

Pro Pro Pro Ser Gln Ser Asp Gln Leu Leu Ala Glu Ser Ser Ser Ala
                165                 170                 175

Arg Pro Gln Leu Glu Leu His Leu Arg Pro Gln Ala Ala Arg Gly Arg
            180                 185                 190

Arg Arg Ala Arg Ala Arg Asn Gly Asp His Cys Pro Leu Gly Pro Gly
        195                 200                 205

Arg Cys Cys Arg Leu His Thr Val Arg Ala Ser Leu Glu Asp Leu Gly
    210                 215                 220

Trp Ala Asp Trp Val Leu Ser Pro Arg Glu Val Gln Val Thr Met Cys
225                 230                 235                 240

Ile Gly Ala Cys Pro Ser Gln Phe Arg Ala Ala Asn Met His Ala Gln
                245                 250                 255

Ile Lys Thr Ser Leu His Arg Leu Lys Pro Asp Thr Val Pro Ala Pro
            260                 265                 270

Cys Cys Val Pro Ala Ser Tyr Asn Pro Met Val Leu Ile Gln Lys Thr
        275                 280                 285

Asp Thr Gly Val Ser Leu Gln Thr Tyr Asp Asp Leu Leu Ala Lys Asp
    290                 295                 300

Cys His Cys Ile
305

<210> 31
<211> 3677
<212> DNA
<213> Homo sapiens

<400> 31

tcgcggaggc ttggggcagc cgggtagctc ggaggtcgtg gcgctggggg ctagcaccag 60

cgctctgtcg ggaggcgcag cggttaggtg gaccggtcag cggactcacc ggccagggcg 120

ctcggtgctg gaatttgata ttcattgatc cgggttttat ccctcttctt ttttcttaaa 180

catttttttt taaaactgta ttgtttctcg ttttaattta tttttgcttg ccattcccca 240

cttgaatcgg gccgacggct tggggagatt gctctacttc cccaaatcac tgtggatttt 300

ggaaaccagc agaaagagga aagaggtagc aagagctcca gagagaagtc gaggaagaga 360
gagacggggt cagagagagc gcgcgggcgt gcgagcagcg aaagcgacag gggcaaagtg 420
agtgacctgc ttttgggggt gaccgccgga gcgcggcgtg agccctcccc cttgggatcc 480
cgcagctgac cagtcgcgct gacggacaga cagacagaca ccgcccccag ccccagctac 540
cacctcctcc ccggccggcg gcggacagtg gacgcggcgg cgagccgcgg gcaggggccg 600
gagcccgcgc ccggaggcgg ggtggagggg gtcggggctc gcggcgtcgc actgaaactt 660
ttcgtccaac ttctgggctg ttctcgcttc ggaggagccg tggtccgcgc gggggaagcc 720
gagccgagcg gagccgcgag aagtgctagc tcgggccggg aggagccgca gccggaggag 780
ggggaggagg aagaagagaa ggaagaggag agggggccgc agtggcgact cggcgctcgg 840
aagccgggct catggacggg tgaggcggcg gtgtgcgcag acagtgctcc agccgcgcgc 900
gctccccagg ccctggcccg ggcctcgggc cggggaggaa gagtagctcg ccgaggcgcc 960
gaggagagcg ggccgcccca cagcccgagc cggagaggga gcgcgagccg cgccggcccc 1020
ggtcgggcct ccgaaaccat gaactttctg ctgtcttggg tgcattggag ccttgccttg 1080
ctgctctacc tccaccatgc caagtggtcc caggctgcac ccatggcaga aggaggaggg 1140
cagaatcatc acgaagtggt gaagttcatg gatgtctatc agcgcagcta ctgccatcca 1200
atcgagaccc tggtggacat cttccaggag taccctgatg agatcgagta catcttcaag 1260
ccatcctgtg tgcccctgat gcgatgcggg ggctgctgca atgacgaggg cctggagtgt 1320
gtgcccactg aggagtccaa catcaccatg cagattatgc ggatcaaacc tcaccaaggc 1380
cagcacatag gagagatgag cttcctacag cacaacaaat gtgaatgcag accaaagaaa 1440
gatagagcaa gacaagaaaa aaaatcagtt cgaggaaagg gaaaggggca aaaacgaaag 1500
cgcaagaaat cccggtataa gtcctggagc gtgtacgttg gtgcccgctg ctgtctaatg 1560
ccctggagcc tccctggccc ccatccctgt gggccttgct cagagcggag aaagcatttg 1620
tttgtacaag atccgcagac gtgtaaatgt tcctgcaaaa acacagactc gcgttgcaag 1680
gcgaggcagc ttgagttaaa cgaacgtact tgcagatgtg acaagccgag gcggtgagcc 1740
gggcaggagg aaggagcctc cctcagggtt tcgggaacca gatctctcac caggaaagac 1800
tgatacagaa cgatcgatac agaaaccacg ctgccgccac cacaccatca ccatcgacag 1860
aacagtcctt aatccagaaa cctgaaatga aggaagagga gactctgcgc agagcacttt 1920
gggtccggag ggcgagactc cggcggaagc attcccgggc gggtgaccca gcacggtccc 1980
tcttggaatt ggattcgcca ttttattttt cttgctgcta aatcaccgag cccggaagat 2040
tagagagttt tatttctggg attcctgtag acacacccac ccacatacat acatttatat 2100

atatatatat tatatatata taaaaataaa tatctctatt ttatatatat aaaatatata 2160
tattcttttt ttaaattaac agtgctaatg ttattggtgt cttcactgga tgtatttgac 2220
tgctgtggac ttgagttggg aggggaatgt tcccactcag atcctgacag ggaagaggag 2280
gagatgagag actctggcat gatctttttt ttgtcccact tggtggggcc agggtcctct 2340
cccctgccca ggaatgtgca aggccagggc atgggggcaa atatgaccca gttttgggaa 2400
caccgacaaa cccagccctg gcgctgagcc tctctacccc aggtcagacg gacagaaaga 2460
cagatcacag gtacagggat gaggacaccg gctctgacca ggagtttggg gagcttcagg 2520
acattgctgt gctttgggga ttccctccac atgctgcacg cgcatctcgc ccccaggggc 2580
actgcctgga agattcagga gcctgggcgg ccttcgctta ctctcacctg cttctgagtt 2640
gcccaggaga ccactggcag atgtcccggc gaagagaaga gacacattgt tggaagaagc 2700
agcccatgac agctcccctt cctgggactc gccctcatcc tcttcctgct ccccttcctg 2760
gggtgcagcc taaaaggacc tatgtcctca caccattgaa accactagtt ctgtcccccc 2820
aggagacctg gttgtgtgtg tgtgagtggt tgaccttcct ccatcccctg gtccttccct 2880
tcccttcccg aggcacagag agacagggca ggatccacgt gcccattgtg gaggcagaga 2940
aaagagaaag tgttttatat acggtactta tttaatatcc ctttttaatt agaaattaaa 3000
acagttaatt taattaaaga gtagggtttt ttttcagtat tcttggttaa tatttaattt 3060
caactattta tgagatgtat cttttgctct ctcttgctct cttatttgta ccggtttttg 3120
tatataaaat tcatgtttcc aatctctctc tccctgatcg gtgacagtca ctagcttatc 3180
ttgaacagat atttaatttt gctaacactc agctctgccc tccccgatcc cctggctccc 3240
cagcacacat tcctttgaaa taaggtttca atatacatct acatactata tatatatttg 3300
gcaacttgta tttgtgtgta tatatatata tatatgttta tgtatatatg tgattctgat 3360
aaaatagaca ttgctattct gttttttata tgtaaaaaca aaacaagaaa aaatagagaa 3420
ttctacatac taaatctctc tcctttttta attttaatat ttgttatcat ttatttattg 3480
gtgctactgt ttatccgtaa taattgtggg gaaaagatat taacatcacg tctttgtctc 3540
tagtgcagtt tttcgagata ttccgtagta catatttatt tttaaacaac gacaaagaaa 3600
tacagatata tcttaaaaaa aaaaaagcat tttgtattaa agaatttaat tctgatctca 3660
aaaaaaaaaa aaaaaaa 3677

<210> 32
<211> 412
<212> PRT
<213> Homo sapiens

<400> 32

Met Thr Asp Arg Gln Thr Asp Thr Ala Pro Ser Pro Ser Tyr His Leu
1               5                   10                  15

Leu Pro Gly Arg Arg Arg Thr Val Asp Ala Ala Ala Ser Arg Gly Gln
            20                  25                  30

Gly Pro Glu Pro Ala Pro Gly Gly Gly Val Glu Gly Val Gly Ala Arg
        35                  40                  45

Gly Val Ala Leu Lys Leu Phe Val Gln Leu Leu Gly Cys Ser Arg Phe
    50                  55                  60

Gly Gly Ala Val Val Arg Ala Gly Glu Ala Glu Pro Ser Gly Ala Ala
65                  70                  75                  80

Arg Ser Ala Ser Ser Gly Arg Glu Glu Pro Gln Pro Glu Glu Gly Glu
                85                  90                  95

Glu Glu Glu Glu Lys Glu Glu Glu Arg Gly Pro Gln Trp Arg Leu Gly
            100                 105                 110

Ala Arg Lys Pro Gly Ser Trp Thr Gly Glu Ala Ala Val Cys Ala Asp
        115                 120                 125

Ser Ala Pro Ala Ala Arg Ala Pro Gln Ala Leu Ala Arg Ala Ser Gly
    130                 135                 140

Arg Gly Gly Arg Val Ala Arg Arg Gly Ala Glu Glu Ser Gly Pro Pro
145                 150                 155                 160

His Ser Pro Ser Arg Arg Gly Ser Ala Ser Arg Ala Gly Pro Gly Arg
                165                 170                 175

Ala Ser Glu Thr Met Asn Phe Leu Leu Ser Trp Val His Trp Ser Leu
            180                 185                 190

Ala Leu Leu Leu Tyr Leu His His Ala Lys Trp Ser Gln Ala Ala Pro
        195                 200                 205

Met Ala Glu Gly Gly Gly Gln Asn His His Glu Val Val Lys Phe Met
    210                 215                 220

Asp Val Tyr Gln Arg Ser Tyr Cys His Pro Ile Glu Thr Leu Val Asp
225                 230                 235                 240

Ile Phe Gln Glu Tyr Pro Asp Glu Ile Glu Tyr Ile Phe Lys Pro Ser
                245                 250                 255

Cys Val Pro Leu Met Arg Cys Gly Gly Cys Cys Asn Asp Glu Gly Leu
            260                 265                 270

Glu Cys Val Pro Thr Glu Glu Ser Asn Ile Thr Met Gln Ile Met Arg
        275                 280                 285

Ile Lys Pro His Gln Gly Gln His Ile Gly Glu Met Ser Phe Leu Gln
    290                 295                 300

His Asn Lys Cys Glu Cys Arg Pro Lys Lys Asp Arg Ala Arg Gln Glu
305                 310                 315                 320

Lys Lys Ser Val Arg Gly Lys Gly Lys Gly Gln Lys Arg Lys Arg Lys
                325                 330                 335

Lys Ser Arg Tyr Lys Ser Trp Ser Val Tyr Val Gly Ala Arg Cys Cys
            340                 345                 350

Leu Met Pro Trp Ser Leu Pro Gly Pro His Pro Cys Gly Pro Cys Ser
        355                 360                 365

Glu Arg Arg Lys His Leu Phe Val Gln Asp Pro Gln Thr Cys Lys Cys
    370                 375                 380

Ser Cys Lys Asn Thr Asp Ser Arg Cys Lys Ala Arg Gln Leu Glu Leu
385                 390                 395                 400

Asn Glu Arg Thr Cys Arg Cys Asp Lys Pro Arg Arg
                405                 410